(19) Europäisches Patentamt
European Patent Office
Office européen des brevets

(11) Numéro de publication : **0 445 026 B1**

(12) # FASCICULE DE BREVET EUROPEEN

(45) Date de publication du fascicule du brevet : **26.01.94 Bulletin 94/04**

(21) Numéro de dépôt : **91400527.7**

(22) Date de dépôt : **27.02.91**

(51) Int. Cl.$^5$ : **C07D 417/14,** A61K 31/34, C07D 417/04, C07D 413/14, C07D 413/04, C07D 405/14, C07D 401/04, C07D 401/14, A61K 31/445, A61K 31/425

(54) **Dérivés d'aminométhyl-pipéridine, leur procédé de préparation et les compositions pharmaceutiques qui les contiennent.**

(30) Priorité : **27.02.90 FR 9002394**

(43) Date de publication de la demande : **04.09.91 Bulletin 91/36**

(45) Mention de la délivrance du brevet : **26.01.94 Bulletin 94/04**

(84) Etats contractants désignés : **AT BE CH DE DK ES FR GB GR IT LI LU NL SE**

(56) Documents cités :
**EP-A- 0 217 700**
**EP-A- 0 304 353**
**DE-A- 2 314 239**
**GB-A- 1 077 173**

(56) Documents cités :
**JOURNAL OF HETEROCYCLIC CHEMISTRY. vol. 24, no. 1, 1 janvier 1987, pages 31-37, PROVO, US; R. IEMURA et al.: "Synthesis of bezimidazole derivatives as potential H1-antihistaminic agents"**

(73) Titulaire : **ADIR ET COMPAGNIE**
**1 rue Carle Hébert**
**F-92415 Courbevoie Cédex (FR)**

(72) Inventeur : **Malen, Charles**
**3 allée Traversière**
**F-94260 Fresnes (FR)**
Inventeur : **de Nanteuil, Guillaume**
**12 rue du Chemin Vert**
**F-92150 Suresnes (FR)**
Inventeur : **Colpaert, Francis**
**36bis boulevard Carnot**
**F-78110 Le Vesinet (FR)**

**EP 0 445 026 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés d'aminométhylpipéridine, leur procédé de préparation et les compositions pharmaceutiques qui les contiennent.

De nombreux dérivés de la pipéridine ont été décrits dans la littérature. On peut citer parmi ceux-ci les composés mentionnés dans les brevets FR 2618435 qui présentent des propriétés antihistaminiques ou bien encore dans le brevet FR 2587029 qui sont utiles dans le traitement des allergies.

Les composés de la présente invention diffèrent de ceux décrits dans l'art antérieur par le fait qu'il s'agit de 4-(aminométhyl)pipéridine substituées en 1 par un hétérocycle.

D'autre part, outre le fait qu'ils soient nouveaux, ils possèdent des propriétés pharmacologiques très intéressantes.

Leur action s'éxerce tant au niveau du système nerveux central qu'au niveau du système nerveux périphérique par le biais des récepteurs $5\text{-}HT_{1A}$ pour lesquels ils présentent une très grande affinité. Cette propriété les rend donc utilisables dans le traitement de la dépression, de l'anxiété, du stress, de la schizophrénie, de la douleur, des maladies cardio-vasculaires et de l'hypertension. Ils peuvent également être utilisés pour modifier le comportement alimentaire et sexuel.

Plus spécifiquement, la présente invention concerne les dérivés de formule (I) :

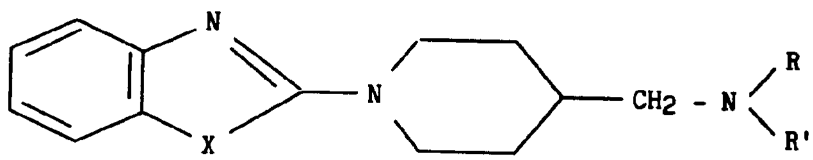

(I)

dans laquelle :

X représente un atome de soufre, un atome d'oxygène, un groupement amino éventuellement substitué par un groupement alkyle inférieur linéaire ou ramifié,

R représente un atome d'hydrogène ou l'un quelconque des groupements suivants :

A

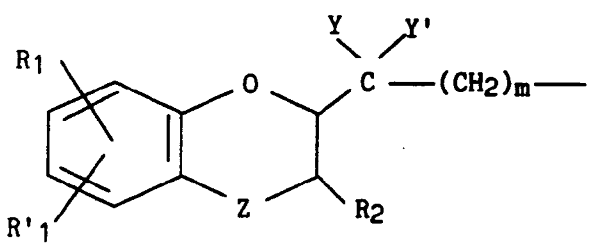

dans lequel :

Z représente un atome d'oxygène ou un groupement $CH_2$,

Y représente un atome d'hydrogène,

Y' représente un atome d'hydrogène,

ou bien, Y et Y' forment ensemble un atome d'oxygène,

$R_1$ représente un atome d'hydrogène, un halogène, un groupement alkyle inférieur linéaire ou ramifié, un groupement alcoxy inférieur linéaire ou ramifié,

$R'_1$ représente un atome d'hydrogène, un halogène, un groupement alcoxy inférieur linéaire ou ramifié,

$R_2$ représente un atome d'hydrogène ou un groupement alkyle inférieur linéaire ou ramifié

m est égal à 0 ou 1,

B

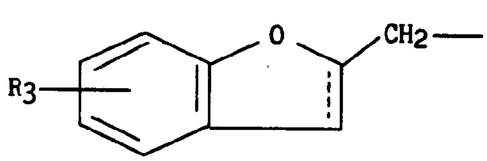

dans lequel

$R_3$ représente un atome d'hydrogène, un groupement alkyle inférieur, linéaire ou ramifié, un halogène,

C 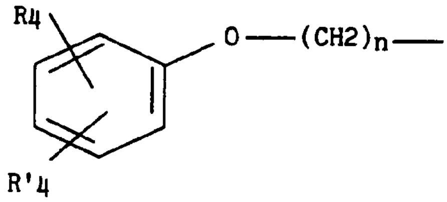

dans lequel :

R$_4$ et R'$_4$ , identiques ou différents représentent un atome d'hydrogène, un halogène ou un groupement alcoxy inférieur, linéaire ou ramifié, ou forment ensemble lorsqu'ils sont situés sur deux carbones adjacents un groupement éthylènedioxy,

n est égal à 2 ou 3,

D 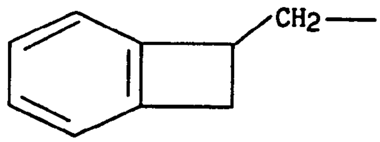

E 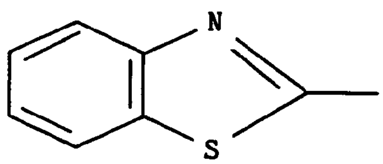

F 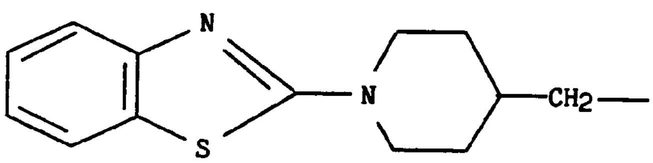

G 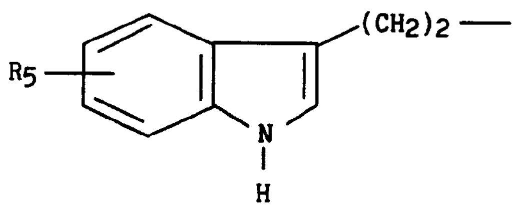

dans lequel:

R$_5$ représente un atome d'hydrogène ou un groupement alkoxy ($C_1$-$C_6$) linéaire ou ramifié,

R' représente un atome d'hydrogène, un groupement alkyle inférieur, linéaire ou ramifié ou l'un quelconque des groupements A, B, C, D, E ou F, avec la condition que R' représente un atome d'hydrogène lorsque R représente le groupement G,

ou bien

R et R' forment avec l'atome d'azote auxquels ils sont attachés un cycle pipérazine non substitué ou substitué sur l'azote libre de la pipérazine par un groupement phényle (non substitué ou substitué par un ou plusieurs atomes d'halogène ou groupements alkyle ($C_1$-$C_6$) ou alkoxy ($C_1$-$C_6$)).

le terme inférieur indiquant que les groupements ainsi qualifiés comptent de 1 à 6 atomes de carbone, le pointillé sur le groupement B indiquant la présence d'une simple ou d'une double liaison,

leurs énantiomères, diastéréoisomères et épimères ainsi que leurs sels d'addition à un acide pharmaceutiquement acceptable.

Parmi les acides pharmaceutiquement acceptables ,on peut citer à titre non limitatif les acides chlorhydrique, sulfurique, tartrique, maléique, fumarique, oxalique, méthane sulfonique, camphorique etc...

L'invention s'étend aussi au procédé de préparation des dérivés de formule (I) caractérisé en ce que l'on

fait réagir sur un dérivé de formule (II) :

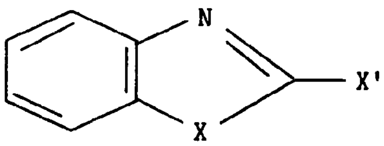

(II)

dans laquelle :

X a la même signification que dans la formule (I) et X' est un atome d'halogène,

1/ soit :

la 4-aminométhylpipéridine de formule (III) :

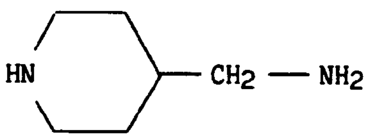

(III)

pour conduire à un dérivé de formule (I/a), cas particulier des dérivés de formule (I) :

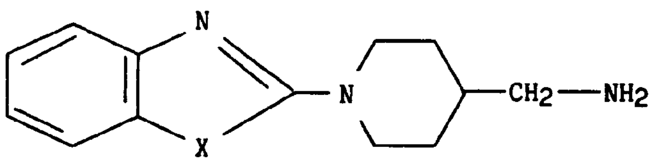

(I/a)

dans laquelle X a la même signification que dans la formule (I), que l'on condense sur un dérivé de formule (IV) :

$$R_1 - L \qquad (IV)$$

dans laquelle $R_1$ représente l'un quelconque des groupements A, B, C, D, E ou F,
L est un groupement libérable tel que le toluène sulfonate, le méthanesulfonate ou un atome d'halogène,
et dont on a éventuellement préparé les isomères par une technique classique de préparation,
pour conduire à un dérivé de formule (I/b), cas particulier des dérivés de formule (I)

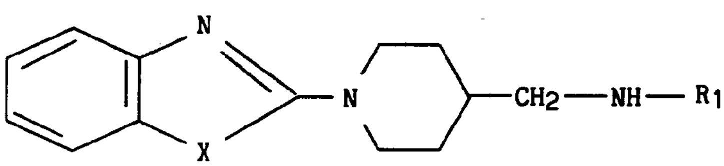

(I/b)

dans laquelle X et $R_1$ ont la même signification que précédemment, dont on sépare éventuellement les isomères par une technique classique de séparation,
que l'on soumet si on le désire,

- ou bien, à l'action d'un composé de formule (V) :

$$R'' - L \qquad (V)$$

dans laquelle L a la même signification que précedemment, et R" est l'un quelconque des groupements A, B, C, D, E ou F, pour conduire à un dérivé de formule (I/c), cas particulier des dérivés de formule (I)

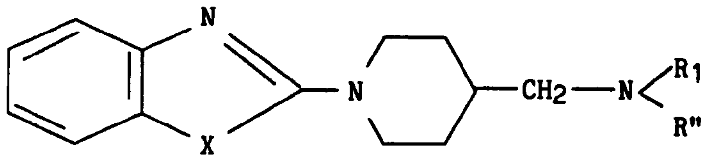

(I/c)

dans laquelle X, $R_1$ et R" ont la même signification que précédemment dont on sépare éventuellement les isomères par une technique classique de séparation,

- ou bien à l'action d'un aldéhyde de formule (VI)

R''' - CHO (VI)

dans laquelle R''' est un groupement alkyle inférieur, linéaire ou ramifié, en présence d'un réducteur tel que le borohydrure de sodium, le cyanoborohydrure de sodium, l'hydrogène, le nickel de Raney ou l'acide formique pour conduire à un dérivé de formule (I/d) :

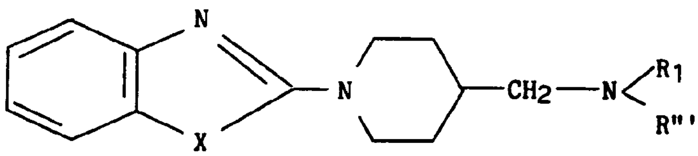

(I/d)

dans laquelle X, $R_1$ et R''' ont la même signification que précedemment, dont on sépare éventuellement les isomères par une technique classique de séparation,

2/ soit :

la 4-hydroxyméthylpipéridine de formule (VII) :

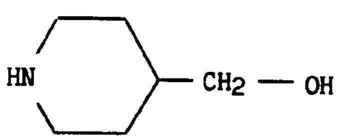

(VII)

pour conduire à un composé de formule (VIII) :

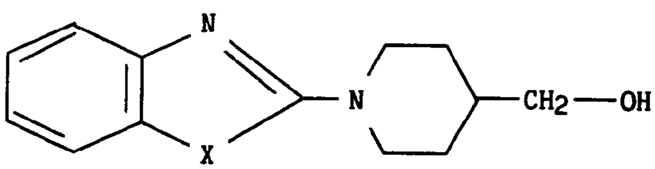

(VIII)

dans laquelle X a la même signification que précédemment
dont on sépare éventuellement les isomères par une technique classique de séparation,
que l'on soumet à l'action du chlorure de tosyle (Cl-Tos),
pour conduire à un composé de formule (IX) :

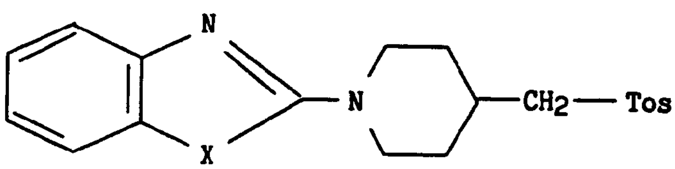

(IX)

dans laquelle X a la même signification que précédemment
que l'on fait réagir :

- ou bien avec un composé de formule (X) :

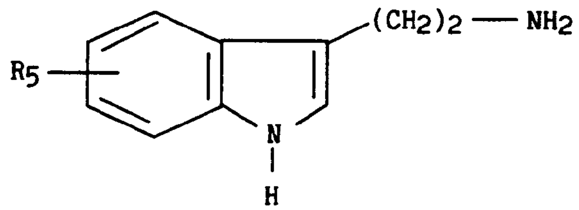

(X)

dans laquelle $R_5$ a la même signification que dans la formule (I)
pour conduire à un composé de formule (I/e), cas particulier des dérivés de formule (I) :

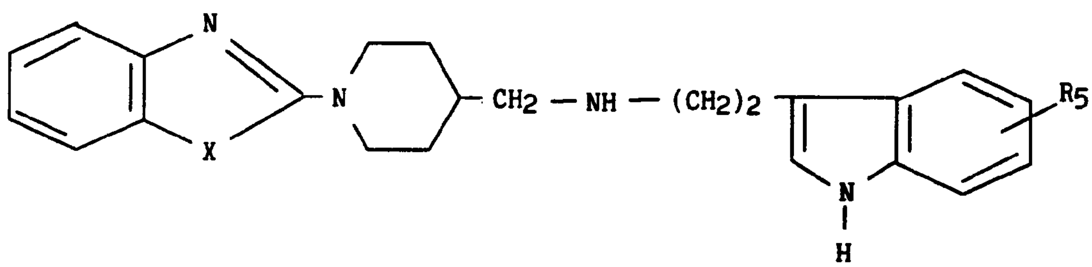

(I/e)

dans laquelle X et $R_5$ ont la même signification que précédemment, dont on sépare éventuellement les isomères par une technique classique de séparation

- ou bien avec une pipérazine de formule (XI) :

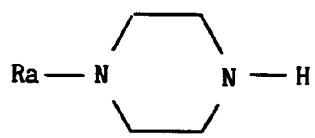

(XI)

dans laquelle Ra représente un atome d'hydrogène ou un groupement phényle non substitué ou substitué par un ou plusieurs atomes d'halogène, ou groupements alkyle ($C_1$-$C_6$) ou alkoxy ($C_1$-$C_6$),
pour conduire à un composé de formule (I/f), cas particulier des dérivés de formule (I) :

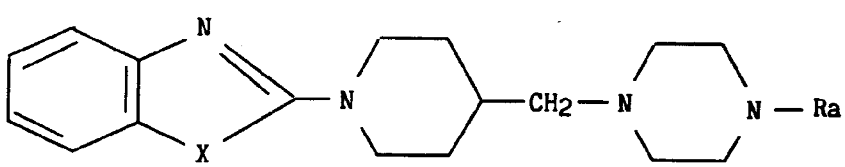

(I/f)

dans laquelle X et Ra ont la même signification que précédemment,
dont on sépare éventuellement les isomères par une technique classique de séparation,
dérivés (I/a), (I/b), (I/c), (I/d), (I/e) et (I/f) que l'on purifie par une technique classique de purification et que l'on transforme, si on le souhaite, en leurs sels d'addition à un acide pharmaceutiquement acceptable.

Les composés de formule (I) possèdent des propriétés pharmacologiques très intéressantes.

Les essais de binding ont montré que les composés de l'invention se comportaient comme de très puissants ligands des récepteurs 5 - $HT_{1A}$, avec une activité agoniste ou antagoniste au niveau du système nerveux central.

Les composés de l'invention trouvent donc leur application dans le traitement du stress (Neuropharmac., 1989, 25, (5), p 471-476), de l'anxiété, de la dépression, de la schizophrénie et de la douleur (Pharmacology and Toxicology, 1989, 64, p 3-5), (Drugs of the Future, 1988, 13, (5), p 429-437), (J. Neural. Transm. 1988, 74, p 195-198).

Les composés actifs au niveau des récepteurs 5 - $HT_{1A}$ peuvent aussi modifier le comportement alimentaire et sexuel (J. Receptor Research, 1988, 8, p 59-81).

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragifiés, les comprimés sublinguaux, les gélules, les suppositoires, les crèmes, les gels dermiques etc...

La posologie utile varie selon l'age et le poids du patient, la nature et la sévérité de l'affection ainsi que la voie d'administration. Celle-ci peut être orale, nasale, rectale, ou parentérale. D'une manière générale, la posologie unitaire s'échelonne entre 0,5 et 500 mg pour un traitement en 1 à 3 prises par 24 heures.

Les exemples suivants illustrent l'invention mais ne la limitent en aucune façon.

**EXEMPLE 1 : 1-(BENZOTHIAZOL-2-YL)-4-AMINOMÉTHYLPIPÉRIDINE, CHLORHYDRATE.**

85 g de 4-aminométhylpipéridine sont placés, à température ambiante dans 750 ml de toluène anhydre en présence de 103 g de carbonate de potassium et de 125 g de 2-chlorobenzothiazole. Ce mélange est porté à reflux pendant 8 heures, puis filtré et la solution évaporée sous vide.

Le résidu est repris par de l'éthanol et après addition d'un excès d'éther chlorhydrique, le précipité formé est essoré et lavé à l'éther. Le produit attendu est obtenu après recristallisation de l'éthanol.

Point de fusion : 155 - 160°C.

Microanalyse élémentaire :

| | C % | H % | N % | S % | Cl % |
|---|---|---|---|---|---|
| calculé : | 48,75 | 5,98 | 13,12 | 10,01 | 22,14 |
| trouvé : | 48,85 | 5,73 | 13,10 | 10,24 | 22,48 |

**EXEMPLE 2 : 1-(BENZOXAZOL-2-YL)-4-AMINOMÉTHYLPIPÉRIDINE.**

45 g de 4-aminométhylpipéridine sont placés, à température ambiante, dans 500 ml de toluène anhydre en présence de 55 g de carbonate de potassium. 50 g de 2-chlorobenzoxazole sont alors additionnés à 5° c.

Le mélange est chauffé 2 heures à 80°c puis filtré et la solution évaporée sous vide.

Le résidu est repris par 100 ml d'éthanol et après addition d'un excès d'éther chlorhydrique, le précipité formé est essoré puis lavé à l'éther. Ce précipité est repris par un litre de soude 1 N. Le produit attendu est alors extrait par du dichlorométhane. Après séchage, la phase organique est filtrée et évaporée. L'huile ainsi obtenue est purifiée par chromatographie sur gel de silice (solvant d'élution : dichlorométhane /méthanol / ammoniaque : 9/1/0,1).

Point de fusion : 64 - 66°C.

Microanalyse élémentaire :

| | C % | H % | N % |
|---|---|---|---|
| calculé : | 67,51 | 7,41 | 18,17 |
| trouvé : | 67,43 | 7,53 | 17,76 |

**EXEMPLE 3 : 1-(BENZIMIDAZOL-2-YL)-4-AMINOMÉTHYLPIPÉRIDINE.**

45 g de 4-aminométhylpipéridine sont placés dans 500 ml de toluène anhydre en présence de 55 g de carbonate de potassium et de 50 g de 2-chlorobenzimidazole. Le mélange est porté une nuit à reflux. Après évaporation et cristallisation du résidu huileux , le produit attendu est obtenu après lavage au dichlorométhane.

Point de fusion : 136 - 138°C.

Microanalyse élémentaire :

| | C % | H % | N % |
|---|---|---|---|
| calculé : | 67,80 | 7,88 | 24,33 |
| trouvé : | 67,32 | 7,90 | 24,28 |

**EXEMPLE 4 : 1-(1-MÉTHYLBENZIMIDAZOL-2-YL)-4-AMINOMÉTHYLPIPÉRIDINE.**

27 g de 4-aminométhylpipéridine sont placés dans 300 ml de toluène anhydre en présence de 33 g de carbonate de potassium et de 33 g de 1-méthyl-2-chlorobenzimidazole. Le mélange est porté 48 heures à reflux. Après évaporation le résidu est repris par de l'éther et un excès d'éther chlorhydrique. Le précipité formé est essoré, lavé à l'éther puis repris par 750 ml d'eau et de la soude à 30 % est additionnée jusqu'à pH basique.

Le produit attendu est alors extrait par du dichlorométhane. Après séchage , la phase organique est filtrée et évaporée. L'huile ainsi obtenue est purifiée par chromatographie sur gel de silice (solvant d'élution : dichlorométhane / méthanol / ammoniaque : 9/1/0,1).
Point de fusion : 106°C.

Microanalyse élémentaire :

| | C % | H % | N % |
|---|---|---|---|
| calculé : | 67,21 | 8,68 | 22,35 |
| trouvé : | 67,51 | 8,02 | 24,12 |

**EXEMPLE 5 : 1-(BENZOTHIAZOL-2-YL)-4-[ (1,4-BENZODIOXAN-2-YL)MÉTHYLAMINOMÉTHYL ] PIPÉRIDINE, DICHLORHYDRATE.**

**STADE A : 1-(-BENZOTHIAZOL-2-YL)-4-AMINOMÉTHYLPIPÉRIDINE.**

Le composé de l'exemple 1 est repris par de la soude 1 N. Le produit attendu est alors obtenu après extraction par du dichlorométhane. Après séchage, la phase organique est filtrée et évaporée. L'huile ainsi obtenue est reprise par de l'hexane bouillant. Après refroidissement, le produit cristallise.
Point de fusion : 65 - 70°C.

Microanalyse élémentaire :

| | C % | H % | N % | S % |
|---|---|---|---|---|
| calculé : | 63,12 | 6,93 | 16,99 | 12,96 |
| trouvé : | 63,69 | 6,19 | 17,08 | 13,01 |

**STADE B : 1-( BENZOTHIAZOL-2-YL)-4-[(1,4-BENZODIOXAN-2-YL) MÉTHYLAMINOMÉTHYL] PIPÉRIDINE, DICHLORHYDRATE.**

9 g du composé préparé au stade A et 9 g de 2-mésyloxyméthyl -1,4-benzodioxane sont placés dans 100 ml d'acétonitrile anhydre en présence de 10 g de carbonate de potassium. Le mélange est porté une nuit à reflux, puis filtré et la solution évaporée sous vide. Le résidu est repris par 100 ml d'acide chlorhydrique 2N et la solution lavée plusieurs fois par de l'éther. La phase aqueuse est alors rendue alcaline par de la soude et extraite par de l'acétate d'éthyle. Après séchage de la phase organique, filtration et évaporation, l'huile ainsi obtenue est purifiée par chromatographie sur gel de silice (solvant d'élution : éther/méthanol : 95/5). Le produit attendu est obtenu après addition d'éther chlorhydrique et précipitation et recristallisation de l'éthanol.
Point de fusion : 263°C.

Microanalyse élémentaire :

| | C % | H % | N % | S % | Cl % |
|---|---|---|---|---|---|
| calculé : | 56,41 | 5,81 | 8,97 | 6,84 | 15,14 |
| trouvé : | 56,01 | 5,78 | 8,98 | 6,92 | 15,04 |

**EXEMPLE 6 : 1-(BENZOTHIAZOL-2-YL)-4-[ (BENZOCYCLOBUTAN-1-YL)-METHYL AMINOMETHYL] PIPÉRIDINE, DICHLORHYDRATE.**

En procédant comme dans l'exemple 5, mais en remplacant au stade B le 2-mésyloxyméthyl-1,4-benzodioxane par le 1-(mésyloxyméthyl) benzocyclobutane (décrit dans Tetrahedron, 1974, 1053), on obtient le produit attendu.
Point de fusion : 235°C.

Microanalyse élémentaire :

| | C % | H % | N % | S % | Cl % |
|---|---|---|---|---|---|
| calculé : | 60,54 | 6,24 | 9,63 | 7,35 | 16,25 |
| trouvé : | 60,56 | 6,26 | 9,78 | 7,56 | 16,28 |

**EXEMPLE 7 : 1-(BENZOTHIAZOL-2-YL)-4-[ (1,4-BENZODIOXAN-2-YL) ACETAMIDOMETHYL] PIPÉRIDINE, CHLORHYDRATE.**

A 0°c, 10 ml de chlorure d'oxalyle sont additionnés sur 2 g d'acide benzodioxan-2-yl carboxylique en solution dans 50 ml d'éther anhydre. Le mélange est abandonné une nuit à température ambiante puis évaporé.Le résidu est repris par 10 ml de toluène anhydre et 2,7 g du composé préparé au stade A de l'exemple 5 sont additionnés à 0°c. Le précipité formé est filtré puis repris par de l'éthanol et après addition d'un excès d'éther chlorhydrique, le produit attendu est obtenu après filtration et recristallisation de l'éthanol.
Point de fusion : 200 - 202°C.

Microanalyse élémentaire :

| | C % | H % | N % | S % | Cl % |
|---|---|---|---|---|---|
| calculé : | 59,25 | 5,42 | 9,42 | 7,19 | 7,95 |
| trouvé : | 58,95 | 5,51 | 9,34 | 7,07 | 7,85 |

**EXEMPLE 8 : 1-(BENZOTHIAZOL-2-YL)-4-[ (CHROMAN-2-YL)-METHYLAMINOMETHYL] PIPÉRIDINE, DICHLORHYDRATE.**

En procédant comme dans l'exemple 5 mais en remplaçant au stade B le 2-mésyloxyméthyl-1,4-benzodioxane par le 2-mésyloxyméthylchromane, on obtient le produit attendu.
Point de fusion : 240°C.

Microanalyse élémentaire :

| | C % | H % | N % | S % | Cl % |
|---|---|---|---|---|---|
| calculé : | 59,22 | 6,27 | 9,01 | 6,87 | 15,20 |
| trouvé : | 59,13 | 6,28 | 9,02 | 7,20 | 15,16 |

**EXEMPLE 9 : 1-(BENZOTHIAZOL-2-YL)-4-[ (7-CHLORO-1,4-BENZODIOXAN-2-YL)METHYLAMINOMETHYL] PIPÉRIDINE, DICHLORHYDRATE.**

En procédant comme dans l'exemple 5 mais en remplaçant au stade B le 2-mésyloxyméthyl-1,4-benzodioxane par le 2-mésyloxyméthyl-7-chloro-1,4-benzodioxane, on obtient le produit attendu.
Point de fusion : 280 - 282°C.

Microanalyse élémentaire :

| | C % | H % | N % | S % | Cl % |
|---|---|---|---|---|---|
| calculé : | 52,54 | 5,21 | 8,36 | 6,38 | 21,15 |
| trouvé : | 52,88 | 5,36 | 8,40 | 6,35 | 20,90 |

**EXEMPLE 10 : 1-(BENZOTHIAZOL-2-YL)-4-[ (5-METHOXY-1,4-BENZODIOXAN-2-YL) METHYLAMINOMETHYL] PIPÉRIDINE, DICHLORHYDRATE.**

En procédant comme dans l'exemple 5 mais en remplaçant au stade B le 2-mésyloxylméthyl-1,4-benzodioxane par le 2-mésyloxyméthyl-5-methoxy-1,4-benzodioxane (décrit dans le J.Am. Chem. Soc 1955, 5373), on obtient le produit attendu.
Point de fusion : 248 - 250°C.

Microanalyse élémentaire :

| | C % | H % | N % | S % | Cl % |
|---|---|---|---|---|---|
| calculé : | 55,42 | 5,86 | 8,43 | 6,43 | 14,22 |
| trouvé : | 55,56 | 5,93 | 8,47 | 6,59 | 14,33 |

**EXEMPLE 11 : 1-(BENZOTHIAZOL-2-YL)-4-[ (3-METHYL-1,4-BENZODIOXAN-2-YL) METHYLAMINOMETHYL] PIPÉRIDINE, DICHLORHYDRATE.**

En procédant comme dans l'exemple 5 mais en remplaçant au stade B le 2-mésyloxyméthyl-1,4-benzodioxane par le 2-mésyloxyméthyl-3-méthyl-1,4-benzodioxane, on obtient le produit attendu.
Point de fusion : 220 - 228°C.

Microanalyse élémentaire :

| | C % | H % | N % | S % | Cl % |
|---|---|---|---|---|---|
| calculé : | 57,26 | 6,06 | 8,71 | 6,65 | 14,70 |
| trouvé : | 57,33 | 6,26 | 8,83 | 6,75 | 14,71 |

**EXEMPLE 12 : 1-(BENZOTHIAZOL-2-YL)-4-[ N-[ (1,4-BENZODIOXAN-2-YL)METHYL]-N-(METHYL)AMINOMETHYL ] PIPÉRIDINE, DICHLORHYDRATE.**

1 g du produit préparé dans l'exemple 5 est relargué par de la soude pour fournir la base correspondante qui est reprise par 7,5 ml d'acétonitrile anhydre. Après addition de 1 ml d'une solution à 40 % de formaldéhyde et de 250 mg de cyanoborohydrure de sodium, le mélange est laissé sous agitation 15 minutes à température ambiante. 1 ml d'acide acétique est alors ajouté et le mélange est à nouveau agité 15 minutes à température ambiante. Après évaporation du solvant, addition de 10 ml de soude 2N, extraction par de l'éther, la phase organique est extraite par de l'acide chlorhydrique 3N. La phase aqueuse ainsi obtenue est alcalinisée par de la soude et à nouveau extraite par de l'éther. Après séchage et filtration, le solvant est évaporé. Le produit attendu est obtenu par précipitation du dichlorhydrate dans de l'éther chlorhydrique.
Point de fusion : 165°C.

Microanalyse élémentaire :

| | C % | H % | N % | S % | Cl % |
|---|---|---|---|---|---|
| calculé : | 57,26 | 6,06 | 8,71 | 6,65 | 14,70 |
| trouvé : | 57,21 | 6,12 | 8,46 | 6,61 | 14,39 |

**EXEMPLE 13 ET 14 :**

**EXEMPLE 13 : 1-(BENZOTHIAZOL-2-YL)-4-[ BENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-chlorométhylbenzofurane et purification de l'huile par chromatographie sur gel de silice (solvant de l'élution : éther), on obtient le produit attendu.
Point de fusion : 241-242°C.

Microanalyse élémentaire :

| | C % | H % | N % | S % | Cl % |
|---|---|---|---|---|---|
| calculé : | 58,66 | 5,59 | 9,33 | 7,12 | 15,74 |
| trouvé : | 58,52 | 5,67 | 9,41 | 7,08 | 15,69 |

**EXEMPLE 14 : 1-(BENZOTHIAZOL-2 -YL)-4-[ DI [(BENZOFURAN-2-YL) METHYL]AMINOMETHYL ] PIPÉRIDINE, DICHLORHYDRATE.**

1 g du produit préparé dans l'exemple 13 est relargué par de la soude pour fournir la base correspondante qui est reprise par de l'acétonitrile anhydre. En procédant comme au stade B de l'exemple 5 mais en remplaçant le 2-mésyloxyméthyl-1,4 benzodioxane par le 2-chlorométhylbenzofurane, on obtient le produit attendu.
Point de fusion : 183 - 185°C.

Microanalyse élémentaire :

| | C % | H % | N % | S % | Cl % |
|---|---|---|---|---|---|
| calculé : | 64,13 | 5,38 | 7,24 | 5,52 | 12,21 |
| trouvé : | 64,03 | 5,48 | 7,15 | 5,52 | 12,26 |

**EXEMPLE 15 : 1-(BENZOTHIAZOL-2-YL)-4-[ [ 2-(2-METHOXYPHENOXY) ETHYL]AMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 1-tosyloxy-2-(2-méthoxyphénoxy)éthane, on obtient le produit attendu.
Point de fusion : 220°C.

Microanalyse élémentaire :

| | C % | H % | N % | S % | Cl % |
|---|---|---|---|---|---|
| calculé : | 56,17 | 6,21 | 8,93 | 6,82 | 15,07 |
| trouvé : | 56,42 | 6,23 | 9,05 | 6,95 | 15,33 |

**EXEMPLE 16 : 1-(BENZOTHIAZOL-2-YL)-4-[(8-METHOXY-1,4-BENZODIOXAN-2-YL)METHYLAMINOMETHYL ] PIPÉRIDINE, DICHLORHYDRATE.**

En procédant comme dans l'exemple 5 mais en remplacant au stade B le 2-mésyloxyméthyl-1,4-benzodioxane par le 2-tosyloxyméthyl-8-méthoxy-1,4-benzodioxane (décrit dans le brevet EP 210581),on obtient le produit attendu.
Point de fusion : 265 - 268°C

Microanalyse élémentaire :

| | C % | H % | N % | S % | Cl % |
|---|---|---|---|---|---|
| calculé : | 55,42 | 5,86 | 8,43 | 6,43 | 14,22 |
| trouvé : | 55,54 | 6,06 | 8,85 | 6,57 | 14,26 |

**EXEMPLE 17 : 1-(BENZOTHIAZOL-2-YL)-4-[ (6-CHLORO-1,4-BENZODIOXAN-2-YL)-METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-6-chloro-1,4-benzodioxane (décrit dans le brevet BE 843 995 ), on obtient le produit attendu.
Point de fusion : 260 - 264°C.

Microanalyse élémentaire :

| | C % | H % | N % | S % | Cl % |
|---|---|---|---|---|---|
| calculé : | 52,54 | 5,21 | 8,36 | 6,38 | 21,15 |
| trouvé : | 52,37 | 5,14 | 8,40 | 6,55 | 21,24 |

**EXEMPLE 18 : 1-(BENZOTHIAZOL-2-YL)-4-[ (7-METHYL-2,3-DIHYDROBENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-7-méthyl-2,3-dihydrobenzofurane, on obtient le produit attendu
Point de fusion : 222 - 224°C.

Microanalyse élémentaire :

| | C % | H % | N % | S % | Cl % |
|---|---|---|---|---|---|
| calculé : | 59,22 | 6,27 | 9,01 | 6,87 | 15,20 |
| trouvé : | 59,09 | 6,22 | 9,05 | 6,93 | 15,25 |

**EXEMPLE 19 : 1-(BENZOTHIAZOL-2-YL)-4-[ (7-CHLORO-2,3-DIHYDROBENZOFURAN-2-YL)METHYLAMINOMETHYL ] PIPÉRIDINE, DICHLORHYDRATE.**

En procédant comme dans l'exemple 5 mais en remplacant au stade B le 2-mésyloxyméthyl-1,4-benzodioxane par le 2-tosyloxyméthyl-7-chloro-2,3-dihydrobenzofurane,on obtient le produit attendu.
Point de fusion : 212 - 214°C.

Microanalyse élémentaire :

| | C % | H % | N % | S % | Cl % |
|---|---|---|---|---|---|
| calculé : | 54,27 | 5,38 | 8,63 | 6,59 | 21,84 |
| trouvé : | 53,97 | 5,45 | 8,76 | 6,66 | 21,98 |

**EXEMPLE 20 : 1-(BENZOTHIAZOL-2-YL)-4-[ (7-FLUORO-2,3-DIHYDROBENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-7-fluoro-2,3-dihydrobenzofurane, on obtient le produit attendu.
Point de fusion : 205 - 208°C.

Microanalyse élémentaire :

| | C % | H % | N % | S % | Cl % |
|---|---|---|---|---|---|
| calculé : | 56,17 | 5,57 | 8,93 | 6,82 | 15,07 |
| trouvé : | 55,95 | 5,59 | 9,15 | 6,78 | 15,31 |

**EXEMPLE 21 : 1-(BENZOTHIAZOL-2-YL)-4-[ (7-ISOPROPYL-2,3-DIHYDROBENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-7-isopropyl-2,3-dihydrobenzofurane, on obtient le produit attendu.
Point de fusion : 218 - 220°C.

Microanalyse élémentaire :

| | C % | H % | N % | S % | Cl % |
|---|---|---|---|---|---|
| calculé : | 60,72 | 6,74 | 8,50 | 6,48 | 14,34 |
| trouvé : | 60,56 | 6,49 | 8,62 | 6,50 | 14,39 |

**EXEMPLE 22 : 1-(BENZOTHIAZOL-2-YL)-4-[ (5-FLUORO-2,3-DIHYDROBENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-5-fluoro-2,3-dihydrobenzofurane, on obtient le produit attendu.
Point de fusion : 223 - 228°C.

Microanalyse élémentaire :

| | C % | H % | N % | S % | Cl % |
|---|---|---|---|---|---|
| calculé : | 56,17 | 5,57 | 8,93 | 6,82 | 15,07 |
| trouvé : | 56,16 | 5,57 | 8,91 | 6,83 | 15,02 |

**EXEMPLE 23 : 1-(BENZOTHIAZOL-2-YL )-4-[ (2,3-DIHYDROBENZOFURAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-2,3-dihydrobenzofurane, on obtient le produit attendu.
Point de fusion : 230 - 235°C.

Microanalyse élémentaire :

| | C % | H % | N % | S % | Cl % |
|---|---|---|---|---|---|
| calculé : | 58,40 | 6,02 | 9,29 | 7,09 | 15,67 |
| trouvé : | 58,46 | 5,78 | 9,18 | 7,21 | 15,72 |

**EXEMPLE 24 : 1-(BENZOTHIAZOL-2-YL)-4-[ (7-METHOXY-1,4-BENZODIOXAN-2-YL)METHYLAMINOMETHYL ]PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-7-méthoxy-1,4-benzodioxane (décrit dans J. Med. Chem, 1965, 8, 446),on obtient le produit attendu.
Point de fusion : 267 - 270°C.

Microanalyse élémentaire :

| | C % | H % | N % | S % | Cl % |
|---|---|---|---|---|---|
| calculé : | 55,42 | 5,86 | 8,43 | 6,43 | 14,22 |
| trouvé : | 55,11 | 5,87 | 8,63 | 6,72 | 14,17 |

**EXEMPLE 25 : 1-(BENZOTHIAZOL-2-YL)-4-[ (6-METHOXY-1,4-BENZODIOXAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-6-méthoxy-1,4-benzodioxane (décrit dans J. Med. Chem, 1965, 8, 446), on obtient le produit attendu.
Point de fusion : 235 - 240°C.

Microanalyse élémentaire :

| | C % | H % | N % | S % | Cl % |
|---|---|---|---|---|---|
| calculé : | 55,42 | 5,86 | 8,43 | 6,43 | 14,22 |
| trouvé : | 55,25 | 5,67 | 7,89 | 6,51 | 14,12 |

**EXEMPLE 26 : 1-(BENZOTHIAZOL-2-YL)-4-[ (BENZOTHIAZOL-2-YL)AMINOMETHYL ] PIPÉRIDINE, DICHLORHYDRATE.**

En procédant comme dans l'exemple 1 mais en plaçant à température ambiante, dans du toluène anhydre, un équivalent de 4-aminométhylpipéridine pour deux équivalents de 2-chlorobenzothiazole, on obtient le produit attendu.
Point de fusion : 190 - 195°C.

Microanalyse élémentaire :

| | C % | H % | N % | S % | Cl % |
|---|---|---|---|---|---|
| calculé : | 52,98 | 4,89 | 12,36 | 14,14 | 15,64 |
| trouvé : | 53,04 | 4,85 | 12,48 | 14,20 | 15,76 |

**EXEMPLE 27 : 1-(BENZOTHIAZOL-2-YL)-4-[ (3-PHENOXYPROPYL)AMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le (1-bromo-3-phenoxy) propane,on obtient le produit attendu.
Point de fusion : 247 - 249°C.

Microanalyse élémentaire :

| | C % | H % | N % | S % | Cl % |
|---|---|---|---|---|---|
| calculé : | 58,14 | 6,43 | 9,25 | 7,06 | 15,60 |
| trouvé : | 58,34 | 6,38 | 9,29 | 7,16 | 15,58 |

**EXEMPLE 28 : 1-(BENZOTHIAZOL-2-YL)-4-[ (6-FLUORO-1,4-BENZODIOXAN-2-YL)METHYLAMINOMETHYL ] PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-6-fluorobenzodioxane (décrit dans le brevet BE 843995 ), on obtient le produit attendu.
Point de fusion : 260 - 265°C.

Microanalyse élémentaire :

| | C % | H % | N % | S % | Cl % |
|---|---|---|---|---|---|
| calculé : | 54,32 | 5,39 | 8,64 | 6,59 | 11,58 |
| trouvé : | 54,65 | 5,40 | 8,41 | 6,56 | 14,44 |

**EXEMPLE 29 : 1-(BENZOTHIAZOL-2-YL)-4-[ [2-(2,6-DIMETHOXY-PHENOXY)-ETHYL ] AMINOMETHYL ]PIPÉRIDINE, DICHLORHYDRATE.**

En procédant comme dans l'exemple 5 mais en replacant au stade B le 2-mésyloxyméthyl-1,4-benzodioxane par le 1-bromo-2-(2,6-diméthoxy-phénoxy) éthane, on obtient le produit attendu.
Point de fusion : 176 - 180°C.

Microanalyse élémentaire :

| | C % | H % | N % | S % | Cl % |
|---|---|---|---|---|---|
| calculé : | 55,20 | 6,24 | 8,40 | 6,41 | 14,17 |
| trouvé : | 54,90 | 5,77 | 8,23 | 6,65 | 14,11 |

**EXEMPLE 30 : 1-(BENZOTHIAZOL-2-YL)-4-[ [2-(4-METHOXY-PHENOXY)-ETHYL]AMINOMETHYL ]PIPÉRIDINE, DICHLORHYDRATE**

En procédant comme dans l'exemple 5 mais en remplacant au stade B le 2-mésyloxyméthyl-1,4-benzodioxane par le 1-bromo-2-(4-méthoxy-phénoxy) éthane, on obtient le produit attendu.
Point de fusion : 203°C.

Microanalyse élémentaire :

| | C % | H % | N % | S % | Cl % |
|---|---|---|---|---|---|
| calculé : | 56,17 | 6,21 | 8,93 | 6,82 | 15,07 |
| trouvé : | 56,17 | 6,21 | 8,99 | 7,03 | 14,98 |

**EXEMPLE 31 : 1-(BENZOTHIAZOL-2-YL)-4-[ [2-(3-METHOXY-PHENOXY)ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-bromo-2-(3-méthoxy-phénoxy) éthane, on obtient le produit attendu.
Point de fusion : 211 - 213°C.

Microanalyse élémentaire :

| | C % | H % | N % | S % | Cl % |
|---|---|---|---|---|---|
| calculé : | 56,17 | 6,21 | 8,93 | 6,82 | 15,07 |
| trouvé : | 56,01 | 6,04 | 8,92 | 6,84 | 15,12 |

**EXEMPLE 32 : 1-(BENZOTHIAZOL-2-YL)-4-[ [(5-CHLORO-1,4-BENZODIOXAN-2-YL)METHYLAMINOMETHYL ]PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-5-chloro-1,4-benzodioxane (décrit dans J. Med. Chem, 1965, 8, 446), on obtient le produit attendu.
Point de fusion : 235 - 238°C.

Microanalyse élémentaire :

| | C % | H % | N % | S % | Cl % |
|---|---|---|---|---|---|
| calculé : | 52,54 | 5,21 | 8,36 | 6,38 | 21,15 |
| trouvé : | 52,77 | 5,25 | 8,32 | 6,51 | 21,14 |

**EXEMPLE 33 : 1-(BENZOTHIAZOL-2-YL)-4-[ (5,6 DICHLORO-1,4-BENZODIOXAN-2-YL)METHYLAMINOMETHYL ]PIPÉRIDINE, DICHLORHYDRATE.**

En procédant comme dans l'exemple 5 mais en remplaçant au stade B le 2-mésyloxyméthyl-1,4-benzodioxane par le 2-tosyloxyméthyl-5,6-dichloro-1,4-benzodioxane (décrit dans J. Med.Chem ,1965, 8,446), on obtient le produit attendu.
Point de fusion : 269 - 272°C.

Microanalyse élémentaire :

| | C % | H % | N % | S % | Cl % |
|---|---|---|---|---|---|
| calculé : | 49,18 | 4,69 | 7,82 | 5,97 | 26,39 |
| trouvé : | 48,89 | 4,81 | 7,69 | 6,00 | 26,64 |

**EXEMPLE 34 : 1-(BENZOTHIAZOL-2-YL)-4-[ [2-(BENZODIOXAN-5-YLOXY)-ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-chloro-2-(1,4-benzodioxan-5-yloxy)éthane (décrit dans le brevet FR 1343644), on obtient le produit attendu.
Point de fusion : 176 - 178°C.

Microanalyse élémentaire :

| | C % | H % | N % | S % | Cl % |
|---|---|---|---|---|---|
| calculé : | 55,42 | 5,86 | 8,43 | 6,43 | 14,22 |
| trouvé : | 55,22 | 6,48 | 8,20 | 6,45 | 14,23 |

**EXEMPLE 35 : 1-(BENZOXAZOL-2-YL)-4-[ (1,4-BENZODIOXAN-2-YL)METHYLAMINOMETHYL]PIPÉRIDINE, DICHLORHYDRATE.**

En procédant comme au stade B de l'exemple 5 mais en remplaçant le 2-mésyloxyméthyl-1,4-benzodioxane par le 2-tosyloxyméthyl-1,4-benzodioxane et le 1-(benzothiazol-2-yl)-4-aminométhylpipéridine par le 1-(benzoxazol-2-yl)-4-aminométhylpipéridine préparé dans l'exemple 2, on obtient le produit attendu.
Point de fusion : 260 - 261°C.

Microanalyse élémentaire :

| | C % | H % | N % | Cl % |
|---|---|---|---|---|
| calculé : | 58,41 | 6,02 | 9,29 | 15,67 |
| trouvé : | 58,37 | 6,07 | 9,40 | 15,59 |

**EXEMPLE 36 : 1-(BENZOTHIAZOL-2-YL)-4-[ (2-PHENOXYETHYL)AMINOMETHYL],PIPÉRIDINE, DICHLORHYDRATE**

En procédant comme dans l'exemple 5 mais en remplaçant au stade B le 2-mésyloxyméthyl-1,4-benzodioxane par le β-bromophénétole,on obtient le produit attendu.
Point de fusion : 240 - 242°C.

Microanalyse élémentaire :

| | C % | H % | N % | S % | Cl % |
|---|---|---|---|---|---|
| calculé : | 57,27 | 6,18 | 9,54 | 7,28 | 16,10 |
| trouvé : | 57,41 | 6,08 | 9,57 | 7,39 | 16,41 |

**EXEMPLE 37 : 1-(BENZOTHIAZOL-2-YL)-4-[ [2-(2-CHLOROPHENOXY)ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-bromo-2-(2-chlorophénoxy)éthane, on obtient le produit attendu.
Point de fusion : 225 - 228°C.

Microanalyse élémentaire :

| | C % | H % | N % | S % | Cl % |
|---|---|---|---|---|---|
| calculé : | 53,11 | 5,52 | 8,85 | 6,75 | 22,40 |
| trouvé : | 53,25 | 5,53 | 8,80 | 6,15 | 22,07 |

**EXEMPLE 38 : 1-(BENZOTHIAZOL-2-YL)-4-[ [2-(3-CHLOROPHENOXY)ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-bromo-2(3-chlorophénoxy)éthane, on obtient le produit attendu.
Point de fusion : 185 - 190°C.

Microanalyse élémentaire :

| | C % | H % | N % | S % | Cl % |
|---|---|---|---|---|---|
| calculé : | 53,11 | 5,52 | 8,85 | 6,75 | 22,40 |
| trouvé : | 53,08 | 5,59 | 8,91 | 6,47 | 22,42 |

**EXEMPLE 39 : 1-(BENZOTHIAZOL-2-YL)-4-[ [2-(4-CHLOROPHENOXY)ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-bromo-2-(4-chlorophénoxy)éthane, on obtient le produit attendu.
Point de fusion : 255 - 260°C.

Microanalyse élémentaire :

| | C % | H % | N % | S % | Cl % |
|---|---|---|---|---|---|
| calculé : | 53,11 | 5,52 | 8,85 | 6,75 | 22,40 |
| trouvé : | 53,09 | 5,42 | 8,86 | 6,72 | 22,04 |

**EXEMPLE 40 : 1-(BENZIMIDAZOL-2-yl)-4-[ (1,4-BENZODIOXAN-2-YL)METHYLAMINOMETHYL]PIPÉRIDINE, DICHLORHYDRATE.**

En procédant comme dans l'exemple 5 mais en remplaçant le 2-mésyloxyméthyl-1,4-benzodioxane par le 2-tosyloxyméthyl-1,4-benzodioxane et le 1-(benzothiazol-2-yl)-4-aminométhylpipéridine par le 1-(benzimidazol-2-yl)-4-aminométhylpipéridine préparé dans l'exemple 3, on obtient le produit attendu.
Point de fusion : > 260°C.

Microanalyse élémentaire :

| | C % | H % | N % | Cl % |
|---|---|---|---|---|
| calculé : | 58,54 | 6,25 | 12,41 | 15,71 |
| trouvé : | 58,87 | 6,22 | 12,37 | 15,79 |

**EXEMPLE 41 : 1-(BENZOTHIAZOL-2-YL)-4-[ [2-(2,6-DICHLOROPHENOXY)ETHYL]AMINOMETHYL]PIPÉRIDINE, DICHLORHYDRATE.**

En procédant comme dans l'exemple 5 mais en remplaçant au stade B le 2-mésyloxyméthyl-1,4-benzodioxane par le 1-bromo-2-(2,6-dichlorophénoxy)éthane, on obtient le produit attendu.
Point de fusion : 212- 216°C.

Microanalyse élémentaire :

| | C % | H % | N % | S % | Cl % |
|---|---|---|---|---|---|
| calculé : | 49,52 | 4,95 | 8,25 | 6,30 | 27,84 |
| trouvé : | 49,23 | 5,05 | 8,24 | 6,63 | 27,94 |

**EXEMPLE 42 : 1-(BENZOXAZOL-2-YL)-4-[ (8-METHOXY-1,4-BENZODIOXAN-2-YL)METHYLAMINOMETHYL]PIPÉRIDINE, DICHLORHYDRATE.**

En procédant commme dans l'exemple 35 mais en remplaçant le 2-tosyloxyméthyl-1,4-benzodioxane par le 2-tosyloxyméthyl-8-méthoxy-1,4-benzodioxane (décrit dans le brevet EP 210581) , on obtient le produit attendu.
Point de fusion : 226 - 228°C.

Microanalyse élémentaire :

| | C % | H % | N % | Cl % |
|---|---|---|---|---|
| calculé : | 57,27 | 6,06 | 8,71 | 14,70 |
| trouvé : | 56,99 | 5,98 | 8,62 | 14,81 |

**EXEMPLE 43 : 1-(BENZIMIDAZOL-2-YL)-4-[ (2,3-DIHYDROBENZOFURAN-2-YL)METHYLAMINOMETHYL]PIPÉRIDINE, DICHLORHYDRATE**

En procédant commme dans l'exemple 40 mais en remplaçant le 2-tosyloxyméthyl-1,4-benzodioxane par le 2-tosyloxyméthyl-2,3-dihydrobenzofurane, on obtient le produit attendu.
Point de fusion : 241 - 245°C.

Microanalyse élémentaire :

| | C % | H % | N % | Cl % |
|---|---|---|---|---|
| calculé : | 60,69 | 6,48 | 12,87 | 16,29 |
| trouvé : | 60,61 | 6,52 | 12,93 | 16,24 |

**EXEMPLE 44 : 1-(BENZOXAZOL-2-YL)-4-[ [2-(2-METHOXYPHENOXY)ETHYL]AMINOMETHYL]PIPÉRIDINE, DICHLORHYDRATE.**

En procédant comme dans l'exemple 35 mais en remplaçant le 2-tosyloxyméthyl-1,4-benzodioxane par le 1-tosyloxy-2-(2-methoxyphénoxy)éthane, on obtient le produit attendu.
Point de fusion : 200 - 202°C.

Microanalyse élémentaire :

| | C % | H % | N % | Cl % |
|---|---|---|---|---|
| calculé : | 58,15 | 6,43 | 9,25 | 15,60 |
| trouvé : | 57,97 | 6,65 | 9,10 | 15,47 |

**EXEMPLE 45 : 1-(BENZIMIDAZOL-2-YL)-4-[ [2-(2-METHOXYPHENOXY)ETHYL]AMINOMETHYL]PIPÉRIDINE, DICHLORHYDRATE.**

En procédant comme dans l'exemple 40 mais en remplaçant le 2-tosyloxyméthyl-1,4-benzodioxane par le 1-tosyloxy-2-(2-méthoxyphénoxy)éthane, on obtient le produit attendu.
Point de fusion : 275 - 280°C.

Microanalyse élémentaire :

| | C % | H % | N % | Cl % |
|---|---|---|---|---|
| calculé : | 58,41 | 6,46 | 12,38 | 15,67 |
| trouvé : | 58,13 | 6,69 | 11,96 | 15,62 |

**EXEMPLE 46 : 1-(BENZIMIDAZOL-2-YL)-4-[ (8-METHOXY-1,4-BENZODIOXAN-2-YL)METHYLAMINOMETHYL]PIPÉRIDINE, DICHLORYDRATE.**

En procédant comme dans l'exemple 40 mais en remplaçant le 2-tosyloxyméthyl-1,4-benzodioxane par le 2-tosyloxyméthyl-8-méthoxy-1,4-benzodioxane (décrit dans le brevet EP 210581 ), on obtient le produit attendu.
Point de fusion : 315 - 317°C.

Microanalyse élémentaire :

| | C % | H % | N % | Cl % |
|---|---|---|---|---|
| calculé : | 57,38 | 6,28 | 11,64 | 14,73 |
| trouvé : | 57,34 | 6,32 | 11,51 | 14,59 |

**EXEMPLE 47 : 1-(1-METHYLBENZIMIDAZOL-2-YL)-4-[(8-METHOXY-1,4-BENZODIOXAN-2-YL]METHYLAMINOMETHYL]PIPERIDINE ,DICHLORHYDRATE.**

En procédant comme dans l'exemple 5 mais en remplaçant le 2-mésyloxyméthyl-1,4-benzodioxane par le 2-tosyloxyméthyl-8-méthoxy-1,4-benzodioxane (décrit dans le brevet EP 210581 ) et le 1-(benzothiazol-2-yl)-4-aminométhylpipéridine par le 1-(1-méthylbenzimidazol-2-yl)-4-aminométhylpipéridine obtenu dans l'exemple 4, on obtient le produit attendu.
Point de fusion : 265 - 267°C.

Microanalyse élémentaire :

| | C % | H % | N % | Cl % |
|---|---|---|---|---|
| calculé : | 58,18 | 6,51 | 11,31 | 14,31 |
| trouvé : | 58,28 | 6,69 | 11,15 | 14,38 |

**EXEMPLE 48 : 1-(1-METHYLBENZIMIDAZOL-2-YL)-4-[ (1,4-BENZODIOXAN-2-YL)METHYLAMINOMETHYL] PIPERIDINE, DICHLORHYDRATE.**

En procédant comme dans l'exemple 47 mais en remplaçant le 2-tosyloxyméthyl-8-méthoxy-1,4-benzodioxane par le 2-tosyloxyméthyl-1,4-benzodioxane , on obtient le produit attendu.
Point de fusion : 253 - 255°C.

Microanalyse élémentaire :

| | C % | H % | N % | Cl % |
|---|---|---|---|---|
| calculé : | 59,36 | 6,50 | 12,04 | 15,23 |
| trouvé : | 59,55 | 6,70 | 11,95 | 15,34 |

**EXEMPLE 49 : 1-(BENZOTHIAZOL-2-YL)-4-[ [ 3-(2,6-DIMETHOXYPHENOXY)PROPYL]AMINOMETHYL] PIPERIDINE, DICHLORHYDRATE.**

En procédant comme dans l'exemple 5 mais en remplaçant au stade B le 2-mésyloxyméthyl-1,4-benzodioxane par le 1-bromo-3-(2,6-diméthoxyphénoxy)propane , on obtient le produit attendu.
Point de fusion : 205 - 207°C.

Microanalyse élémentaire :

| | C % | H % | N % | S % | Cl % |
|---|---|---|---|---|---|
| calculé : | 56,03 | 6,46 | 8,17 | 6,23 | 13,78 |
| trouvé : | 55,71 | 6,71 | 8,06 | 6,35 | 13,90 |

**EXEMPLE 50 : 1-(1-METHYLBENZIMIDAZOL-2-YL)-4-[ [2-(2-METHOXYPHENOXY)ETHYL]AMINOMETHYL]PIPERIDINE, DICHLORHYDRATE.**

En procédant comme dans l'exemple 47 mais en remplaçant au stade B le 2-tosyloxyméthyl-8-méthoxy-1,4-benzodioxane par le 1-tosyloxy-2-(2-méthoxyphénoxy)éthane , on obtient le produit attendu.
Point de fusion : 270 - 274°C.

Microanalyse élémentaire :

| | C % | H % | N % | Cl % |
|---|---|---|---|---|
| calculé : | 59,10 | 6,90 | 11,99 | 15,17 |
| trouvé : | 59,24 | 7,01 | 12,01 | 15,10 |

**EXEMPLE 51 : 1-(BENZOTHIAZOL-2-YL)-4-[ (8-CHLORO-1,4-BENZODIOXAN-2-YL)METHYLAMINOMETHYL]PIPERIDINE, DICHLORHYDRATE.**

En procédant comme dans l'exemple 5 mais en remplaçant au stade B le 2-mésyloxyméthyl-1,4-benzodioxane par le 2-tosyloxyméthyl-8-méthoxy-1,4-benzodioxane (décrit dans J.Med. Chem. 1965, 8, 446), on obtient le produit attendu.
Point de fusion : 220 - 222°C.

Microanalyse élémentaire :

| | C % | H % | N % | S % | Cl % |
|---|---|---|---|---|---|
| calculé : | 52,54 | 5,21 | 8,36 | 6,44 | 21,15 |
| trouvé : | 52,63 | 5,24 | 8,03 | 6,38 | 21,08 |

**EXEMPLE 52 : 1-(BENZOTHIAZOL-2-YL)-4-[[2-(1,4-BENZODIOXAN-2-YL)ETHYL]AMINOMETHYL]PIPERIDINE, DICHLORHYDRATE.**

En procédant comme dans l'exemple 5 mais en remplaçant au stade B le 2-mésyloxyméthyl-1,4-benzodioxane par le 2-tosyloxyéthyl-1,4-benzodioxane, on obtient le produit attendu.
Point de fusion : 260 - 264°C.

Microanalyse élémentaire :

| | C % | H % | N % | S % | Cl % |
|---|---|---|---|---|---|
| calculé : | 57,26 | 6,06 | 8,71 | 6,65 | 14,70 |
| trouvé : | 57,16 | 5,95 | 8,62 | 6,86 | 14,81 |

**EXEMPLE 53 : N,N-DI[[1-(BENZOTHIAZOL-2-YL)PIPERIDIN-4-YL]METHYL]AMINE, TRICHLORHYDRATE**

**STADE A : 1-(BENZOTHIAZOL-2-YL)-4-HYDROXYMÉTHYLPIPÉRIDINE**

30 g de 4-hydroxyméthyl pipéridine, 44 g de 2-chlorobenzothiazole et 72 g de carbonate de potassium sont mis en réaction dans 600 ml d'acétonitrile à reflux pendant une nuit.

Après filtration et évaporation du solvant, le résidu est repris par 200 ml d'acétate d'éthyle et extrait par de l'acide chlorhydrique 1N.

La phase aqueuse est alcalinisée par de la soude et extraite par du dichlorométhane. Le produit attendu est obtenu par séchage et évaporation.
Point de fusion : 76 - 80°C

**STADE B : 1-(BENZOTHIAZOL-2-YL)-4-TOSYLOXYMÉTHYLPIPÉRIDINE**

Après dissolution du composé obtenu au stade A dans de la piridine et addition d'un équivalent de chlorure de tosyle, le mélange réactionnel est agité une nuit à température ambiante. Le produit attendu est obtenu après lavage à l'acide chlorhydrique dilué et à l'eau.
Point de fusion : 184°C

**STADE C : N,N-DI[[1-BENZOTHIAZOL-2-YL)PIPÉRIDIN-4-YL]MÉTHYL]AMINE, TRICHLORHYDRATE**

En procédant comme dans l'exemple 5 mais en remplaçant au stade B, le 2-mésyloxyméthyl-1,4-benzodioxane par le 1-(benzothiazol-2-yl)-4-tosyloxyméthylpipéridine obtenu au stade précédent, on obtient le produit attendu.
Point de fusion : 265 - 270°C.

Microanalyse élémentaire :

| | C % | H % | N % | S % | Cl % |
|---|---|---|---|---|---|
| calculé : | 53,19 | 5,84 | 11,93 | 10,92 | 18,12 |
| trouvé : | 53,62 | 5,96 | 11,92 | 10,90 | 17,72 |

**EXEMPLE 54 : 1-(BENZOXAZOL-2-YL)-4-[(2,3-DIHYDROBENZOFURAN-2-YL)METHYLAMINOMETHYL]PIPERIDINE, DICHLORHYDRATE.**

En procédant comme dans l'exemple 35 mais en remplaçant au stade B le 2-tosyloxyméthyl-1,4-benzodioxane par le 2-tosyloxyméthyl-2,3-dihydrobenzofurane, on obtient le produit attendu.
Point de fusion : 228 - 230°C.

Microanalyse élémentaire :

| | C % | H % | N % | Cl % |
|---|---|---|---|---|
| calculé : | 60,55 | 6,24 | 9,63 | 16,25 |
| trouvé : | 60,62 | 6,43 | 9,58 | 15,94 |

**EXEMPLE 55 : 1-(BENZOTHIAZOL-2-YL)-4-[(5,8-DIMETHOXY-1,4-BENZODIOXAN-2-YL)METHYLAMINOMETHYL]PIPERIDINE, DICHLORHYDRATE.**

En procédant comme dans l'exemple 5 mais en remplacant au stade B le 2-mésyloxyméthyl-1,4-benzodioxane par le 2-tosyloxyméthyl-5,8-diméthoxy-1,4-benzodioxane (décrit dans J.Chem. Soc. Perkin. Trans. **I**, 1987, 2017-22), on obtient le produit attendu.
Point de fusion : 240 - 245°C.

Microanalyse élémentaire :

| | C % | H % | N % | S % | Cl % |
|---|---|---|---|---|---|
| calculé : | 54,54 | 5,91 | 7,95 | 6,07 | 13,42 |
| trouvé : | 54,62 | 5,86 | 7,82 | 6,09 | 13,34 |

**EXEMPLE 56 : 1-(2-MÉTHOXYPHÉNYL)-4-[[1-(BENZOTHIAZOL-2-YL)PIPÉRIDIN-4-YL]MÉTHYL]PIPÉRAZINE**

6 g du composé décrit au stade B de l'exemple 53, 2,9 g de 1-(2-méthoxyphényl)pipérazine et 4,8 g de carbonate de potassium sont mis en réaction dans 90 ml d'acétonitrile à reflux pendant une nuit. Après filtration et évaporation des solvants, le résidu est repris par 20 ml d'acide chlorhydrique 2N et la solution est lavée plusieurs fois par de l'éther. La phase aqueuse rendue alcaline par de la soude est extraite par du chlorure de méthylène. Le produit attendu est obtenu après séchage et évaporation du solvant.
Point de fusion : 150 - 152°C

Microanalyse élémentaire :

| | C % | H % | N % | S % |
|---|---|---|---|---|
| calculé : | 68,21 | 7,16 | 13,26 | 7,59 |
| trouvé : | 68,15 | 7,32 | 13,33 | 7,51 |

**EXEMPLE 57 : 1-(BENZOTHIAZOL-2-YL)-4-[2-[(INDOL-3-YL)ÉTHYL]AMINOMÉTHYL] PIPÉRIDINE, DICHLORHYDRATE**

En procédant comme dans l'exemple 56 mais en remplaçant la 1-(2-méthoxyphényl)pipérazine par la tryptamine, on obtient le produit attendu sous forme de base qui est transformée en dichlorhydrate par addition d'éther chlorhydrique et cristallisé dans de l'éthanol.
Point de fusion : 278 - 282°C

Microanalyse élémentaire :

| | | C % | H % | N % | S % | Cl % |
|---|---|---|---|---|---|---|
| calculé | : | 59,61 | 6,09 | 12,09 | 6,92 | 15,30 |
| trouvé | : | 59,23 | 6,13 | 11,96 | 6,91 | 15,03 |

**EXEMPLE 58 : 1-(BENZOTHIAZOL-2-YL)-4-[[2-(5-METHOXYINDOL-3-YL)ÉTHYL]AMINO MÉTHYL]PIPÉRIDINE, DICHLORHYDRATE**

En procédant comme dans l'exemple 57 mais en remplaçant la tryptamine par la 5-méthoxytryptamine, on obtient le produit attendu.
Point de fusion : 250 - 254°C

Microanalyse élémentaire :

| | | C % | H % | N % | S % | Cl % |
|---|---|---|---|---|---|---|
| calculé | : | 58,29 | 6,32 | 11,33 | 6,48 | 14,34 |
| trouvé | : | 58,48 | 6,36 | 11,25 | 6,53 | 14,13 |

**EXEMPLE 59 : (+)-1-(BENZOTHIAZOL-2-YL)-4-[(1,4-BENZODIOXAN-2-YL) MÉTHYL AMINOMÉTHYL]PIPÉRIDINE**

**STADE A : (+)-2-HYDROXYMÉTHYL-1,4-BENZODIOXANE**

En procédant, selon la technique décrite par A. DELGADO et coll. (Tetrahedron letters, 1988, 3671-4), à partir de catéchol et de tosylate de S-(+)-glycidyl, on obtient le produit attendu.
Point de fusion : 70°

**STADE B : (+)-2-TOSYLMÉTHYL-1,4-BENZODIOXANE**

Après dissolution du composé obtenu au stade A dans de la pyridine et addition d'un équivalent de chlorure de tosyle, le mélange est agité 48 heures à température ambiante. Le produit attendu est obtenu après lavage par de l'acide chlorhydrique dilué, puis par du bicarbonate de sodium et enfin par de l'eau.
Point de fusion : 60°C

**STADE C : (+)-1-BENZOTHIAZOL-2-YL)4-[(1,4-BENZODIOXAN-2-YL)MÉTHYLAMINO MÉTHYL] PIPÉRIDINE**

En procédant comme au stade B de l'exemple 5, mais en remplaçant le 2-mésyloxyméthyl-1,4-benzodioxane par le produit obtenu au stade précédent, on obtient le produit attendu sous forme de base.
Point de fusion : 132°C
Pouvoir rotatoire : $\alpha_D^{20°C} = +89,8°$ (C = 10 mg/ml/$CHCl_3$)

Microanalyse élémentaire :

| | | C % | H % | N % | S % |
|---|---|---|---|---|---|
| calculé | : | 66,81 | 6,37 | 10,62 | 8,11 |
| trouvé | : | 66,44 | 6,49 | 10,78 | 8,25 |

**EXEMPLE 60 : (-)-1-(BENZOTHIAZOL-2-YL)-4-[(1,4-BENZODIOXAN-2-YL)MÉTHYL AMINOMÉTHYL] PIPÉRIDINE**

En procédant comme dans l'exemple 59 mais en remplaçant au stade A le tosylate de (S)-(+)-glycidyl par le tosylate de (R)-(-)-glycidyl, on obtient le produit attendu.
Point de fusion : 131°C
Pouvoir rotatoire : $\alpha_D^{20°C}$= - 89,9° (C = 10 mg/ml/$CHCl_3$)

Microanalyse élémentaire :

| | | C % | H % | N % | S % |
|---|---|---|---|---|---|
| calculé | : | 66,81 | 6,37 | 10,62 | 8,11 |
| trouvé | : | 66,57 | 6,30 | 10,70 | 8,20 |

**ETUDE PHARMACOLOGIQUE DES DERIVES DE L'INVENTION**

**EXEMPLE 61 : TEST D'AFFINITE POUR LES RECEPTEURS 5-$HT_{1A}$ IN VITRO**

L'affinité des composés de l'invention pour les récepteurs 5 - $HT_{1A}$ a été mesurée par rapport à celle de la 8-hydroxy-2-(dipropylamino)tétraline (8-OH-DPAT) qui présente une très grande affinité pour les sites 5-$HT_{1A}$ associée à une grande sélectivité.

Les tests sont réalisés sur du tissu de l'hippocampe de rats Wistar mâles décapités. Les rats sont sacrifiés 48 heures avant l'expérience et les tissus isolés sont conservés à -86° C.

Pour la préparation des membranes, les tissus sont homogénéisés, à 0° C, dans 20 volumes d'une solution tampon 50 mM Tris HCl (pH = 7,7 ajusté avec HCl 5N à 25° C), avec un homogénéisateur Polytron pendant 6 secondes. L'ensemble est ensuite centrifugé (35000 g pendant 20 minutes à 4°C). Cette opération d'homogénéisation, centrifugation est renouvelée une deuxième fois dans les mêmes conditions. Le culot ainsi obtenu est mis en suspension une dernière fois dans 20 volumes du tampon précédent, incubé 15 minutes à 37°C puis à nouveau centrifugé. Le culot final est alors mis en suspension dans 100 volumes de tampon d'incubation (Tris 50 mM, pargyline 10 µM, CaCl2 4mM, acide ascorbique 0,1 % (m/v), pH ajusté à 7,7 avec HCl 5N à 25°C).

Les composés à tester sont mis en solution dans le tampon d'incubation. Les solutions essais sont préparées en plaçant dans des tubes de verre 12 X 75 mm, 100 µl de la solution de composés à tester, 100 µl d'une solution contenant 0,4 mM de [3H] 8-OH-DPAT (radioactivité spécifique 205 Ci/mmol). Le binding non spécifique est déterminé à l'aide d'une solution de 5-hydroxytryptamine (10 µM) et représente 5 à 10 % du binding final. Les tubes sont incubés 30 minutes à 37°C. Les solutions sont alors filtrées sur filtres en fibre de verre prétraités avec 0,1 % de polyéthyleneimine. Les filtres sont alors rincés deux fois avec 5 ml de tampon d'incubation à 4°C puis placés dans des "vials" à scintillation dans lesquels est ajouté du fluide à scintillation "Picofluor". La radioactivité est alors déterminée à l'aide d'étalons externes.

Les valeurs des pKi sont déterminées par l'équation de Cheung-Prusoff :

$$pKi = -\log \left( \frac{IC\ 50}{1 + ( [3H]\ 8\text{-}OH\text{-}DPAT/Kd)} \right)$$

Les composés de l'invention présentent une grande affinité pour les sites 5-$HT_{1A}$ . Le composé de l'exemple 16 possède un pKi égal à 9,3, celui de l'exemple 44 est égal à 9,0.

**EXEMPLE 62 : TEST DE "TAIL-FLICKS" IN VIVO**

Le potentiel d'action in vivo, des composés de l'invention, sur les récepteurs 5-$HT_{1A}$ a été mis en évidence selon une méthode mise au point par Millan et Coll. (Neurosci. Lett, 1989, 107, 227-232). L'injection sous cutanée de 8-OH-DPAT induit chez le rat, spontanément, sous vide, des mouvements de la queue importants. Ce modèle est alors utilisé pour évaluer le potentiel d'intéraction sur les récepteurs 5-$HT_{1A}$ chez le rat des composés de l'invention, par injection sous cutanée. L'$ED_{50}$ , c'est à dire la dose réduisant de 50 % l'action du 8-OH-DPAT est alors déterminée.

Celle-ci est égale par exemple, à 0,31 mg/Kg pour les composés des exemples 16 et 44 ce qui implique que, dans ce test, ces composés possèdent des propriétés antagonistes aux récepteurs 5-$HT_{1A}$.

**EXEMPLE 63 : COMPOSITION PHARMACEUTIQUE**

Comprimé : formule de préparation pour 1000 comprimés dosés à 2 mg de principe actif.

| | |
|---|---|
| 1-(benzothiazol-2-yl)-4-[(8-méthoxy-1,4-benzodioxan-2-yl)méthylaminométhyl] pipéridine, dichlorhydrate | 2 g |
| Hydroxypropyl cellulose | 2 g |
| Amidon de blé | 10 g |
| Lactose | 100 g |
| Stéarate de magnésium | 3 g |
| Talc | 3 g |

## Revendications

**Revendications pour les Etats contractants suivants : AT, BE, CH, DE, DK, FR, GB, IT, LI, LU, NL, SE**

**1.** Composés de formule générale (I) :

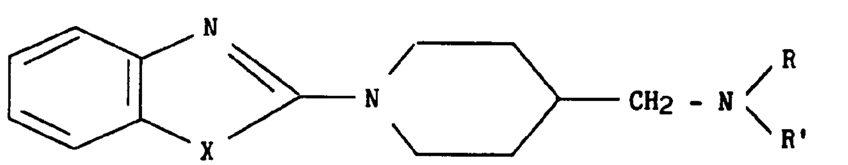

(I)

dans laquelle :

- X représente un atome de soufre, un atome d'oxygène, un groupement amino éventuellement substitué par un groupement alkyle inférieur linéaire ou ramifié,
- R représente un atome d'hydrogène ou l'un quelconque des groupements suivants :

A

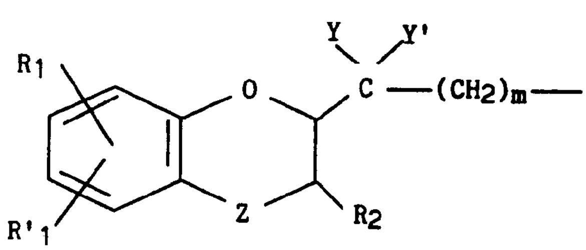

dans lequel :

- Z représente un atome d'oxygène ou un groupement $CH_2$,
- Y représente un atome d'hydrogène,
- Y' représente un atome d'hydrogène,
- ou bien, Y et Y' forment ensemble un atome d'oxygène,
- $R_1$ représente un atome d'hydrogène, un halogène, un groupement alkyle inférieur linéaire ou ramifié, un groupement alcoxy inférieur linéaire ou ramifié,
- $R'_1$ représente un atome d'hydrogène, un halogène, un groupement alcoxy inférieur linéaire ou ramifié,
- $R_2$ représente un atome d'hydrogène ou un groupement alkyle inférieur linéaire ou ramifié,
- m est égal à 0 ou 1,

B 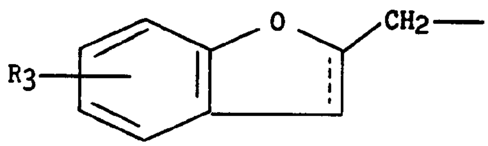

dans lequel $R_3$ représente un atome d'hydrogène, un groupement alkyle inférieur, linéaire ou ramifié, un halogène,

C 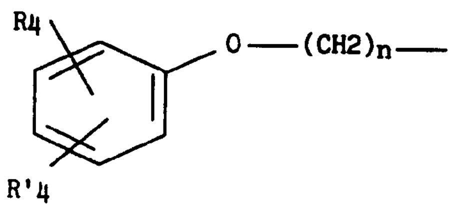

dans lequel :

- $R_4$ et $R'_4$ , identiques ou différents représentent un atome d'hydrogène, un halogène ou un groupement alcoxy inférieur, linéaire ou ramifié, ou forment ensemble lorsqu'ils sont situés sur deux carbones adjacents un groupement éthylènedioxy,
- n est égal à 2 ou 3,

D 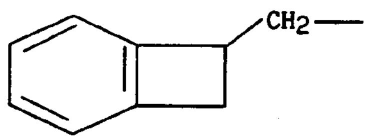

E 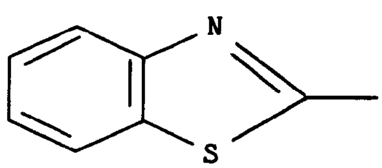

F 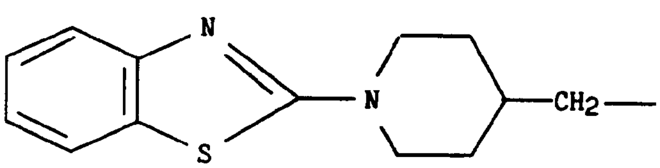

G 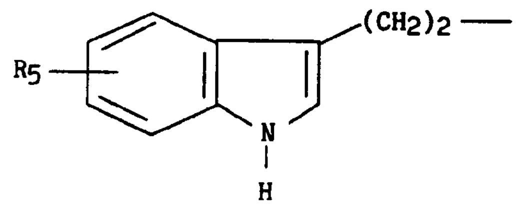

dans lequel :

$R_5$ représente un atome d'hydrogène ou un groupement alkoxy ($C_1$-$C_6$) linéaire ou ramifié,

R' représente un atome d'hydrogène, un groupement alkyle inférieur, linéaire ou ramifié ou l'un quelconque des groupements A, B, C, D, E ou F, avec la condition que R' représente un atome d'hydrogène lorsque R représente le groupement G,

ou bien

R et R' forment avec l'atome d'azote auxquels ils sont attachés un cycle pipérazine non substitué ou substitué sur l'azote libre de la pipérazine par un groupement phényle (non substitué ou substitué par un ou plusieurs atomes d'halogène ou groupements alkyle ($C_1$-$C_6$) ou alkoxy ($C_1$-$C_6$)),

le terme inférieur indiquant que les groupements ainsi qualifiés comptent de 1 à 6 atomes de carbone, le pointillé sur le groupement B indiquant la présence d'une simple ou d'une double liaison,

leurs énantiomères, diastéréoisomères et épimères ainsi que leurs sels d'addition à un acide pharmaceutiquement acceptable.

2. Composés selon la revendication 1 tels que X représente un atome de soufre.

3. Composés selon la revendication 1 tels que X représente un atome d'oxygène.

4. Composés selon la revendication 1 tels que X représente un groupement amino éventuellement substitué par un groupement alkyle inférieur linéaire ou ramifié.

5. Composés selon la revendication 1 tels que R représente un groupement (1,4-benzodioxan-2-yl)méthyl éventuellement substitué.

6. Composés selon la revendication 1 tels que R représente un groupement phénoxyéthyl éventuellement substitué.

7. Composés selon la revendication 1 tels que R représente un groupement (indol-3-yl) éthyl éventuellement substitué, leurs énantiomères, diastéréoisomères et épimères ainsi que leurs sels d'addition à un acide pharmaceutiquement acceptable.

8. Composé selon l'une quelconque des revendications 1, 2 ou 6 qui est la 1-(benzothiazol-2-yl)-4-[[2-(2-méthoxyphénoxy)éthyl]aminométhyl] pipéridine, ses énantiomères ainsi que ses sels d'addition à un acide pharmaceutiquement acceptable.

9. Composé selon l'une quelconque des revendications 1, 2 ou 5 qui est la 1-(benzothiazol-2-yl)-4-[(8-méthoxy-1,4-benzodioxan-2-yl) méthyl aminométhyl] pipéridine, ses énantiomères et diastéréoisomères ainsi que ses sels d'addition à un acide pharmaceutiquement acceptable.

10. Composé selon l'une quelconque des revendications 1, 3 ou 6 qui est la 1-(benzoxazol-2-yl)-4-[[2-(2-méthoxyphénoxy)éthyl]aminométhyl] pipéridine, ses énantiomères ainsi que ses sels d'addition à un acide pharmceutiquement acceptable.

11. Composé selon l'une quelconque des revendications 1, 4 ou 6 qui est la 1-(benzimidazol-2-yl)-4-[[2-(2-méthoxyphénoxy)éthyl] aminométhyl]pipéridine, ses énantiomères ainsi que ses sels d'addition à un acide pharmaceutiquement acceptable.

12. Composé selon l'une quelconque des revendications 1, 2 ou 7 qui est la 1-(benzothiazol-2-yl)-4-[[2-(5-méthoxyindol-3-yl)éthyl]aminométhyl]pipéridine, ses énantiomères ainsi que ses sels d'addition à un acide pharmaceutiquement acceptable.

**13.** Procédé de préparation des dérivés de formule (I) selon la revendication 1 caractérisé en ce que l'on fait réagir sur un dérivé de formule (II) :

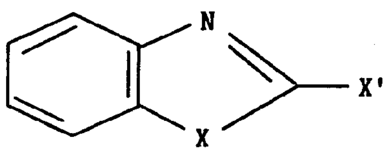

(II)

dans laquelle :

X a la même signification que dans la formule (I) et X' est un atome d'halogène,

1/ soit :

la 4-aminométhylpipéridine de formule (III) :

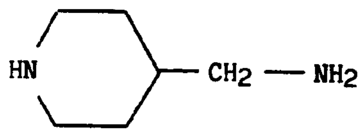

(III)

pour conduire à un dérivé de formule (I/a), cas particulier des dérivés de formule (I) :

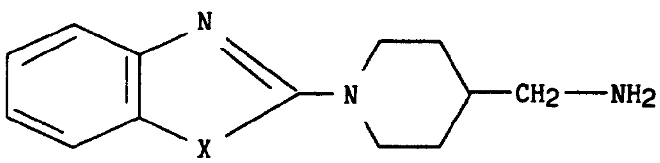

(I/a)

dans laquelle X a la même signification que dans la formule (I), que l'on condense sur un dérivé de formule (IV) :

$$R_1 - L \qquad (IV)$$

dans laquelle $R_1$ représente l'un quelconque des groupements A, B, C, D, E ou F,

L est un groupement libérable tel que le toluène sulfonate, le méthanesulfonate ou un atome d'halogène, et dont on a éventuellement préparé les isomères par une technique classique de préparation,

pour conduire à un dérivé de formule (I/b), cas particulier des dérivés de formule (I)

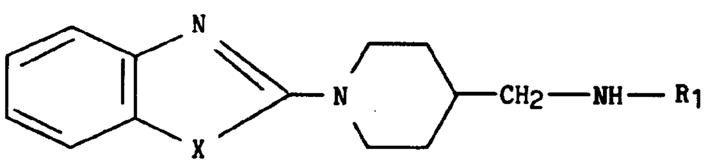

(I/b)

dans laquelle X et $R_1$ ont la même signification que précédemment, dont on sépare éventuellement les isomères par une technique classique de séparation,

que l'on soumet si on le désire,

- ou bien, à l'action d'un composé de formule (V) :

$$R'' - L \qquad (V)$$

dans laquelle L a la même signification que précedemment, et R" est l'un quelconque des groupements A, B, C, D, E ou F, pour conduire à un dérivé de formule (I/c), cas particulier des dérivés de formule (I)

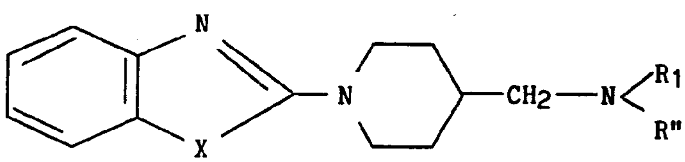

(I/c)

dans laquelle X, $R_1$ et R" ont la même signification que précédemment dont on sépare éventuellement les isomères par une technique classique de séparation,

- ou bien à l'action d'un aldéhyde de formule (VI)

R"' - CHO (VI)

dans laquelle R"' est un groupement alkyle inférieur, linéaire ou ramifié, en présence d'un réducteur tel que le borohydrure de sodium, le cyanoborohydrure de sodium, l'hydrogène, le nickel de Raney ou l'acide formique pour conduire à un dérivé de formule (I/d) :

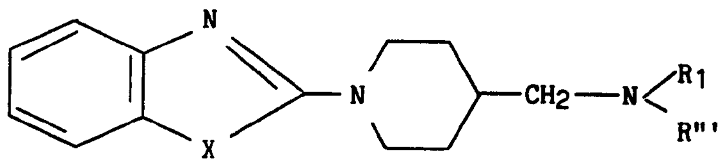

(I/d)

dans laquelle X, $R_1$ et R"' ont la même signification que précédemment, dont on sépare éventuellement les isomères par une technique classique de séparation,

2/ <u>soit</u> :

la 4-hydroxyméthylpipéridine de formule (VII) :

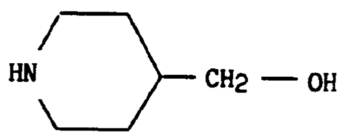

(VII)

pour conduire à un composé de formule (VIII) :

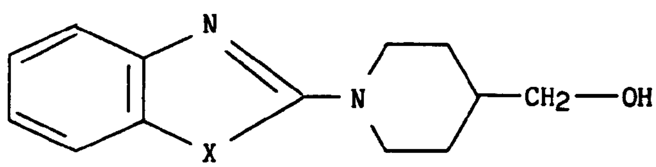

(VIII)

dans laquelle X a la même signification que précédemment
dont on sépare éventuellement les isomères par une technique classique de séparation,
que l'on soumet à l'action du chlorure de tosyle (Cl-Tos),
pour conduire à un composé de formule (IX) :

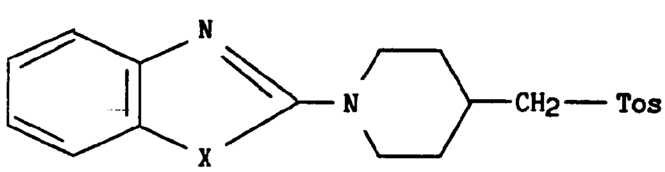

(IX)

dans laquelle X a la même signification que précédemment que l'on fait réagir :

- <u>ou bien</u> avec un composé de formule (X) :

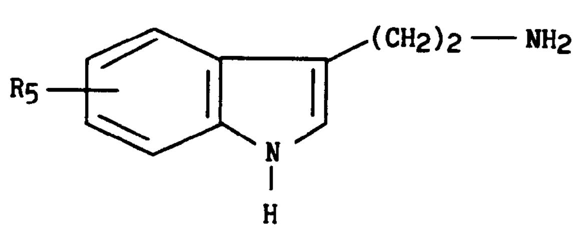

(X)

dans laquelle $R_5$ a la même signification que dans la formule (I) pour conduire à un composé de formule (I/e), cas particulier des dérivés de formule (I) :

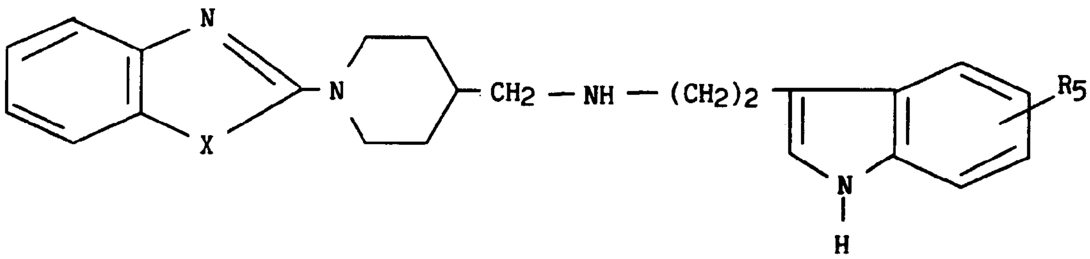

(I/e)

dans laquelle X et $R_5$ ont la même signification que précédemment, dont on sépare éventuellement les isomères par une technique classique de séparation

- ou bien avec une pipérazine de formule (XI) :

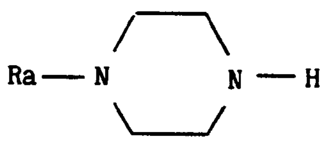

(XI)

dans laquelle Ra représente un atome d'hydrogène ou un groupement phényle non substitué ou substitué par un ou plusieurs atomes d'halogène, ou groupements alkyle ($C_1$-$C_6$) ou alkoxy ($C_1$-$C_6$), pour conduire à un composé de formule (I/f), cas particulier des dérivés de formule (I) :

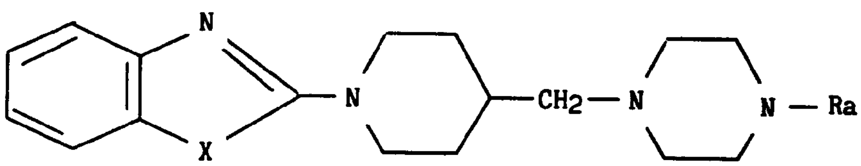

(I/f)

dans laquelle X et Ra ont la même signification que précédemment, dont on sépare éventuellement les isomères par une technique classique de séparation, dérivés (I/a), (I/b), (I/c), (I/d), (I/e) et (I/f) que l'on purifie par une technique classique de purification et que l'on transforme, si on le souhaite, en leurs sels d'addition à un acide pharmaceutiquement acceptable.

14. Compositions pharmaceutiques contenant comme principe actif au moins un composé selon l'une quelconque des revendications 1 à 12 , seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une des revendications 1 à 12 utiles pour le traitement de la dépression, du stress, des psychoses, d'anxiété, de la douleur, de la schizophrénie, de l'hypertension et des maladies cardio-vasculaires, et comme modificateur du comportement alimentaire et sexuel.

**Revendications pour l'Etat contractant suivant : ES**

1. Procédé de préparation des composés de formule générale (I) :

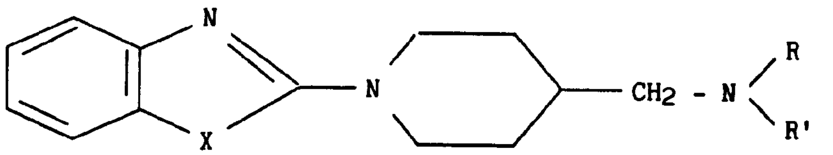

(I)

dans laquelle :

- X représente un atome de soufre, un atome d'oxygène, un groupement amino éventuellement substitué par un groupement alkyle inférieur linéaire ou ramifié,
- R représente un atome d'hydrogène ou l'un quelconque des groupements suivants :

A

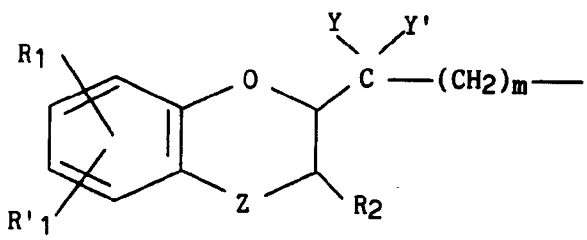

dans lequel :

- Z représente un atome d'oxygène ou un groupement $CH_2$,
- Y représente un atome d'hydrogène,
- Y' représente un atome d'hydrogène,
- ou bien, Y et Y' forment ensemble un atome d'oxygène,
- $R_1$ représente un atome d'hydrogène, un halogène, un groupement alkyle inférieur linéaire ou ramifié, un groupement alcoxy inférieur linéaire ou ramifié,
- $R'_1$ représente un atome d'hydrogène, un halogène, un groupement alcoxy inférieur linéaire ou ramifié,
- $R_2$ représente un atome d'hydrogène ou un groupement alkyle inférieur linéaire ou ramifié,
- m est égal à 0 ou 1,

B

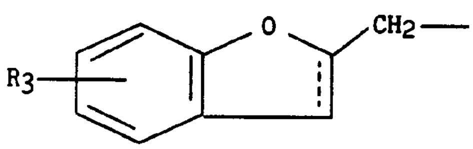

dans lequel $R_3$ représente un atome d'hydrogène, un groupement alkyle inférieur, linéaire ou ramifié, un halogène,

C

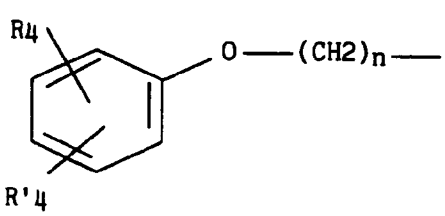

dans lequel :

- $R_4$ et $R'_4$ , identiques ou différents représentent un atome d'hydrogène, un halogène ou un groupement alcoxy inférieur, linéaire ou ramifié, ou forment ensemble lorsqu'ils sont situés sur deux carbones adjacents un groupement éthylènedioxy,
- n est égal à 2 ou 3,

D

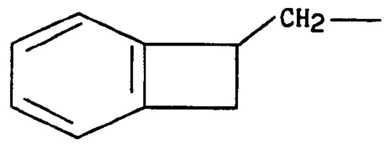

E

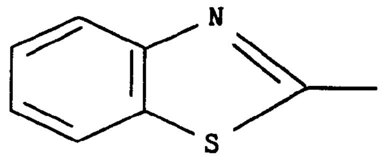

F

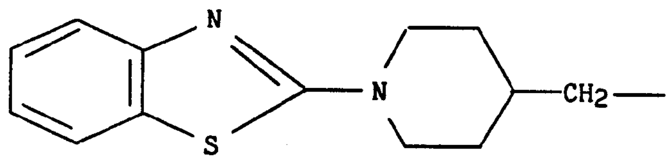

G

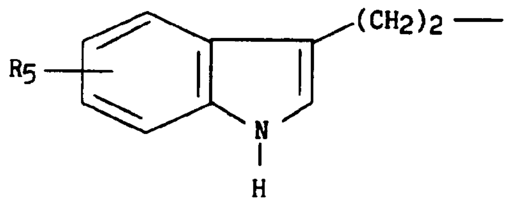

dans lequel:

$R_5$ représente un atome d'hydrogène ou un groupement alkoxy ($C_1$-$C_6$) linéaire ou ramifié,

R' représente un atome d'hydrogène, un groupement alkyle inférieur, linéaire ou ramifié ou l'un quelconque des groupements A, B, C, D, E ou F, avec la condition que R' représente un atome d'hydrogène lorsque R représente le groupement G,

ou bien

R et R' forment avec l'atome d'azote auxquels ils sont attachés un cycle pipérazine non substitué ou substitué sur l'azote libre de la pipérazine par un groupement phényle (non substitué ou substitué par un ou plusieurs atomes d'halogène ou groupements alkyle ($C_1$-$C_6$) ou alkoxy ($C_1$-$C_6$)),

le terme inférieur indiquant que les groupements ainsi qualifiés comptent de 1 à 6 atomes de carbone, le pointillé sur le groupement B indiquant la présence d'une simple ou d'une double liaison,

leurs énantiomères, diastéréoisomères et épimères ainsi que leurs sels d'addition à un acide pharmaceutiquement acceptable,

caractérisé en ce que l'on fait réagir sur un dérivé de formule (II) :

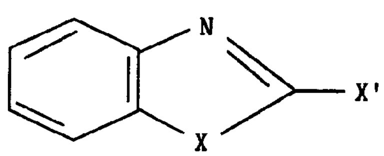

(II)

dans laquelle :

X a la même signification que dans la formule (I) et X' est un atome d'halogène,

1/ soit :

la 4-aminométhylpipéridine de formule (III) :

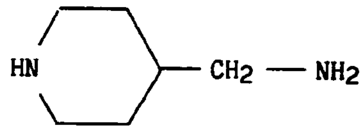 (III)

pour conduire à un dérivé de formule (I/a), cas particulier des dérivés de formule (I) :

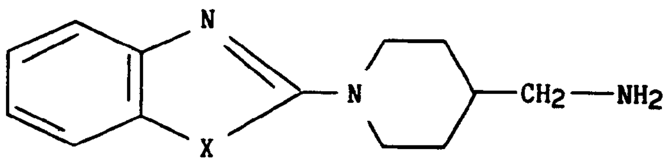 (I/a)

dans laquelle X a la même signification que dans la formule (I), que l'on condense sur un dérivé de formule (IV) :

$R_1$ - L (IV)

dans laquelle $R_1$ représente l'un quelconque des groupements A, B, C, D, E ou F,
L est un groupement libérable tel que le toluène sulfonate, le méthanesulfonate ou un atome d'halogène, et dont on a éventuellement préparé les isomères par une technique classique de préparation,
pour conduire à un dérivé de formule (I/b), cas particulier des dérivés de formule (I)

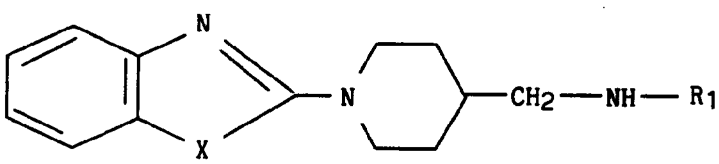 (I/b)

dans laquelle X et $R_1$ ont la même signification que précédemment, dont on sépare éventuellement les isomères par une technique classique de séparation,
que l'on soumet si on le désire,

- ou bien, à l'action d'un composé de formule (V):

R" - L (V)

dans laquelle L a la même signification que précedemment, et R" est l'un quelconque des groupements A, B, C, D, E ou F, pour conduire à un dérivé de formule (I/c), cas particulier des dérivés de formule (I)

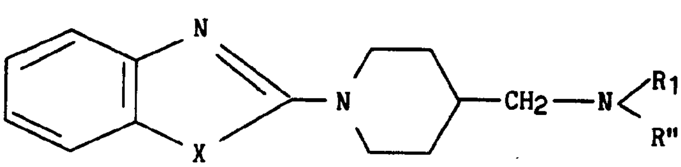 (I/c)

dans laquelle X, $R_1$ et R" ont la même signification que précédemment dont on sépare éventuellement les isomères par une technique classique de séparation,

- ou bien à l'action d'un aldéhyde de formule (VI)

R"' - CHO (VI)

dans laquelle R"' est un groupement alkyle inférieur, linéaire ou ramifié, en présence d'un réducteur tel que le borohydrure de sodium, le cyanoborohydrure de sodium, l'hydrogène, le nickel de Raney ou l'acide formique pour conduire à un dérivé de formule (I/d) :

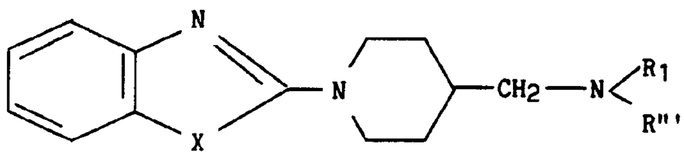

(I/d)

dans laquelle X, $R_1$ et R''' ont la même signification que précedemment, dont on sépare éventuellement les isomères par une technique classique de séparation,

2/ soit :

la 4-hydroxyméthylpipéridine de formule (VII) :

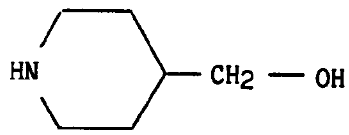

(VII)

pour conduire à un composé de formule (VIII) :

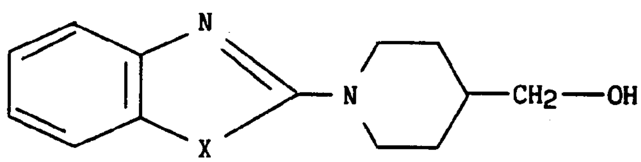

(VIII)

dans laquelle X a la même signification que précédemment
dont on sépare éventuellement les isomères par une technique classique de séparation,
que l'on soumet à l'action du chlorure de tosyle (Cl-Tos),
pour conduire à un composé de formule (IX) :

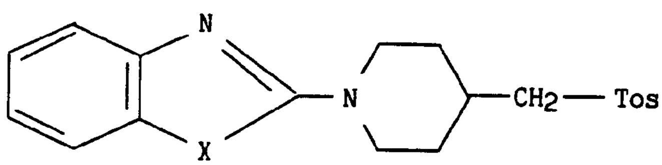

(IX)

dans laquelle X a la même signification que précédemment
que l'on fait réagir :

- ou bien avec un composé de formule (X) :

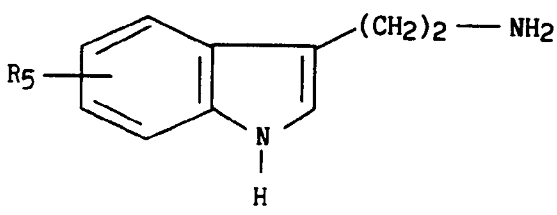

(X)

dans laquelle $R_5$ a la même signification que dans la formule (I)
pour conduire à un composé de formule (I/e), cas particulier des dérivés de formule (I) :

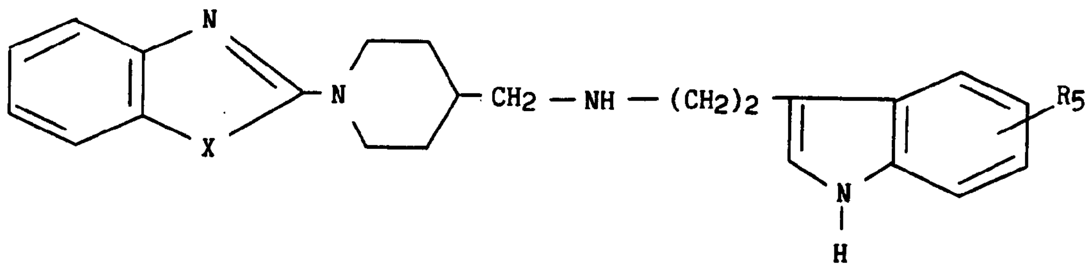

(I/e)

dans laquelle X et $R_5$ ont la même signification que précédemment, dont on sépare éventuellement les isomères par une technique classique de séparation

- ou bien avec une pipérazine de formule (XI) :

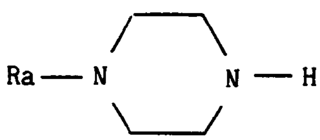

(XI)

dans laquelle Ra représente un atome d'hydrogène ou un groupement phényle non substitué ou substitué par un ou plusieurs atomes d'halogène, ou groupements alkyle ($C_1$-$C_6$) ou alkoxy ($C_1$-$C_6$), pour conduire à un composé de formule (I/f), cas particulier des dérivés de formule (I) :

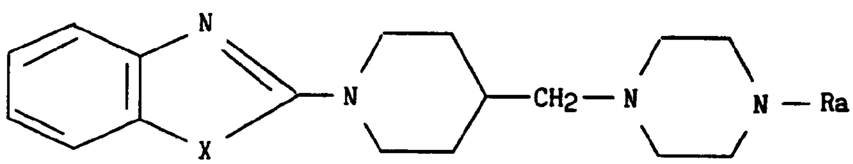

(I/f)

dans laquelle X et Ra ont la même signification que précédemment, dont on sépare éventuellement les isomères par une technique classique de séparation, dérivés (I/a), (I/b), (I/c), (I/d), (I/e) et (I/f) que l'on purifie par une technique classique de purification et que l'on transforme, si on le souhaite, en leurs sels d'addition à un acide pharmaceutiquement acceptable.

2. Procédé de préparation selon la revendication 1 des composés tels que X représente un atome de soufre.

3. Procédé de préparation selon la revendication 1 des composés tels que X représente un atome d'oxygène.

4. Procédé de préparation selon la revendication 1 des composés tels que X représente un groupement amino éventuellement substitué par un groupement alkyle inférieur linéaire ou ramifié.

5. Procédé de préparation selon la revendication 1 des composés tels que R représente un groupement (1,4-benzodioxan-2-yl)méthyl éventuellement substitué.

6. Procédé de préparation selon la revendication 1 des composés tels que R représente un groupement phénoxyéthyl éventuellement substitué.

7. Procédé de préparation selon la revendication 1 des composés tels que R représente un groupement (indol-3-yl) éthyl éventuellement substitué, leurs énantiomères, diastéréoisomères et épimères ainsi que leurs sels d'addition à un acide pharmaceutiquement acceptable.

8. Procédé de préparation selon l'une quelconque des revendications 1, 2 ou 6 du composé qui est la 1-(benzothiazol-2-yl)-4-[[2-(2-méthoxyphénoxy)éthyl]aminométhyl]pipéridine, ses énantiomères ainsi que ses sels d'addition à un acide pharmaceutiquement acceptable.

**9.** Procédé de préparation selon l'une quelconque des revendications 1, 2 ou 5 du composé qui est la 1-(benzothiazol-2-yl)-4-[(8-méthoxy-1,4-benzodioxan-2-yl)méthylaminométhyl]pipéridine, ses énantiomères et diastéréoisomères ainsi que ses sels d'addition à un acide pharmaceutiquement acceptable.

**10.** Procédé de préparation selon l'une quelconque des revendications 1, 3 ou 6 du composé qui est la 1-(benzoxazol-2-yl)-4-[[2-(2-méthoxyphénoxy)éthyl]aminométhyl]pipéridine, ses énantiomères ainsi que ses sels d'addition à un acide pharmceutiquement acceptable.

**11.** Procédé de préparation selon l'une quelconque des revendications 1, 4 ou 6 du composé qui est la 1-(benzimidazol-2-yl)-4-[[2-(2-méthoxyphénoxy)éthyl]aminométhyl]pipéridine, ses énantiomères ainsi que ses sels d'addition à un acide pharmaceutiquement acceptable.

**12.** Procédé de préparation selon l'une quelconque des revendications 1, 2 ou 7 du composé qui est la 1-(benzothiazol-2-yl)-4-[[2-(5-méthoxyindol-3-yl)éthyl]aminométhyl]pipéridine, ses énantiomères ainsi que ses sels d'addition à un acide pharmaceutiquement acceptable.

**Revendications pour l'Etat contractant suivant : GR**

**1.** Composés de formule générale (I) :

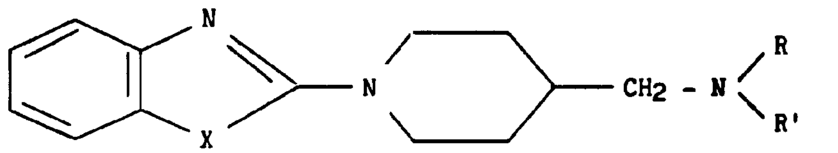

(I)

dans laquelle :
- X représente un atome de soufre, un atome d'oxygène, un groupement amino éventuellement substitué par un groupement alkyle inférieur linéaire ou ramifié,
- R représente un atome d'hydrogène ou l'un quelconque des groupements suivants :

A

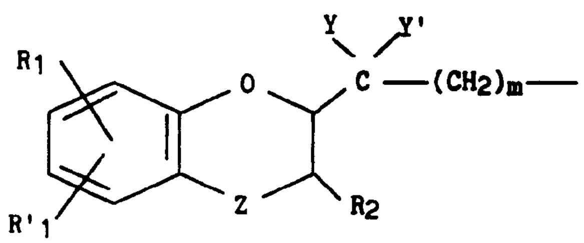

dans lequel :
- Z représente un atome d'oxygène ou un groupement $CH_2$,
- Y représente un atome d'hydrogène,
- Y' représente un atome d'hydrogène,
- ou bien, Y et Y' forment ensemble un atome d'oxygène,
- $R_1$ représente un atome d'hydrogène, un halogène, un groupement alkyle inférieur linéaire ou ramifié, un groupement alcoxy inférieur linéaire ou ramifié,
- $R'_1$ représente un atome d'hydrogène, un halogène, un groupement alcoxy inférieur linéaire ou ramifié,
- $R_2$ représente un atome d'hydrogène ou un groupement alkyle inférieur linéaire ou ramifié,
- m est égal à 0 ou 1,

B

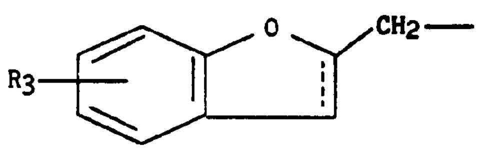

dans lequel $R_3$ représente un atome d'hydrogène, un groupement alkyle inférieur, linéaire ou ramifié, un halogène,

C 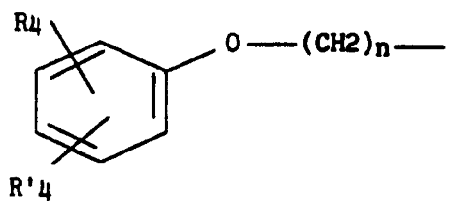

dans lequel :

- $R_4$ et $R'_4$ , identiques ou différents représentent un atome d'hydrogène, un halogène ou un groupement alcoxy inférieur, linéaire ou ramifié, ou forment ensemble lorsqu'ils sont situés sur deux carbones adjacents un groupement éthylènedioxy,
- n est égal à 2 ou 3,

D 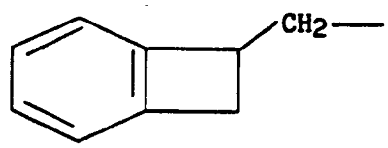

E 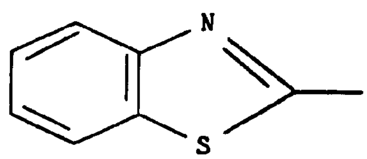

F 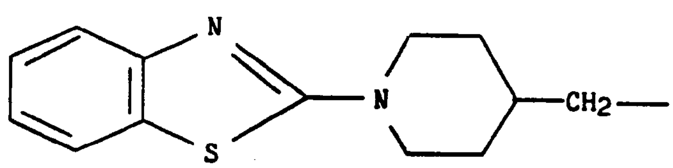

G 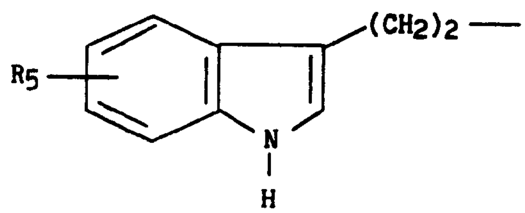

dans lequel :

$R_5$ représente un atome d'hydrogène ou un groupement alkoxy ($C_1$-$C_6$) linéaire ou ramifié,

R' représente un atome d'hydrogène, un groupement alkyle inférieur, linéaire ou ramifié ou l'un quelconque des groupements A, B, C, D, E ou F, avec la condition que R' représente un atome d'hydrogène lorsque R représente le groupement G,

<u>ou bien</u>

R et R' forment avec l'atome d'azote auxquels ils sont attachés un cycle pipérazine non substitué ou substitué sur l'azote libre de la pipérazine par un groupement phényle (non substitué ou substitué par un ou plusieurs atomes d'halogène ou groupements alkyle ($C_1$-$C_6$) ou alkoxy ($C_1$-$C_6$)),

le terme inférieur indiquant que les groupements ainsi qualifiés comptent de 1 à 6 atomes de carbone, le pointillé sur le groupement B indiquant la présence d'une simple ou d'une double liaison,

leurs énantiomères, diastéréoisomères et épimères ainsi que leurs sels d'addition à un acide pharmaceutiquement acceptable.

2. Composés selon la revendication 1 tels que X représente un atome de soufre.

3. Composés selon la revendication 1 tels que X représente un atome d'oxygène.

4. Composés selon la revendication 1 tels que X représente un groupement amino éventuellement substitué par un groupement alkyle inférieur linéaire ou ramifié.

5. Composés selon la revendication 1 tels que R représente un groupement (1,4-benzodioxan-2-yl)méthyl éventuellement substitué.

6. Composés selon la revendication 1 tels que R représente un groupement phénoxyéthyl éventuellement substitué.

7. Composés selon la revendication 1 tels que R représente un groupement (indol-3-yl) éthyl éventuellement substitué, leurs énantiomères, diastéréoisomères et épimères ainsi que leurs sels d'addition à un acide pharmaceutiquement acceptable.

8. Composé selon l'une quelconque des revendications 1, 2 ou 6 qui est la 1-(benzothiazol-2-yl)-4-[[2-(2-méthoxyphénoxy)éthyl]aminométhyl] pipéridine, ses énantiomères ainsi que ses sels d'addition à un acide pharmaceutiquement acceptable.

9. Composé selon l'une quelconque des revendications 1, 2 ou 5 qui est la 1-(benzothiazol-2-yl)-4-[(8-méthoxy-1,4-benzodioxan-2-yl) méthyl aminométhyl] pipéridine, ses énantiomères et diastéréoisomères ainsi que ses sels d'addition à un acide pharmaceutiquement acceptable.

10. Composé selon l'une quelconque des revendications 1, 3 ou 6 qui est la 1-(benzoxazol-2-yl)-4-[[2-(2-méthoxyphénoxy)éthyl]aminométhyl] pipéridine, ses énantiomères ainsi que ses sels d'addition à un acide pharmceutiquement acceptable.

11. Composé selon l'une quelconque des revendications 1, 4 ou 6 qui est la 1-(benzimidazol-2-yl)-4-[[2-(2-méthoxyphénoxy)éthyl] aminométhyl]pipéridine, ses énantiomères ainsi que ses sels d'addition à un acide pharmaceutiquement acceptable.

12. Composé selon l'une quelconque des revendications 1, 2 ou 7 qui est la 1-(benzothiazol-2-yl)-4-[[2-(5-méthoxyindol-3-yl)éthyl]aminométhyl]pipéridine, ses énantiomères ainsi que ses sels d'addition à un acide pharmaceutiquement acceptable.

13. Procédé de préparation des dérivés de formule (I) selon la revendication 1 caractérisé en ce que l'on fait réagir sur un dérivé de formule (II) :

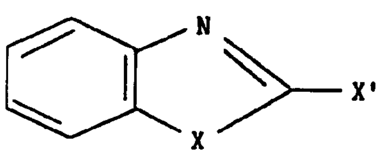

(II)

dans laquelle :

X a la même signification que dans la formule (I) et X' est un atome d'halogène,

1/ soit :

la 4-aminométhylpipéridine de formule (III) :

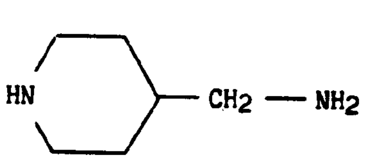

(III)

pour conduire à un dérivé de formule (I/a), cas particulier des dérivés de formule (I) :

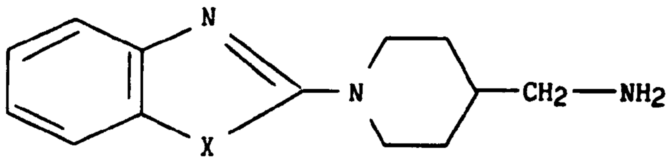

(I/a)

dans laquelle X a la même signification que dans la formule (I), que l'on condense sur un dérivé de formule (IV) :

$R_1$ - L (IV)

dans laquelle $R_1$ représente l'un quelconque des groupements A, B, C, D, E ou F,
L est un groupement libérable tel que le toluène sulfonate, le méthanesulfonate ou un atome d'halogène, et dont on a éventuellement préparé les isomères par une technique classique de préparation,
pour conduire à un dérivé de formule (I/b), cas particulier des dérivés de formule (I)

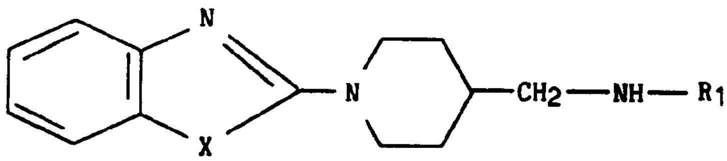

(I/b)

dans laquelle X et $R_1$ ont la même signification que précédemment, dont on sépare éventuellement les isomères par une technique classique de séparation,
que l'on soumet si on le désire,

- ou bien, à l'action d'un composé de formule (V) :

R" - L (V)

dans laquelle L a la même signification que précedemment, et R" est l'un quelconque des groupements A, B, C, D, E ou F, pour conduire à un dérivé de formule (I/c), cas particulier des dérivés de formule (I)

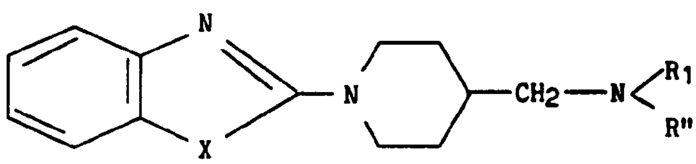

(I/c)

dans laquelle X, $R_1$ et R" ont la même signification que précédemment dont on sépare éventuellement les isomères par une technique classique de séparation,

- ou bien à l'action d'un aldéhyde de formule (VI)

R'" - CHO (VI)

dans laquelle R'" est un groupement alkyle inférieur, linéaire ou ramifié, en présence d'un réducteur tel que le borohydrure de sodium, le cyanoborohydrure de sodium, l'hydrogène, le nickel de Raney ou l'acide formique pour conduire à un dérivé de formule (I/d) :

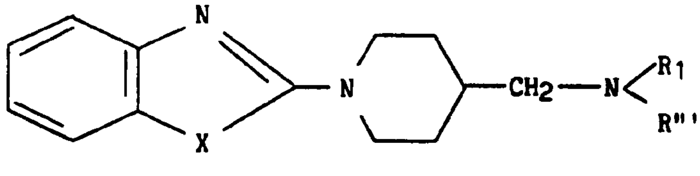

(I/d)

dans laquelle X, $R_1$ et R'" ont la même signification que précedemment, dont on sépare éventuellement les isomères par une technique classique de séparation, 2/ soit :

la 4-hydroxyméthylpipéridine de formule (VII) :

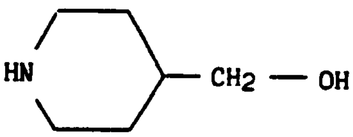

(VII)

pour conduire à un composé de formule (VIII) :

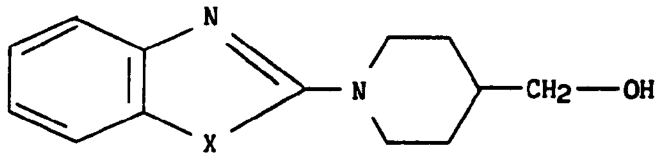

(VIII)

dans laquelle X a la même signification que précédemment
dont on sépare éventuellement les isomères par une technique classique de séparation,
que l'on soumet à l'action du chlorure de tosyle (Cl-Tos),
pour conduire à un composé de formule (IX) :

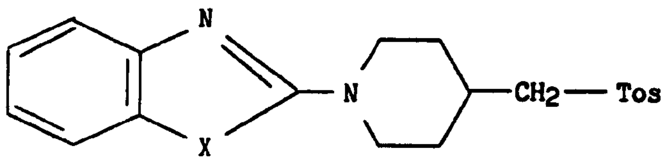

(IX)

dans laquelle X a la même signification que précédemment que l'on fait réagir :

- ou bien avec un composé de formule (X) :

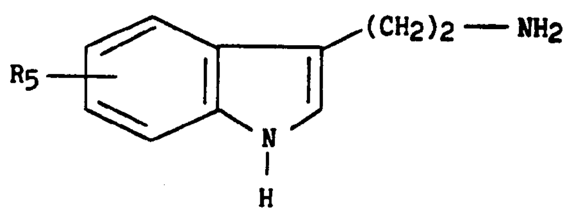

(X)

dans laquelle $R_5$ a la même signification que dans la formule (I)
pour conduire à un composé de formule (I/e), cas particulier des dérivés de formule (I) :

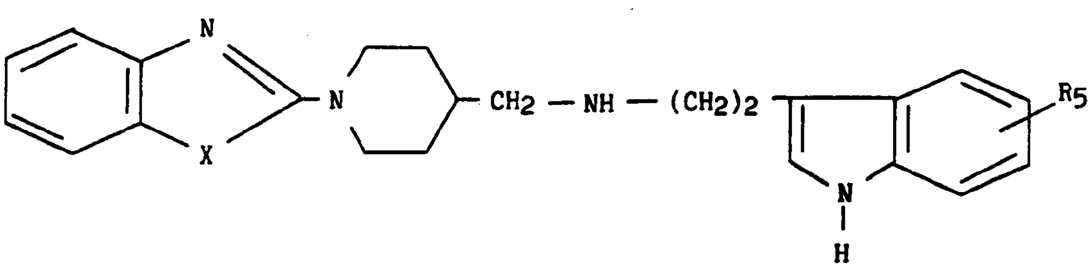

(I/e)

dans laquelle X et $R_5$ ont la même signification que précédemment, dont on sépare éventuellement les isomères par une technique classique de séparation

- ou bien avec une pipérazine de formule (XI) :

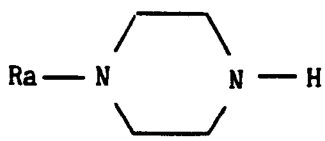

(XI)

dans laquelle Ra représente un atome d'hydrogène ou un groupement phényle non substitué ou substitué par un ou plusieurs atomes d'halogène, ou groupements alkyle ($C_1$-$C_6$) ou alkoxy ($C_1$-$C_6$), pour conduire à un composé de formule (I/f), cas particulier des dérivés de formule (I) :

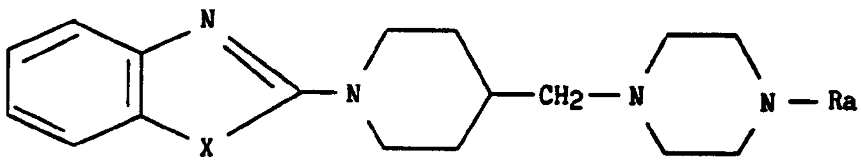

(I/f)

dans laquelle X et Ra ont la même signification que précédemment, dont on sépare éventuellement les isomères par une technique classique de séparation, dérivés (I/a), (I/b), (I/c), (I/d), (I/e) et (I/f) que l'on purifie par une technique classique de purification et que l'on transforme, si on le souhaite, en leurs sels d'addition à un acide pharmaceutiquement acceptable.

**14.** Compositions pharmaceutiques contenant comme principe actif au moins un composé selon l'une quelconque des revendications 1 à 12 , seul ou en combinaison avec un ou plusieurs excipients ou véhicules inertes, non toxiques, pharmaceutiquement acceptables.

**15.** Compositions pharmaceutiques selon la revendication 14 contenant au moins un principe actif selon l'une des revendications 1 à 12 utiles pour le traitement de la dépression, du stress, des psychoses, d'anxiété, de la douleur, de la schizophrénie, de l'hypertension et des maladies cardio-vasculaires, et comme modificateur du comportement alimentaire et sexuel.

## Patentansprüche

### Patentansprüche für folgende Vertragsstaaten : AT, BE, CH, DE, DK, FR, GB, IT, LI, LU, NL, SE

**1.** Verbindungen der allgemeinen Formel (I):

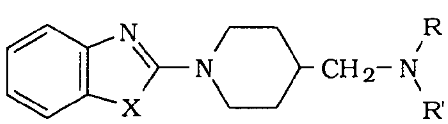

(I)

in der:

- X ein Schwefelatom. ein Sauerstoffatom oder eine gegebenenfalls durch eine geradkettige oder verzweigte Niedrigalkylgruppe substituierte Aminogruppe,
- R ein Wasserstoffatom oder eine der folgenden Gruppen:

A

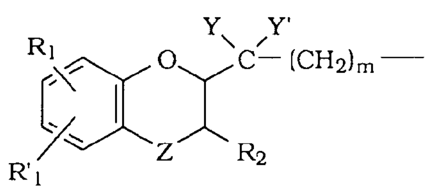

in der:

- Z ein Sauerstoffatom oder eine $CH_2$-Gruppe,
- Y ein Wasserstoffatom,

- Y' ein Wasserstoffatom,
- oder Y und Y' gemeinsam ein Sauerstoffatom,
- $R_1$ ein Wasserstoffatom, ein Halogenatom, eine geradkettige oder verzweigte Niedrigalkylgruppe oder eine geradkettige oder verzweigte Niedrigalkoxygruppe,
- $R'_1$ ein Wasserstoffatom, ein Halogenatom oder eine geradkettige oder verzweigte Niedrigalkoxygruppe,
- $R_2$ ein Wasserstoffatom oder eine geradkettige oder verzweigte Niedrigalkylgruppe und
- m 0 oder 1 darstellen,

B 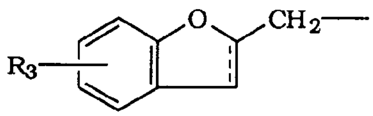

in der $R_3$ ein Wasserstoffatom, eine geradkettige oder verzweigte Niedrigalkylgruppe oder ein Halogenatom darstellt,

C 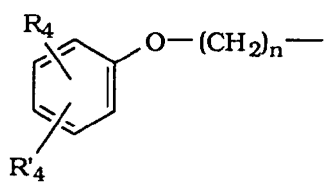

in der:
- $R_4$ und $R'_4$, die gleichartig oder verschieden sein können, Wasserstoffatome, Halogenatome, geradkettige oder verzweigte Niedrigalkoxygruppen oder gemeinsam, wenn sie an zwei benachbarten Kohlenstoffatomen stehen, eine Ethylendioxygruppe und
- n 2 oder 3 darstellen,

D 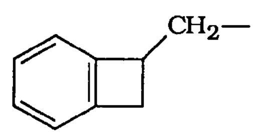

E 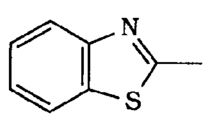

F 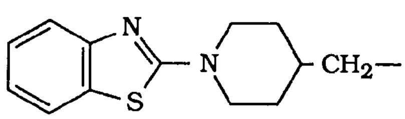

G 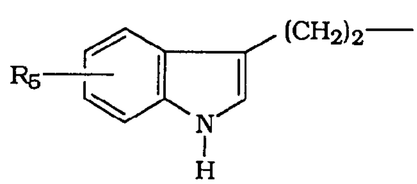

in denen:
- $R_5$ ein Wasserstoffatom oder eine geradkettige oder verzweigte $C_1$-$C_6$-Alkoxygruppe darstellt,

R' ein Wasserstoffatom, eine geradkettige oder verzweigte Niedrigalkylgruppe oder eine der Gruppen A,

B, C, D, E oder F, mit der Maßgabe, daß R' ein Wasserstoffatom darstellt, wenn R die Gruppe G bedeutet, oder
R und R' gemeinsam mit dem Stickstoffatom, an das sie gebunden sind, einen unsubstituierten oder am freien Stickstoffatom durch eine Phenylgruppe (die nicht substituiert oder durch eines oder mehrere Halogenatome oder $C_1$-$C_6$-Alkylgruppen substituiert ist) oder eine $C_1$-$C_6$-Alkoxygruppe substituierten Piperazinring bedeuten,
wobei der Begriff "Niedrig" bedeutet, daß die in dieser Weise bezeichneten Gruppen 1 bis 6 Kohlenstoffatome aufweisen und die gestrichelte Linie der Gruppe B die Anwesenheit einer Einfachbindung oder einer Doppelbindung bedeutet, deren Enantiomere, Diastereoisomere und Epimere sowie deren Additionssalze mit einer pharmazeutisch annehmbaren Säure.

2. Verbindungen nach Anspruch 1, worin X ein Schwefelatom bedeutet.

3. Verbindungen nach Anspruch 1, worin X ein Sauerstoffatom bedeutet.

4. Verbindungen nach Anspruch 1, worin X eine Aminogruppe, die gegebenenfalls durch eine geradkettige oder verzweigte Niedrigalkylgruppe substituiert ist. bedeutet.

5. Verbindungen nach Anspruch 1, worin R eine gegebenenfalls substituierte (1,4-Benzodioxan-2-yl)-methyl-gruppe bedeutet.

6. Verbindungen nach Anspruch 1, worin R eine gegebenenfalls substituierte Phenoxyethylgruppe bedeutet.

7. Verbindungen nach Anspruch 1, worin R eine gegebenenfalls substituierte (Indol-3-yl)-ethyl-gruppe bedeutet, deren Enantiomere, Diastereoisomere und Epimere sowie deren Additionssalze mit einer pharmazeutisch annehmbaren Säure.

8. Verbindung nach einem der Ansprüche 1, 2 oder 6, nämlich 1-(Benzothiazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

9. Verbindung nach einem derAnsprüche 1, 2 oder 5, nämlich 1-(Benzothiazol-2-yl)-4-[(8-methoxy-1,4-benzodioxan-2-yl)-methyl-aminomethyl]-piperidin, dessen Enantiomere und Diastereoisomere sowie dessen Additionssalze mit einer pharmazeutisch annehmbaren Säure.

10. Verbindung nach einem der Ansprüche 1, 3 oder 6, nämlich l-(Benzoxazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

11. Verbindung nach einem der Ansprüche 1, 4 oder 6, nämlich 1-(Benzimidazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

12. Verbindung nach einem der Ansprüche 1, 2 oder 7, nämlich 1-(Benzothiazol-2-yl)-4-[[2-(5-methoxyindol-3-yl)-ethyl]-aminomethyl]-piperidin, dessen Enantiomere sowie dessen Additionssalze mit einer pharmazeutisch annehmbaren Säure.

13. Verfahren zur Herstellung der Derivate der Formel (I) nach Anspruch 1, **dadurch gekennzeichnet,** daß man ein Derivat der Formel (II):

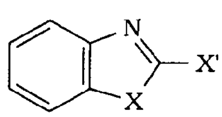

(II)

in der:

X die bezüglich der Formel (I) angegebenen Bedeutungen besitzt und X' ein Halogenatom darstellt,
1/ entweder:
mit 4-Aminomethylpiperidin der Formel (III):

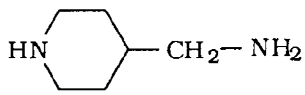

(III)

umsetzt, so daß man ein Derivat der Formel (I/a) erhält. einem Sonderfall der Derivate der Formel (I):

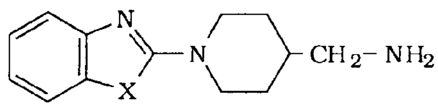

(I/a)

in der X die bezüglich der Formel (I) angegebenen Bedeutungen besitzt, welches man mit einem Derivat der Formel (IV):

$R_1$ - L (IV)

in der $R_1$ eine der Gruppen A, B, C, D, E oder F und L eine freisetzbare Gruppe, wie das Toluolsulfonat, das Methansulfonat oder ein Halogenatom bedeuten, von dem man gegebenenfalls die Isomeren durch eine klassische Herstellungsweise gebildet hat, kondensiert, so daß man ein Derivat der Formel (I/b) erhält, einem Sonderfall der Derivate der Formel (I):

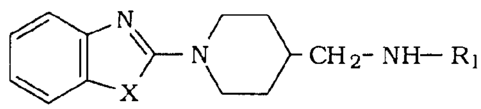

(I/b)

in der X und $R_1$ die oben angegebenen Bedeutungen besitzen. dessen Isomere man gegebenenfalls mit Hilfe einer klassischen Trennungsmethode trennt, welches man gewünschtenfalls

- entweder mit einer Verbindung der Formel (V)

  R" - L (V)

  in der L die oben angegebenen Bedeutungen besitzt und R" eine der Gruppen A, B, C, D, E oder F darstellt. umsetzt zur Bildung eines Derivats der Formel (I/c) erhält, einem Sonderfall der Derivate der Formel (I):

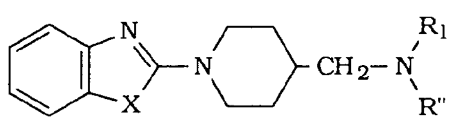

(I/c)

  in der X, $R_1$ und R" die oben angegebenen Bedeutungen besitzen, das man gegebenenfalls mit Hilfe einer klassischen Trennmethode in die Isomeren auftrennt,

- oder mit einem Aldehyd der Formel (VI)

  R"' - CHO (VI)

  in der R"' eine geradkettige oder verzweigte Niedrigalkylgruppe bedeutet, in Gegenwart eines Reduktionsmittels, wie Natriumborhydrid, Natriumcyanoborhydrid, Wasserstoff, Raney-Nickel oder Ameisensäure umsetzt, so daß man ein Derivat der Formel (I/d), erhält:

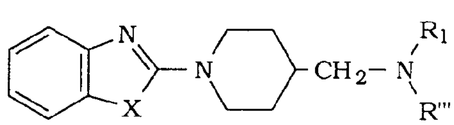

(I/d)

  in der X, $R_1$ und R"' die oben angegebenen Bedeutungen besitzen, das man gegebenenfalls mit Hilfe einer klassischen Trennmethode in die Isomeren auftrennt.

2/ oder

mit 4-Hydroxymethylpiperidin der Formel (VII):

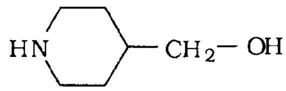 (VII)

umsetzt, so daß man eine Verbindung der Formel (VIII)

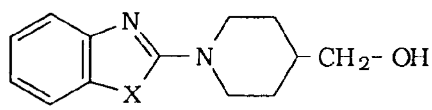 (VIII)

in der X die oben angegebenen Bedeutungen besitzt, erhält, die man gegebenenfalls mit Hilfe einer klassischen Trennmethode in die Isomeren auftrennt, welche man der Einwirkung von Tosylchlorid (Cl-Tos) unterwirft, so daß man eine Verbindung der Formel (IX):

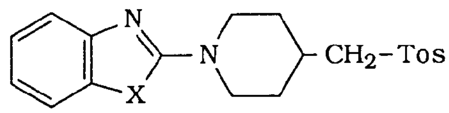 (IX)

erhält, in der X die oben angegebenen Bedeutungen besitzt, welche man:

- entweder mit einer Verbindung der Formel (X):

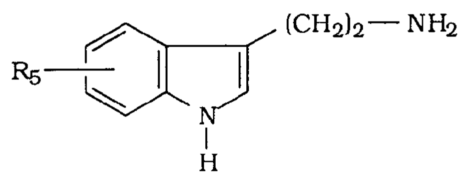 (X)

in der $R_5$ die bezüglich der Formel (I) angegebenen Bedeutungen besitzt, umsetzt, so daß man eine Verbindung der Formel (I/e) erhält, einem Sonderfall der Derivate der Formel (I):

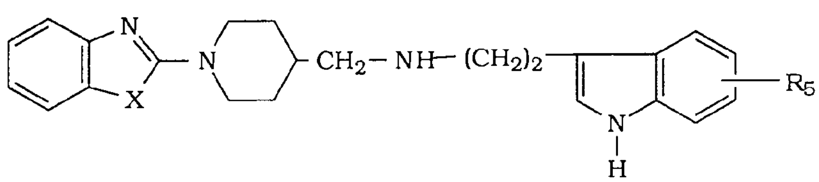 (I/e)

in der X und $R_5$ die oben angegebenen Bedeutungen besitzen. welche man gegebenenfalls mit Hilfe einer klassischen Trennmethode in die Isomeren auftrennt,

- oder mit einem Piperazin der Formel (XI):

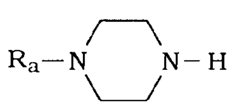 (XI)

in der $R_a$ ein Wasserstoffatom oder eine unsubstituierte oder durch eines oder mehrere Halogenatome, $C_1$-$C_6$-Alkylgruppen oder $C_1$-$C_6$-Alkoxygruppen substituierte Phenylgruppe darstellt, umsetzt, so daß man eine Verbindung der Formel (I/f) erhält, einem Sonderfall der Derivate der Formel (I):

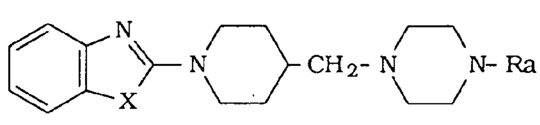 (I/f)

in der X und $R_a$ die oben angegebenen Bedeutungen besitzen, die man gegebenenfalls mit Hilfe einer klassischen Methode in die Isomeren auftrennt,
wobei man die Derivate (I/a), (I/b), (I/c), (I/d), (I/e) und (I/f) mit Hilfe einer klassischen Reinigungsmethode reinigt und gewünschtenfalls mit einer pharmazeutisch annehmbaren Säure in ihre Additionssalze überführt.

**14.** Pharmazeutische Zubereitungen enthaltend als Wirkstoffmindestens eine Verbindung nach einem der Ansprüche 1 bis 12 allein oder in Kombination mit einem oder mehreren inerten, nichttoxischen, pharmazeutisch annehmbaren Bindemitteln oder Trägermaterialien.

**15.** Pharmazeutische Zubereitungen nach Anspruch 14 enthaltend mindestens einen Wirkstoff nach einem der Ansprüche 1 bis 12 zur Behandlung von Depressionen, Streß, Psychosen, der Angst, Schmerzen, der Schizophrenie, der Hypertension und kardio-vaskulären Erkrankungen und als Mittel zur Modifizierung des Ernährungs- und Sexualverhaltens.

**Patentansprüche für folgenden Vertragsstaat : ES**

**1.** Verfahren zur Herstellung der Verbindungen der allgemeinen Formel (I):

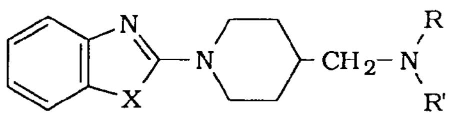

(I)

in der:
- X ein Schwefelatom, ein Sauerstoffatom oder eine gegebenenfalls durch eine geradkettige oder verzweigte Niedrigalkylgruppe substituierte Aminogruppe,
- R ein Wasserstoffatom oder eine der folgenden Gruppen:

A 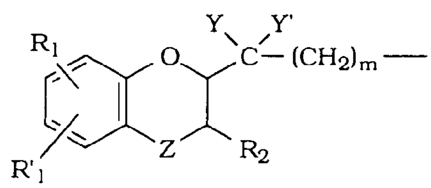

in der:
- Z ein Sauerstoffatom oder eine $CH_2$-Gruppe,
- Y ein Wasserstoffatom,
- Y' ein Wasserstoffatom,
- oder Y und Y' gemeinsam ein Sauerstoffatom,
- $R_1$ ein Wasserstoffatom, ein Halogenatom, eine geradkettige oder verzweigte Niedrigalkylgruppe oder eine geradkettige oder verzweigte Niedrigalkoxygruppe,
- $R'_1$ ein Wasserstoffatom, ein Halogenatom oder eine geradkettige oderverzweigte Niedrigalkoxygruppe,
- $R_2$ ein Wasserstoffatom oder eine geradkettige oder verzweigte Niedrigalkylgruppe und
- m 0 oder 1 darstellen,

B 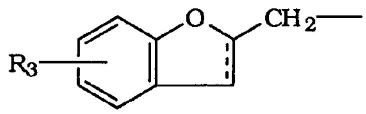

in der $R_3$ ein Wasserstoffatom, eine geradkettige oder verzweigte Niedrigalkylgruppe oder ein Halogenatom darstellt,

C 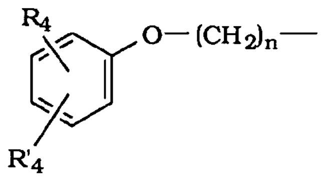

in der:

- $R_4$ und $R'_4$, die gleichartig oder verschieden sein können, Wasserstoffatome, Halogenatome, geradkettige oder verzweigte Niedrigalkoxygruppen oder gemeinsam, wenn sie an zwei benachbarten Kohlenstoffatomen stehen, eine Ethylendioxygruppe und
- n 2 oder 3 darstellen,

D 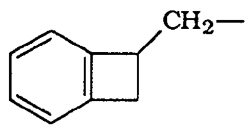

E 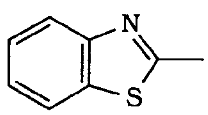

F 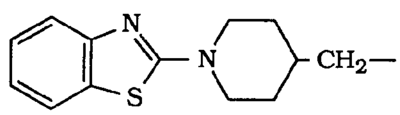

G 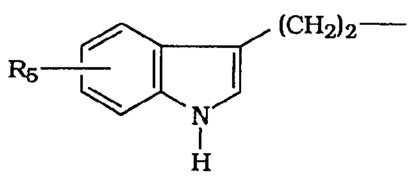

in denen:

- $R_5$ ein Wasserstoffatom oder eine geradkettige oder verzweigte $C_1$-$C_6$-Alkoxygruppe darstellt,

R' ein Wasserstoffatom, eine geradkettige oder verzweigte Niedrigalkylgruppe oder eine der GruppenA, B, C, D, E oder F, mit der Maßgabe, daß R' ein Wasserstoffatom darstellt, wenn R die Gruppe G bedeutet,

oder

R und R' gemeinsam mit dem Stickstoffatom, an das sie gebunden sind, einen unsubstituierten oder am freien Stickstoffatom durch eine Phenylgruppe (die nicht substituiert oder durch eines oder mehrere Halogenatome oder $C_1$-$C_6$-Alkylgruppen substituiert ist) oder eine $C_1$-$C_6$-Alkoxygruppe substituierten Piperazinring bedeuten,

wobei der Begriff "Niedrig" bedeutet, daß die in dieser Weise bezeichneten Gruppen 1 bis 6 Kohlenstoffatome aufweisen und die gestrichelte Linie der Gruppe B die Anwesenheit einer Einfachbindung oder einer Doppelbindung bedeutet, sowie von deren Enantiomeren, Diastereoisomeren und Epimeren sowie von deren Additionssalzen mit einer pharmazeutisch annehmbaren Säure, **dadurch gekennzeichnet,** daß man ein Derivat der Formel (II):

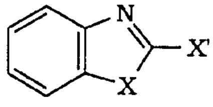 (II)

in der:

X die bezüglich der Formel (I) angegebenen Bedeutungen besitzt und X' ein Halogenatom darstellt,

1/ entweder:
mit 4-Aminomethylpiperidin der Formel (III):

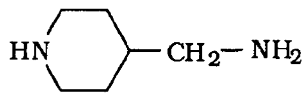

(III)

umsetzt, so daß man ein Derivat der Formel (I/a) erhält, einem Sonderfall der Derivate der Formel (I):

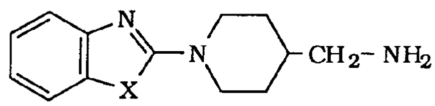

(I/a)

in der X die bezüglich der Formel (I) angegebenen Bedeutungen besitzt, welches man mit einem Derivat der Formel (IV):

$R_1$ - L (IV)

in der $R_1$ eine der Gruppen A, B, C, D, E oder F und L eine freisetzbare Gruppe, wie das Toluolsulfonat, das Methansulfonat oder ein Halogenatom bedeuten, von dem man gegebenenfalls die Isomeren durch eine klassische Herstellungsweise gebildet hat, kondensiert, so daß man ein Derivat der Formel (I/b) erhält, einem Sonderfall der Derivate der Formel (I):

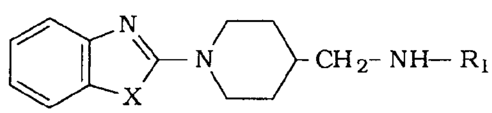

(I/b)

in der X und $R_1$ die oben angegebenen Bedeutungen besitzen, dessen Isomere man gegebenenfalls mit Hilfe einer klassischen Trennungsmethode trennt, welches man gewünschtenfalls

- entweder mit einer Verbindung der Formel (V)

  R" - L (V)

  in der L die oben angegebenen Bedeutungen besitzt und R" eine der Gruppen A, B, C, D, E oder F darstellt. umsetzt zur Bildung eines Derivats der Formel (I/c) erhält, einem Sonderfall der Derivate der Formel (I):

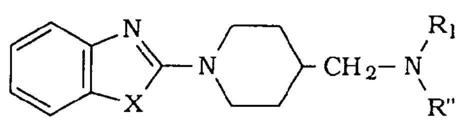

(I/c)

  in der X, $R_1$ und R" die oben angegebenen Bedeutungen besitzen, das man gegebenenfalls mit Hilfe einer klassischen Trennmethode in die Isomeren auftrennt,
- oder mit einem Aldehyd der Formel (VI)

  R"' - CHO (VI)

  in der R"' eine geradkettige oder verzweigte Niedrigalkylgruppe bedeutet, in Gegenwart eines Reduktionsmittels, wie Natriumborhydrid, Natriumcyanoborhydrid, Wasserstoff, Raney-Nickel oder Ameisensäure umsetzt, so daß man ein Derivat der Formel (I/d), erhält:

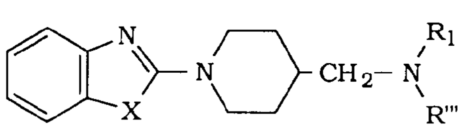

(I/d)

  in der X, $R_1$ und R"' die oben angegebenen Bedeutungen besitzen, das man gegebenenfalls mit Hilfe einer klassischen Trennmethode in die Isomeren auftrennt,

2/ oder

mit 4-Hydroxymethylpiperidin der Formel (VII):

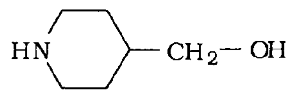

(VII)

umsetzt, so daß man eine Verbindung der Formel (VIII)

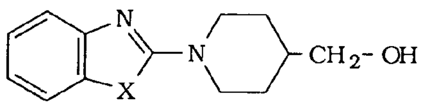

(VIII)

in der X die oben angegebenen Bedeutungen besitzt, erhält, die man gegebenenfalls mit Hilfe einer klassischen Trennmethode in die Isomeren auftrennt, welche man der Einwirkung von Tosylchlorid (Cl-Tos) unterwirft, so daß man eine Verbindung der Formel (IX):

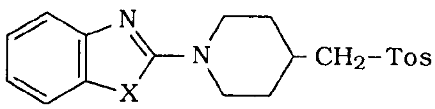

(IX)

erhält, in der X die oben angegebenen Bedeutungen besitzt. welche man:

- entweder mit einer Verbindung der Formel (X):

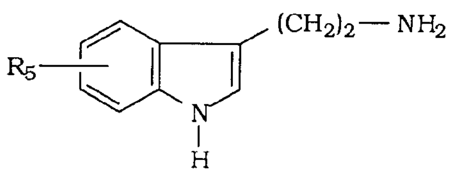

(X)

in der $R_5$ die bezüglich der Formel (I) angegebenen Bedeutungen besitzt, umsetzt, so daß man eine Verbindung der Formel (I/e) erhält, einem Sonderfall der Derivate der Formel (I):

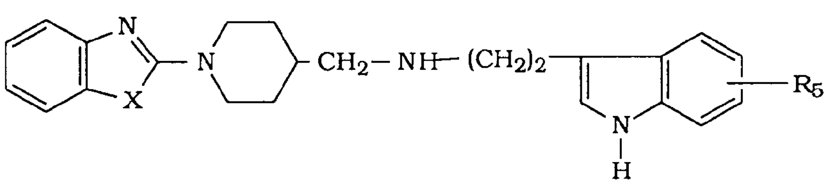

(I/e)

in der X und $R_5$ die oben angegebenen Bedeutungen besitzen. welche man gegebenenfalls mit Hilfe einer klassischen Trennmethode in die Isomeren auftrennt,

- oder mit einem Piperazin der Formel (XI):

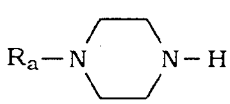

(XI)

in der $R_a$ ein Wasserstoffatom oder eine unsubstituierte oder durch eines oder mehrere Halogenatome, $C_1$-$C_6$-Alkylgruppen oder $C_1$-$C_6$-Alkoxygruppen substituierte Phenylgruppe darstellt, umsetzt, so daß man eine Verbindung der Formel (I/f) erhält, einem Sonderfall der Derivate der Formel (I):

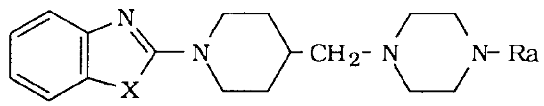 (I/f)

in der X und $R_a$ die oben angegebenen Bedeutungen besitzen, die man gegebenenfalls mit Hilfe einer klassischen Methode in die Isomeren auftrennt,
wobei man die Derivate (I/a), (I/b), (I/c), (I/d), (I/e) und (I/f) mit Hilfe einer klassischen Reinigungsmethode reinigt und gewünschtenfalls mit einer pharmazeutisch annehmbaren Säure in ihre Additionssalze überführt.

**2.** Verfahren nach Anspruch 1 zur Herstellung von Verbindungen, worin X ein Schwefelatom bedeutet.

**3.** Verfahren nach Anspruch 1 zur Herstellung von Verbindungen, worin X ein Sauerstoffatom bedeutet.

**4.** Verfahren nach Anspruch 1 zur Herstellung von Verbindungen, worin X eine gegebenenfalls durch eine geradkettige oder verzweigte Niedrigalkylgruppe substituierte Aminogruppe bedeutet.

**5.** Verfahren nach Anspruch 1 zur Herstellung von Verbindungen, worin R eine gegebenenfalls substituierte (1,4-Benzodioxan-2-yl)-methylgruppe bedeutet.

**6.** Verfahren nach Anspruch 1 zur Herstellung von Verbindungen, worin R eine gegebenenfalls substituierte Phenoxyethylgruppe bedeutet.

**7.** Verfahren nach Anspruch 1 zur Herstellung von Verbindungen, worin R eine gegebenenfalls substituierte (indol-3-yl)-ethylgruppe bedeutet sowie von deren Enantiomeren, Diastereoisomeren und Epimeren sowie von deren Additionssalzen mit einer pharmazeutisch annehmbaren Säure.

**8.** Verfahren nach einem der Ansprüche 1, 2 oder 6 zur Herstellung von 1-(Benzothiazol-2-yl)-4-[[2-(2-methoxyphenoxy)-ethyl]-aminomethyl]-piperidin sowie dessen Enantiomeren und dessen Additionssalzen mit einer pharmazeutisch annehmbaren Säure.

**9.** Verfahren nach einem der Ansprüche 1, 2 oder 5 zur Herstellung von 1-(Benzothiazol-2-yl)-4-[(8-methoxy-1,4-benzodioxan-2-yl)-methylaminomethyl]-piperidin, dessen Enantiomeren und Diastereoisomeren sowie dessen Additionssalzen mit einer pharmazeutisch annehmbaren Säure.

**10.** Verfahren nach einem der Ansprüche 1. 3 oder 4 zur Herstellung von 1-(Benzoxazol-2-yl)-4-[[2-(2-methoxyphenoxy)-ethyl]-aminomethyl]-piperidin, dessen Enantiomeren sowie dessen Additionssalzen mit einer pharmazeutisch annehmbaren Säure.

**11.** Verfahren nach einem der Ansprüche 1, 4 oder 6 zur Herstellung von 1-(Benzimidazol-2-yl)-4-[[2-(2-methoxyphenoxy)-ethyl]-aminomethyl]-piperidin, dessen Enantiomeren sowie dessen Additionssalzen mit einer pharmazeutisch annehmbaren Säure.

**12.** Verfahren nach einem der Ansprüche 1, 2 oder 7 zur Herstellung von 1-(Benzothiazol-2-yl)-4-[[2-(5-methoxyindol-3-yl)-ethyl]-aminomethyl]-piperidin, dessen Enantiomeren sowie dessenAdditionssalzen mit eine pharmazeutisch annehmbaren Säure.

**Patentansprüche für folgenden Vertragsstaat : GR**

**1.** Verbindungen der allgemeinen Formel (I):

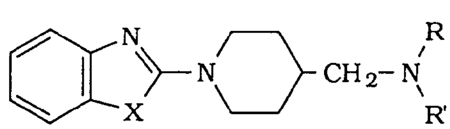 (I)

in der:

- X ein Schwefelatom, ein Sauerstoffatom oder eine gegebenenfalls durch eine geradkettige oder verzweigte Niedrigalkylgruppe substituierte Aminogruppe,

- R ein Wasserstoffatom oder eine der folgenden Gruppen:

A 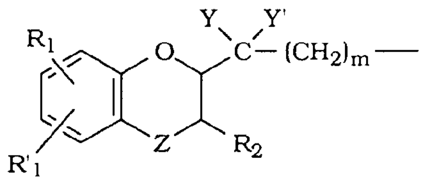

in der:
- Z ein Sauerstoffatom oder eine $CH_2$-Gruppe,
- Y ein Wasserstoffatom,
- Y' ein Wasserstoffatom,
- oder Y und Y' gemeinsam ein Sauerstoffatom,
- $R_1$ ein Wasserstoffatom, ein Halogenatom, eine geradkettige oder verzweigte Niedrigalkylgruppe oder eine geradkettige oder verzweigte Niedrigalkoxygruppe,
- $R'_1$ ein Wasserstoffatom, ein Halogenatom oder eine geradkettige oder verzweigte Niedrigalkoxygruppe,
- $R_2$ ein Wasserstoffatom oder eine geradkettige oder verzweigte Niedrigalkylgruppe und
- m 0 oder 1 darstellen,

B 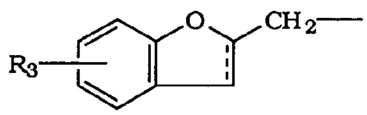

in der $R_3$ ein Wasserstoffatom, eine geradkettige oder verzweigte Niedrigalkylgruppe oder ein Halogenatom darstellt,

C 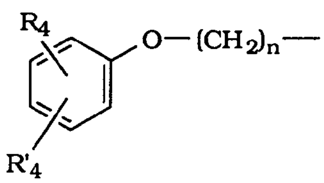

in der:
- $R_4$ und $R'_4$, die gleichartig oder verschieden sein können, Wasserstoffatome, Halogenatome, geradkettige oder verzweigte Niedrigalkoxygruppen oder gemeinsam, wenn sie an zwei benachbarten Kohlenstoffatomen stehen, eine Ethylendioxygruppe und
- n 2 oder 3 darstellen,

D 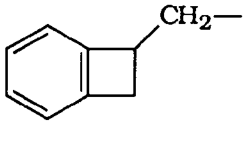

E 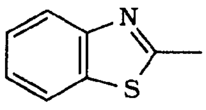

F 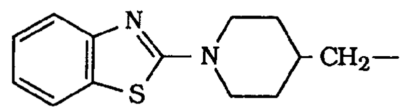

G 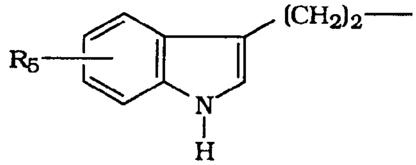

in denen:

- $R_5$ ein Wasserstoffatom oder eine geradkettige oder verzweigte $C_1$-$C_6$-Alkoxygruppe darstellt,

R' ein Wasserstoffatom. eine geradkettige oder verzweigte Niedrigalkylgruppe oder eine der Gruppen A, B, C, D, E oder F, mit der Maßgabe, daß R' ein Wasserstoffatom darstellt, wenn R die Gruppe G bedeutet,

oder

R und R' gemeinsam mit dem Stickstoffatom, an das sie gebunden sind, einen unsubstituierten oder am freien Stickstoffatom durch eine Phenylgruppe (die nicht substituiert oder durch eines oder mehrere Halogenatome oder $C_1$-$C_6$-Alkylgruppen substituiert ist) oder eine $C_1$-$C_6$-Alkoxygruppe substituierten Piperazinring bedeuten,

wobei der Begriff "Niedrig" bedeutet, daß die in dieser Weise bezeichneten Gruppen 1 bis 6 Kohlenstoffatome aufweisen und die gestrichelte Linie der Gruppe B die Anwesenheit einer Einfachbindung oder einer Doppelbindung bedeutet,

deren Enantiomere, Diastereoisomere und Epimere sowie deren Additionssalze mit einer pharmazeutisch annehmbaren Säure.

2. Verbindungen nach Anspruch 1, worin X ein Schwefelatom bedeutet.

3. Verbindungen nach Anspruch 1, worin X ein Sauerstoffatom bedeutet.

4. Verbindungen nach Anspruch 1, worin X eine Aminogruppe, die gegebenenfalls durch eine geradkettige oder verzweigte Niedrigalkylgruppe substituiert ist, bedeutet.

5. Verbindungen nach Anspruch 1, worin R eine gegebenenfalls substituierte (1,4-Benzodioxan-2-yl)-methyl-gruppe bedeutet.

6. Verbindungen nach Anspruch 1, worin R eine gegebenenfalls substituierte Phenoxyethylgruppe bedeutet.

7. Verbindungen nach Anspruch 1, worin R eine gegebenenfalls substituierte (Indol-3-yl)-ethyl-gruppe bedeutet, deren Enantiomere, Diastereoisomere und Epimere sowie deren Additionssalze mit einer pharmazeutisch annehmbaren Säure.

8. Verbindung nach einem der Ansprüche 1, 2 oder 6, nämlich 1 -(Benzothiazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

9. Verbindung nach einem der Ansprüche 1, 2 oder 5, nämlich 1-(Benzothiazol-2-yl)-4-[(8-methoxy-1,4-benzodioxan-2-yl)-methyl-aminomethyl]-piperidin, dessen Enantiomere und Diastereoisomere sowie dessen Additionssalze mit einer pharmazeutisch annehmbaren Säure.

10. Verbindung nach einem der Ansprüche 1, 3 oder 6, nämlich 1-(Benzoxazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

11. Verbindung nach einem der Ansprüche 1, 4 oder 6, nämlich 1-(Benzimidazol-2-yl)-4-[[2-(2-methoxyphenoxy)-ethyl]-aminomethyl]-piperidin, dessen Enantiomere sowie dessen Additionssalze mit einer pharmazeutisch annehmbaren Säure.

12. Verbindung nach einem der Ansprüche 1, 2 oder 7, nämlich 1-(Benzothiazol-2-yl)-4-[[2-(5-methoxyindol-3-yl)-ethyl]-aminomethyl]-piperidin, dessen Enantiomere sowie dessen Additionssalze mit einer pharmazeutisch annehmbaren Säure.

13. Verfahren zur Herstellung der Derivate der Formel (I) nach Anspruch 1, **dadurch gekennzeichnet,** daß man ein Derivat der Formel (II):

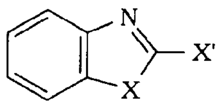

(II)

in der:

X die bezüglich der Formel (I) angegebenen Bedeutungen besitzt und X' ein Halogenatom darstellt.

1/ <u>entweder:</u>

mit 4-Aminomethylpiperidin der Formel (III):

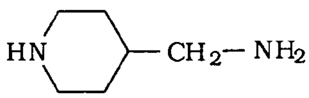

(III)

umsetzt, so daß man ein Derivat der Formel (I/a) erhält, einem Sonderfall der Derivate der Formel (I):

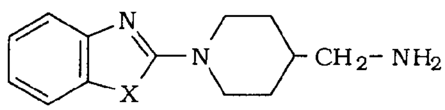

(I/a)

in der X die bezüglich der Formel (I) angegebenen Bedeutungen besitzt, welches man mit einem Derivat der Formel (IV):

$R_1$ - L (IV)

in der $R_1$ eine der Gruppen A, B, C, D, E oder F und L eine freisetzbare Gruppe, wie das Toluolsulfonat, das Methansulfonat oder ein Halogenatom bedeuten, von dem man gegebenenfalls die Isomeren durch eine klassische Herstellungsweise gebildet hat, kondensiert, so daß man ein Derivat der Formel (I/b) erhält, einem Sonderfall der Derivate der Formel (I):

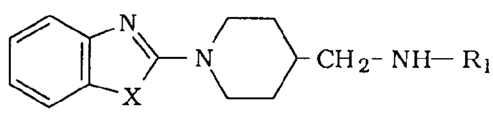

(I/b)

in der X und $R_1$ die oben angegebenen Bedeutungen besitzen, dessen Isomere man gegebenenfalls mit Hilfe einer klassischen Trennungsmethode trennt, welches man gewünschtenfalls

- entweder mit einer Verbindung der Formel (V)

  R" - L (V)

  in der L die oben angegebenen Bedeutungen besitzt und R" eine der Gruppen A, B, C, D, E oder F darstellt, umsetzt zur Bildung eines Derivats der Formel (I/c) erhält, einem Sonderfall der Derivate der Formel (I):

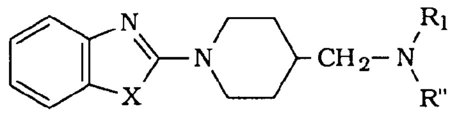

(I/c)

in der X, $R_1$ und R" die oben angegebenen Bedeutungen besitzen, das man gegebenenfalls mit Hilfe einer klassischen Trennmethode in die Isomeren auftrennt,

- oder mit einem Aldehyd der Formel (VI)

R''' - CHO (VI)

in der R''' eine geradkettige oder verzweigte Niedrigalkylgruppe bedeutet, in Gegenwart eines Reduktionsmittels, wie Natriumborhydrid, Natriumcyanoborhydrid, Wasserstoff, Raney-Nickel oder Ameisensäure umsetzt, so daß man ein Derivat der Formel (I/d), erhält:

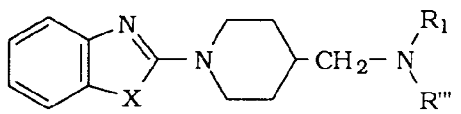
(I/d)

in der X, $R_1$ und R''' die oben angegebenen Bedeutungen besitzen, das man gegebenenfalls mit Hilfe einer klassischen Trennmethode in die Isomeren auftrennt,

2/ oder

mit 4-Hydroxymethylpiperidin der Formel (VII):

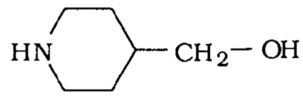
(VII)

umsetzt, so daß man eine Verbindung der Formel (VIII)

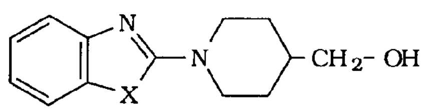
(VIII)

in der X die oben angegebenen Bedeutungen besitzt, erhält, die man gegebenenfalls mit Hilfe einer klassischen Trennmethode in die Isomeren auftrennt, welche man der Einwirkung von Tosylchlorid (Cl-Tos) unterwirft, so daß man eine Verbindung der Formel (IX):

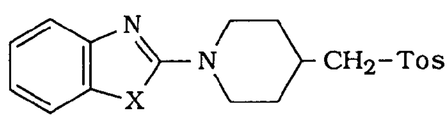
(IX)

erhält, in der X die oben angegebenen Bedeutungen besitzt, welche man:

- entweder mit einer Verbindung der Formel (X):

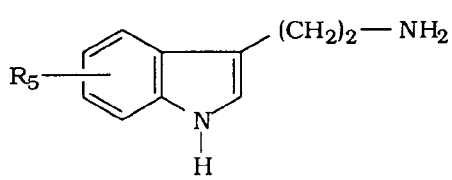
(X)

in der $R_5$ die bezüglich der Formel (I) angegebenen Bedeutungen besitzt, umsetzt, so daß man eine Verbindung der Formel (I/e) erhält, einem Sonderfall der Derivate der Formel (I):

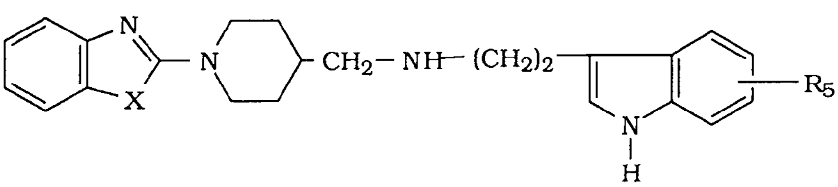
(I/e)

in der X und $R_5$ die oben angegebenen Bedeutungen besitzen, welche man gegebenenfalls mit Hilfe

einer klassischen Trennmethode in die Isomeren auftrennt,

- oder mit einem Piperazin der Formel (XI):

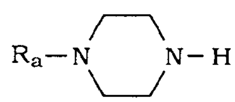

(XI)

in der $R_a$ ein Wasserstoffatom oder eine unsubstituierte oder durch eines oder mehrere Halogenatome, $C_1$-$C_6$-Alkylgruppen oder $C_1$-$C_6$-Alkoxygruppen substituierte Phenylgruppe darstellt, umsetzt, so daß man eine Verbindung der Formel (I/f) erhält, einem Sonderfall der Derivate der Formel (I):

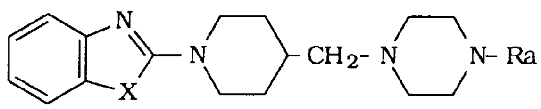

(I/f)

in der X und $R_a$ die oben angegebenen Bedeutungen besitzen, die man gegebenenfalls mit Hilfe einer klassischen Methode in die Isomeren auftrennt,
wobei man die Derivate (I/a), (I/b), (I/c), (I/d), (I/e) und (I/f) mit Hilfe einer klassischen Reinigungsmethode reinigt und gewünschtenfalls mit einer pharmazeutisch annehmbaren Säure in ihre Additionssalze überführt.

**14.** Pharmazeutische Zubereitungen enthaltend als Wirkstoffmindestens eine Verbindung nach einem der Ansprüche 1 bis 12 allein oder in Kombination mit einem oder mehreren inerten, nichttoxischen, pharmazeutisch annehmbaren Bindemitteln oder Trägermaterialien.

**15.** Pharmazeutische Zubereitungen nach Anspruch 14 enthaltend mindestens einen Wirkstoff nach einem der Ansprüche 1 bis 12 zur Behandlung von Depressionen, Streß, Psychosen, der Angst, Schmerzen, der Schizophrenie, der Hypertension und kardio-vaskulären Erkrankungen und als Mittel zur Modifizierung des Ernährungs- und Sexualverhaltens.

## Claims

### Claims for the following Contracting States : AT, BE, CH, DE DK, FR, GB, IT, LI, LU, NL, SE

**1.** Compounds of the general formula (I) :

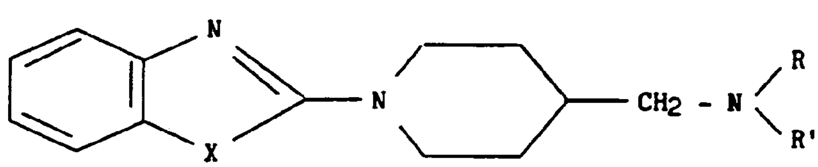

(I)

wherein :

- X represents a sulphur atom, an oxygen atom, or an amino group optionally substituted by a straight-chain or branched lower alkyl group,
- R represents a hydrogen atom or any one of the following groups :

A

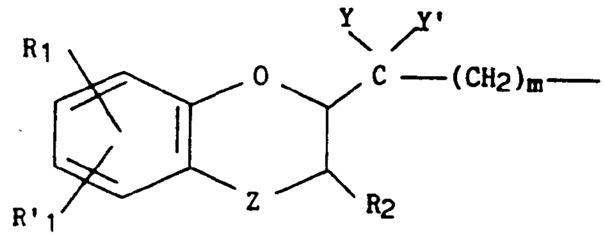

wherein :

- Z represents an oxygen atom or a $CH_2$ group,
- Y represents a hydrogen atom,
- Y' represents a hydrogen atom,
- or Y and Y' together form an oxygen atom,
- $R_1$ represents a hydrogen atom, a halogen atom, a straight-chain or branched lower alkyl group or a straight-chain or branched lower alkoxy group,
- $R'_1$ represents a hydrogen atom, a halogen atom, or a straight-chain or branched lower alkoxy group,
- $R_2$ represents a hydrogen atom or a straight-chain or branched lower alkyl group, and
- m is 0 or 1,

B

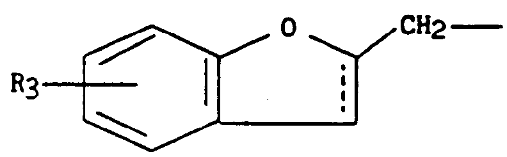

wherein

$R_3$ represents a hydrogen atom, a straight-chain or branched lower alkyl group or a halogen atom,

C

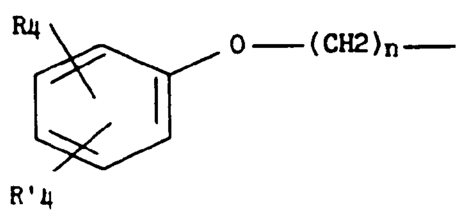

wherein :

- $R_4$ and $R'_4$, which are the same or different, each represents a hydrogen atom, a halogen atom or a straight-chain or branched lower alkoxy group or, when they are positioned at two adjacent carbon atoms, together form an ethylenedioxy group,
- n is 2 or 3,

D

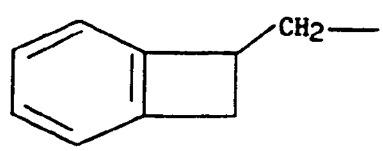

E

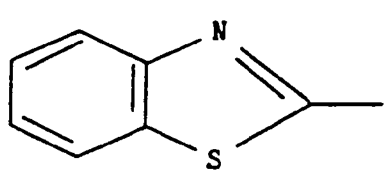

F

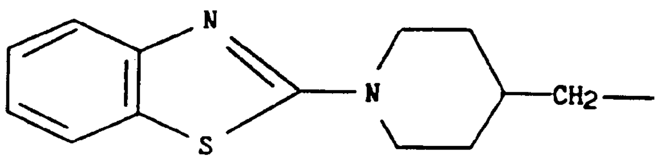

G

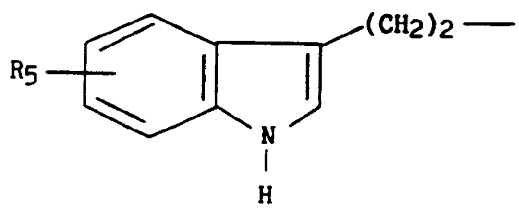

wherein :

$R_5$ represents a hydrogen atom or a straight-chain or branched ($C_1$-$C_6$)alkoxy group,

and

R' represents a hydrogen atom, a straight-chain or branched lower alkyl group or any one of the groups A, B, C, D, E and F, with the proviso that R' represents a hydrogen atom when R represents the group G,

or

R and R', together with the nitrogen atom to which they are attached, form a piperazine ring which is unsubstituted or substituted at the free nitrogen atom of the piperazine by a phenyl group (unsubstituted or substituted by one or more halogen atoms or ($C_1$-$C_6$)alkyl or ($C_1$-$C_6$)alkoxy groups),

the term "lower" indicating that the groups so qualified contain from 1 to 6 carbon atoms, and the dotted line in group B indicating the presence of a single or double bond,

their enantiomers, diastereoisomers and epimers, and also their addition salts with a pharmaceutically acceptable acid.

2. Compounds according to claim 1 wherein X represents a sulphur atom.

3. Compounds according to claim 1 wherein X represents an oxygen atom.

4. Compounds according to claim 1 wherein X represents an amino group optionally substituted by a straight-chain or branched lower alkyl group.

5. Compounds according to claim 1 wherein R represents an optionally substituted (1,4-benzodioxan-2-yl)methyl group.

6. Compounds according to claim 1 wherein R represents an optionally substituted phenoxyethyl group.

7. Compounds according to claim 1 wherein R represents an optionally substituted (indol-3-yl)ethyl group, their enantiomers, diastereoisomers and epimers, and also their addition salts with a pharmaceutically acceptable acid.

8. Compound according to any one of claims 1, 2 and 6, which is 1-(benzothiazol-2-yl)-4-[[2-(2-methoxyphenoxy)ethyl]aminomethyl]piperidine, its enantiomers, and also its addition salts with a pharmaceutically acceptable acid.

9. Compound according to any one of claims 1, 2 and 5, which is 1-(benzothiazol-2-yl)-4-[(8-methoxy-1,4-benzodioxan-2-yl)methylaminomethyl]piperidine, its enantiomers and diastereoisomers, and also its addition salts with a pharmaceutically acceptable acid.

10. Compound according to any one of claims 1, 3 and 6, which is 1-(benzoxazol-2-yl)-4-[[2-(2-methoxyphenoxy)ethyl]aminomethyl]piperidine, its enantiomers, and also its addition salts with a pharmaceutically acceptable acid.

**11.** Compound according to any one of claims 1, 4 and 6, which is 1-(benzimidazol-2-yl)-4-[[2-(2-methoxyphenoxy)ethyl]aminomethyl]piperidine, its enantiomers, and also its addition salts with a pharmaceutically acceptable acid.

**12.** Compound according to any one of claims 1, 2 and 7, which is 1-(benzothiazol-2-yl)-4-[[2-(5-methoxyindol-3-yl)ethyl]aminomethyl]piperidine, its enantiomers, and also its addition salts with a pharmaceutically acceptable acid.

**13.** Process for the preparation of the compounds of formula (I) according to claim 1, characterised in that a compound of formula (II) :

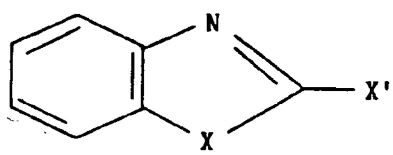

(II)

wherein :

X is as defined for formula (I) and X' is a halogen atom, is reacted

1/ **<u>either</u>**

with 4-aminomethylpiperidine of formula (III) :

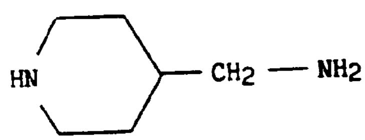

(III)

to yield a compound of formula (I/a), a particular case of compounds of formula (I) :

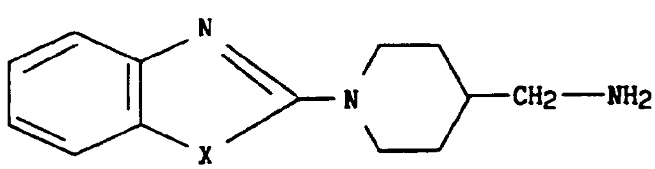

(I/a)

wherein X is as defined for formula (I), which is condensed with a compound of formula (IV) :

$R_1$ - L **(IV)**

wherein $R_1$ represents any one of the groups A, B, C, D, E and F,
L is a leaving group, such as toluenesulphonate, methanesulphonate or a halogen atom,
and the isomers of which are optionally prepared by a conventional method of preparation,
to yield a compound of formula (I/b), a particular case of compounds of formula (I)

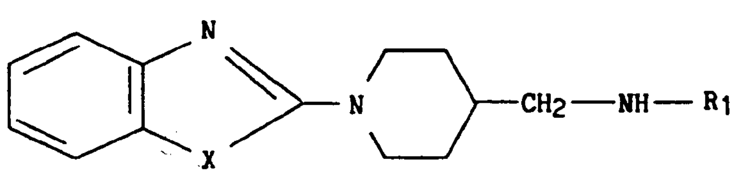

(1/b)

wherein X and $R_1$ are as defined hereinbefore, which is optionally separated into its isomers by a conventional method of separation,
and which, if desired, is subjected

- either to the action of a compound of formula (V):

  R" - L **(V)**

  wherein L is as defined hereinbefore and R" is any one of the groups A, B, C, D, E and F, to yield a compound of formula (I/c), a particular case of compounds of formula (I)

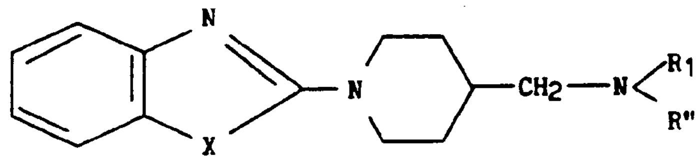

(I/c)

wherein X, $R_1$ and R" are as defined hereinbefore, which is optionally separated into its isomers by a conventional method of separation,

- or to the action of an aldehyde of formula (VI)

  R'" - CHO **(VI)**

  wherein R'" is a straight-chain or branched lower alkyl group, in the presence of a reducing agent, such as sodium borohydride, sodium cyanoborohydride, hydrogen, Raney nickel or formic acid, to yield a compound of formula (I/d) :

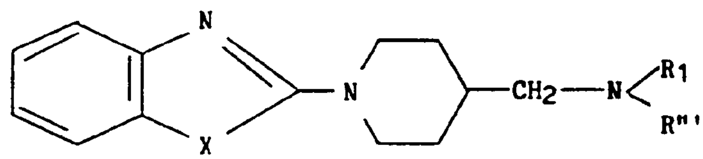

(I/d)

wherein X, $R_1$ and R'" are as defined hereinbefore, which is optionally separated into its isomers by a conventional method of separation,

2/ <u>or</u> :

with 4-hydroxymethylpiperidine of formula (VII) :

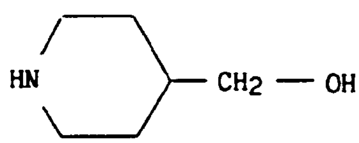

(VII)

to yield a compound of formula (VIII) :

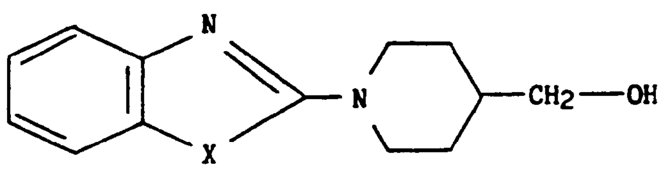

(VIII)

wherein X is as defined hereinbefore,
which is optionally separated into its isomers by a conventional method of separation,
and which is subjected to the action of tosyl chloride (Cl-Tos)
to yield a compound of formula (IX) :

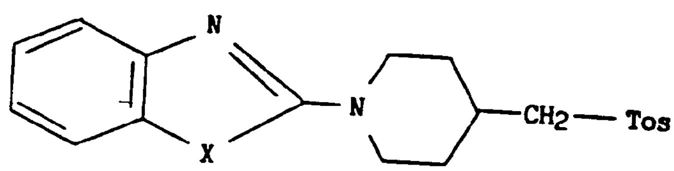

(IX)

wherein X is as defined hereinbefore,
which is reacted :

- <u>either</u> with a compound of formula (X) :

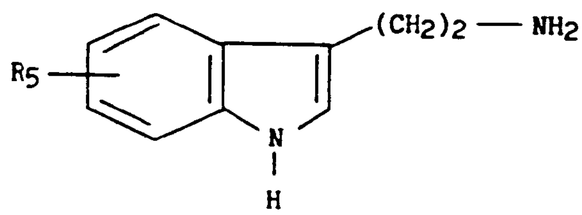

(X)

wherein $R_5$ is as defined for formula (I), to yield a compound of formula (I/e), a particular case of compounds of formula (I) :

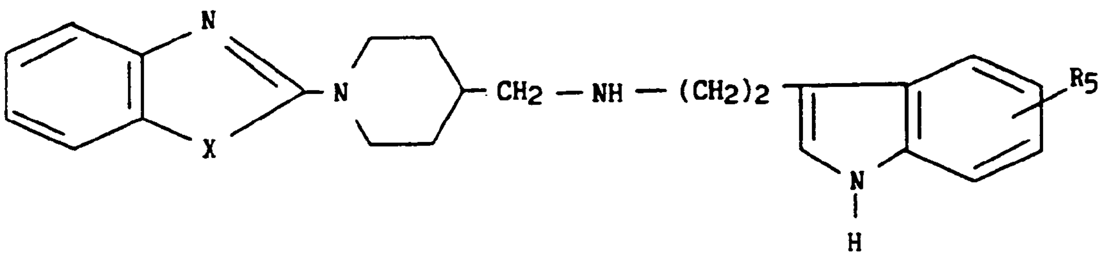

(I/e)

wherein X and $R_5$ are as defined hereinbefore, which is optionally separated into its isomers by a conventional method of separation,

- or with a piperazine of formula (XI) :

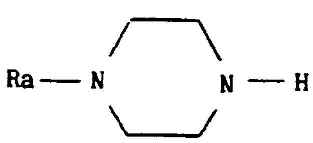

(XI)

wherein Ra represents a hydrogen atom or a phenyl group which is unsubstituted or substituted by one or more halogen atoms or $(C_1-C_6)$alkyl or $(C_1-C_6)$alkoxy groups, to yield a compound of formula (I/f), a particular case of compounds of formula (I) :

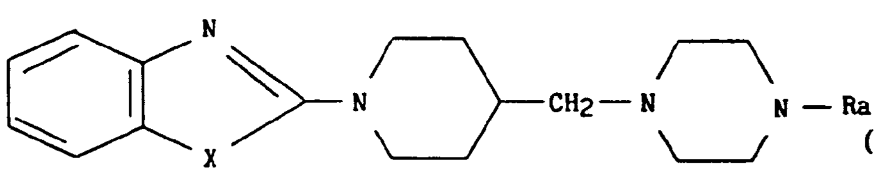

(I/f)

wherein X and Ra are as defined hereinbefore,
which is optionally separated into its isomers by a conventional method of separation,
which compounds (I/a), (I/b), (I/c), (I/d), (I/e) and (I/f) are purified by a conventional method of purification and, if desired, are converted into their addition salts with a pharmaceutically acceptable acid.

**14.** Pharmaceutical compositions comprising as active ingredient at least one compound according to any one of claims 1 to 12, alone or in combination with one or more pharmaceutically acceptable, inert, non-toxic excipients or carriers.

**15.** Pharmaceutical compositions according to claim 14 containing at least one active ingredient according to any one of claims 1 to 12, for use in the treatment of depression, stress, psychoses, anxiety, pain, schizophrenia, hypertension or cardiovascular disorders, or for the modification of eating or sexual behaviour.

**Claims for the following Contracting State : ES**

1. Process for the preparation of compounds of the general formula (I) :

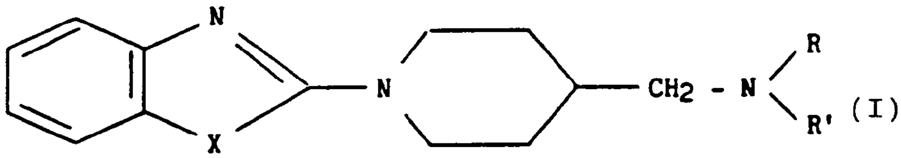

wherein :

- X represents a sulphur atom, an oxygen atom, or an amino group optionally substituted by a straight-chain or branched lower alkyl group,
- R represents a hydrogen atom or any one of the following groups :

A 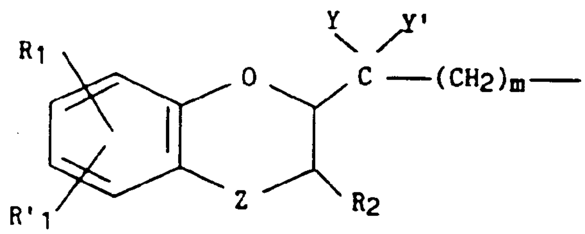

wherein :

- Z represents an oxygen atom or a $CH_2$ group,
- Y represents a hydrogen atom,
- Y' represents a hydrogen atom,
- or Y and Y' together form an oxygen atom,
- $R_1$ represents a hydrogen atom, a halogen atom, a straight-chain or branched lower alkyl group or a straight-chain or branched lower alkoxy group,
- $R'_1$ represents a hydrogen atom, a halogen atom, or a straight-chain or branched lower alkoxy group,
- $R_2$ represents a hydrogen atom or a straight-chain or branched lower alkyl group, and
- m is 0 or 1,

B 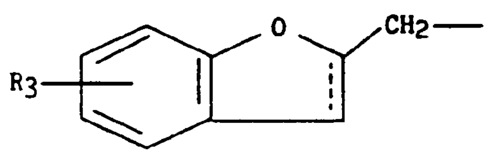

wherein

$R_3$ represents a hydrogen atom, a straight-chain or branched lower alkyl group or a halogen atom,

C 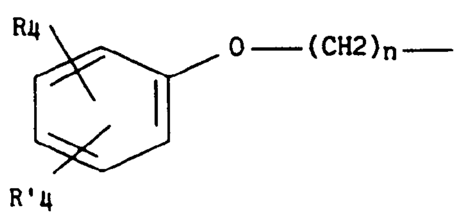

wherein :

- $R_4$ and $R'_4$, which are the same or different, each represents a hydrogen atom, a halogen atom or a straight-chain or branched lower alkoxy group or, when they are positioned at two adjacent carbon atoms, together form an ethylenedioxy group,

- n is 2 or 3,

D 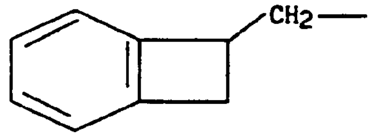

E 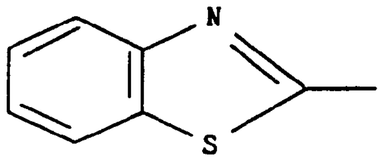

F 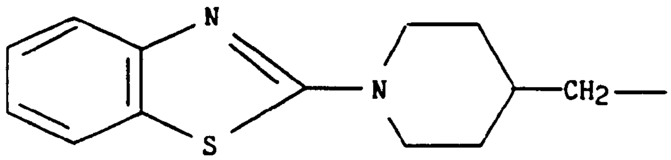

G 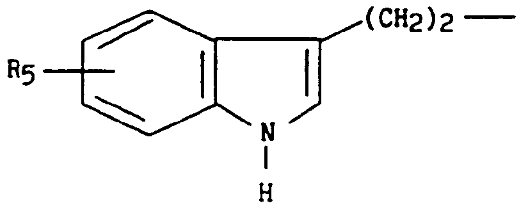

wherein :

$R_5$ represents a hydrogen atom or a straight-chain or branched ($C_1$-$C_6$)alkoxy group,

and

R' represents a hydrogen atom, a straight-chain or branched lower alkyl group or any one of the groups A, B, C, D, E and F, with the proviso that R' represents a hydrogen atom when R represents the group G,

or

R and R', together with the nitrogen atom to which they are attached, form a piperazine ring which is unsubstituted or substituted at the free nitrogen atom of the piperazine by a phenyl group (unsubstituted or substituted by one or more halogen atoms or ($C_1$-$C_6$)alkyl or ($C_1$-$C_6$)alkoxy groups),

the term "lower" indicating that the groups so qualified contain from 1 to 6 carbon atoms, and the dotted line in group B indicating the presence of a single or double bond,

their enantiomers, diastereoisomers and epimers, and also their addition salts with a pharmaceutically acceptable acid,

characterised in that a compound of formula (II) :

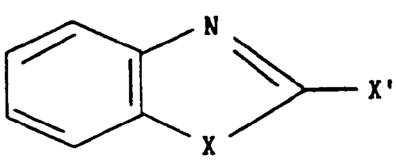

(II)

wherein :

X is as defined for formula (I) and X' is a halogen atom, is reacted

1/ **either**

with 4-aminomethylpiperidine of formula (III) :

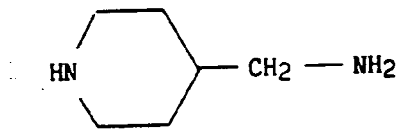 (III)

to yield a compound of formula (I/a), a particular case of compounds of formula (I) :

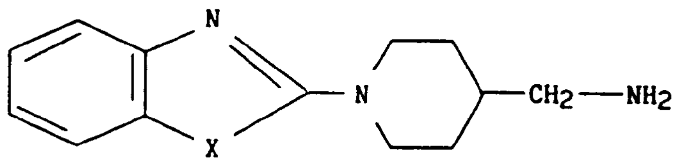 (I/a)

wherein X is as defined for formula (I),
which is condensed with a compound of formula (IV) :

$R_1$ - L **(IV)**

wherein $R_1$ represents any one of the groups A, B, C, D, E and F,
L is a leaving group, such as toluenesulphonate, methanesulphonate or a halogen atom,
and the isomers of which are optionally prepared by a conventional method of preparation,
to yield a compound of formula (I/b), a particular case of compounds of formula (I)

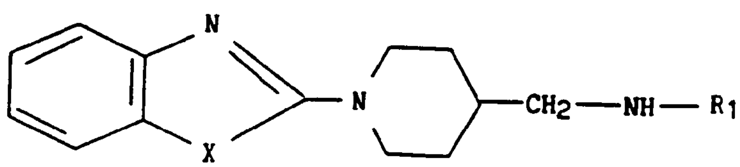 (1/b)

wherein X and $R_1$ are as defined hereinbefore, which is optionally separated into its isomers by a conventional method of separation,
and which, if desired, is subjected

- either to the action of a compound of formula (V):

  R" - L **(V)**

  wherein L is as defined hereinbefore and R" is any one of the groups A, B, C, D, E and F, to yield a compound of formula (I/c), a particular case of compounds of formula (I)

  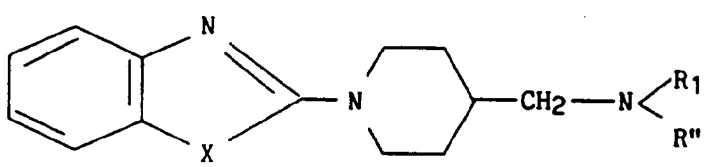 (I/c)

  wherein X, $R_1$ and R" are as defined hereinbefore, which is optionally separated into its isomers by a conventional method of separation,
- or to the action of an aldehyde of formula (VI)

  R'" - CHO **(VI)**

  wherein R'" is a straight-chain or branched lower alkyl group, in the presence of a reducing agent, such as sodium borohydride, sodium cyanoborohydride, hydrogen, Raney nickel or formic acid, to yield a compound of formula (I/d) :

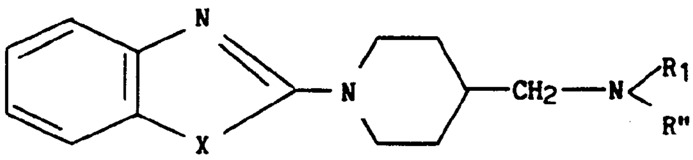

(I/d)

wherein X, $R_1$ and R''' are as defined hereinbefore, which is optionally separated into its isomers by a conventional method of separation,

2/ <u>or</u> :

with 4-hydroxymethylpiperidine of formula (VII) :

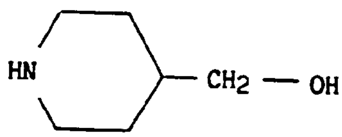

(VII)

to yield a compound of formula (VIII) :

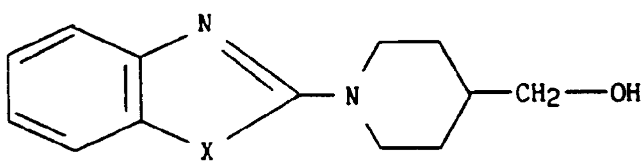

(VIII)

wherein X is as defined hereinbefore,
which is optionally separated into its isomers by a conventional method of separation,
and which is subjected to the action of tosyl chloride (Cl-Tos)
to yield a compound of formula (IX) :

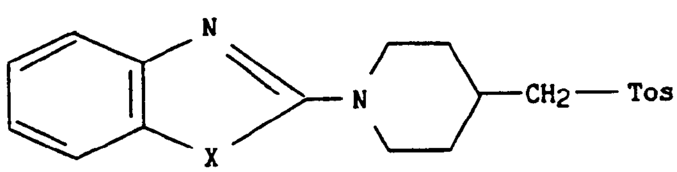

(IX)

wherein X is as defined hereinbefore,
which is reacted :

- <u>either</u> with a compound of formula (X) :

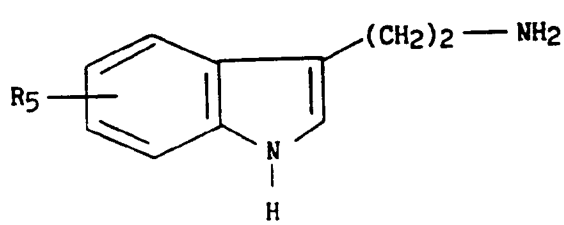

(X)

wherein $R_5$ is as defined for formula (I), to yield a compound of formula (I/e), a particular case of compounds of formula (I) :

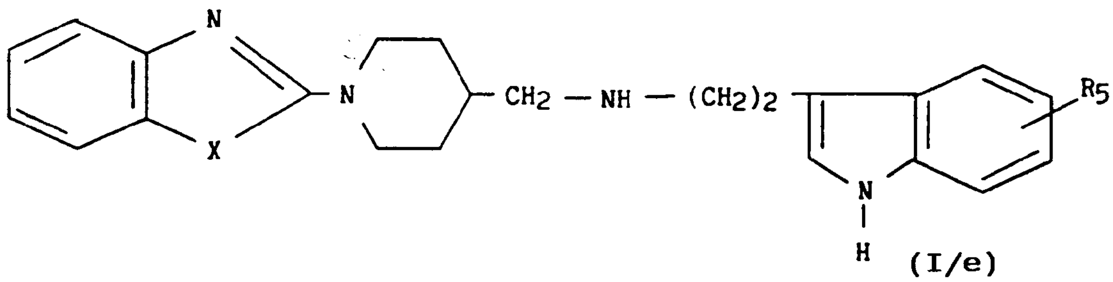

(I/e)

wherein X and $R_5$ are as defined hereinbefore, which is optionally separated into its isomers by a conventional method of separation,

- or with a piperazine of formula (XI) :

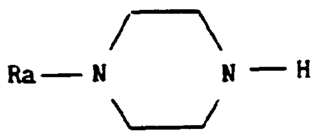

(XI)

wherein Ra represents a hydrogen atom or a phenyl group which is unsubstituted or substituted by one or more halogen atoms or $(C_1\text{-}C_6)$alkyl or $(C_1\text{-}C_6)$alkoxy groups, to yield a compound of formula (I/f), a particular case of compounds of formula (I) :

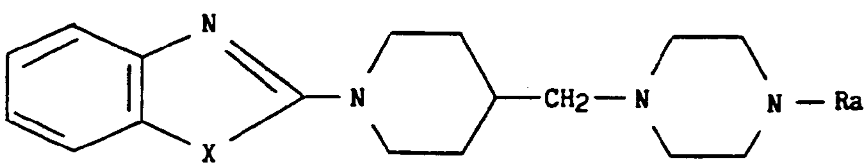

(I/f)

wherein X and Ra are as defined hereinbefore,
which is optionally separated into its isomers by a conventional method of separation,
which compounds (I/a), (I/b), (I/c), (I/d), (I/e) and (I/f) are purified by a conventional method of purification and, if desired, are converted into their addition salts with a pharmaceutically acceptable acid.

2. Process according to claim 1 for the preparation of compounds wherein X represents a sulphur atom.

3. Process according to claim 1 for the preparation of compounds wherein X represents an oxygen atom.

4. Process according to claim 1 for the preparation of compounds wherein X represents an amino group optionally substituted by a straight-chain or branched lower alkyl group.

5. Process according to claim 1 for the preparation of compounds wherein R represents an optionally substituted (1,4-benzodioxan-2-yl)methyl group.

6. Process according to claim 1 for the preparation of compounds wherein R represents an optionally substituted phenoxyethyl group.

7. Process according to claim 1 for the preparation of compounds wherein R represents an optionally substituted (indol-3-yl)ethyl group, their enantiomers, diastereoisomers and epimers, and also their addition salts with a pharmaceutically acceptable acid.

8. Process according to any one of claims 1, 2 and 6 for the preparation of the compound 1-(benzothiazol-2-yl)-4-[[2-(2-methoxyphenoxy)ethyl]aminomethyl]piperidine, its enantiomers, and also its addition salts with a pharmaceutically acceptable acid.

9. Process according to any one of claims 1, 2 and 5 for the preparation of the compound 1-(benzothiazol-2-yl)-4-[(8-methoxy-1,4-benzodioxan-2-yl)methylaminomethyl]piperidine, its enantiomers and diastereoisomers, and also its addition salts with a pharmaceutically acceptable acid.

10. Process according to any one of claims 1, 3 and 6 for the preparation of the compound 1-(benzoxazol-2-yl)-4-[[2-(2-methoxyphenoxy)ethyl]aminomethyl]piperidine, its enantiomers, and also its addition salts with a pharmaceutically acceptable acid.

11. Process according to any one of claims 1, 4 and 6 for the preparation of the compound 1-(benzimidazol-2-yl)-4-[[2-(2-methoxyphenoxy)ethyl]aminomethyl]piperidine, its enantiomers, and also its addition salts with a pharmaceutically acceptable acid.

12. Process according to any one of claims 1, 2 and 7 for the preparation of the compound 1-(benzothiazol-2-yl)-4-[[2-(5-methoxyindol-3-yl)ethyl]aminomethyl]piperidine, its enantiomers, and also its addition salts with a pharmaceutically acceptable acid.

**Claims for the following Contracting State : GR**

1. Compounds of the general formula (I) :

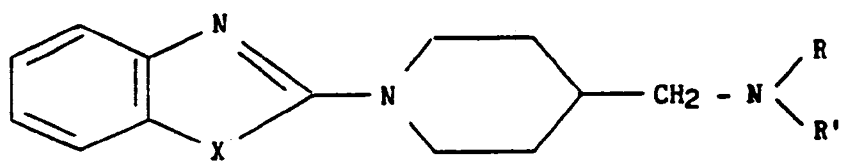

(I)

wherein :
- X represents a sulphur atom, an oxygen atom, or an amino group optionally substituted by a straight-chain or branched lower alkyl group,
- R represents a hydrogen atom or any one of the following groups :

A

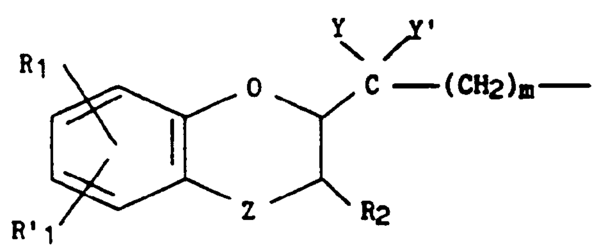

wherein :
- Z represents an oxygen atom or a $CH_2$ group,
- Y represents a hydrogen atom,
- Y' represents a hydrogen atom,
- or Y and Y' together form an oxygen atom,
- $R_1$ represents a hydrogen atom, a halogen atom, a straight-chain or branched lower alkyl group or a straight-chain or branched lower alkoxy group,
- $R'_1$ represents a hydrogen atom, a halogen atom, or a straight-chain or branched lower alkoxy group,
- $R_2$ represents a hydrogen atom or a straight-chain or branched lower alkyl group, and
- m is 0 or 1,

B

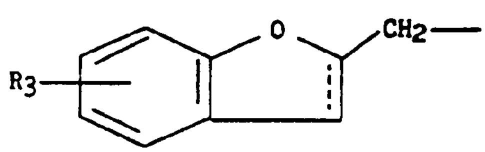

wherein

$R_3$ represents a hydrogen atom, a straight-chain or branched lower alkyl group or a halogen atom,

C 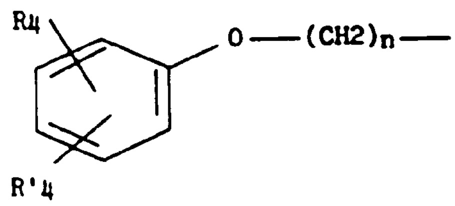

wherein :

- $R_4$ and $R'_4$, which are the same or different, each represents a hydrogen atom, a halogen atom or a straight-chain or branched lower alkoxy group or, when they are positioned at two adjacent carbon atoms, together form an ethylenedioxy group,
- n is 2 or 3,

D 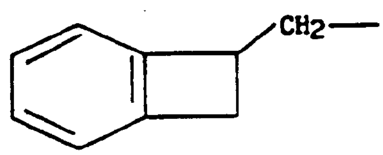

E 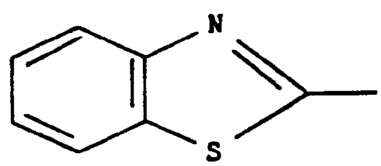

F 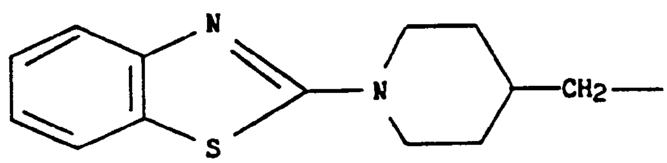

G 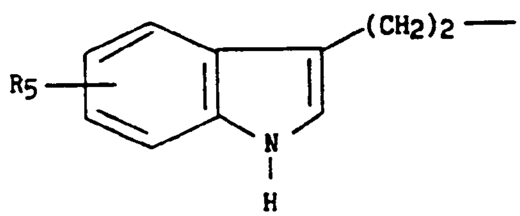

wherein :

$R_5$ represents a hydrogen atom or a straight-chain or branched ($C_1$-$C_6$)alkoxy group,

and

R' represents a hydrogen atom, a straight-chain or branched lower alkyl group or any one of the groups A, B, C, D, E and F, with the proviso that R' represents a hydrogen atom when R represents the group G,

or

R and R', together with the nitrogen atom to which they are attached, form a piperazine ring which is unsubstituted or substituted at the free nitrogen atom of the piperazine by a phenyl group (unsubstituted or substituted by one or more halogen atoms or ($C_1$-$C_6$)alkyl or ($C_1$-$C_6$)alkoxy groups),

the term "lower" indicating that the groups so qualified contain from 1 to 6 carbon atoms, and the dotted line in group B indicating the presence of a single or double bond,

their enantiomers, diastereoisomers and epimers, and also their addition salts with a pharmaceutically acceptable acid.

2. Compounds according to claim 1 wherein X represents a sulphur atom.

3. Compounds according to claim 1 wherein X represents an oxygen atom.

4. Compounds according to claim 1 wherein X represents an amino group optionally substituted by a straight-chain or branched lower alkyl group.

5. Compounds according to claim 1 wherein R represents an optionally substituted (1,4-benzodioxan-2-yl)methyl group.

6. Compounds according to claim 1 wherein R represents an optionally substituted phenoxyethyl group.

7. Compounds according to claim 1 wherein R represents an optionally substituted (indol-3-yl)ethyl group, their enantiomers, diastereoisomers and epimers, and also their addition salts with a pharmaceutically acceptable acid.

8. Compound according to any one of claims 1, 2 and 6, which is 1-(benzothiazol-2-yl)-4-[[2-(2-methoxyphenoxy)ethyl]aminomethyl]piperidine, its enantiomers, and also its addition salts with a pharmaceutically acceptable acid.

9. Compound according to any one of claims 1, 2 and 5, which is 1-(benzothiazol-2-yl)-4-[(8-methoxy-1,4-benzodioxan-2-yl)methylaminomethyl]piperidine, its enantiomers and diastereoisomers, and also its addition salts with a pharmaceutically acceptable acid.

10. Compound according to any one of claims 1, 3 and 6, which is 1-(benzoxazol-2-yl)-4-[[2-(2-methoxyphenoxy)ethyl]aminomethyl]piperidine, its enantiomers, and also its addition salts with a pharmaceutically acceptable acid.

11. Compound according to any one of claims 1, 4 and 6, which is 1-(benzimidazol-2-yl)-4-[[2-(2-methoxyphenoxy)ethyl]aminomethyl]piperidine, its enantiomers, and also its addition salts with a pharmaceutically acceptable acid.

12. Compound according to any one of claims 1, 2 and 7, which is 1-(benzothiazol-2-yl)-4-[[2-(5-methoxyindol-3-yl)ethyl]aminomethyl]piperidine, its enantiomers, and also its addition salts with a pharmaceutically acceptable acid.

13. Process for the preparation of the compounds of formula (I) according to claim 1, characterised in that a compound of formula (II) :

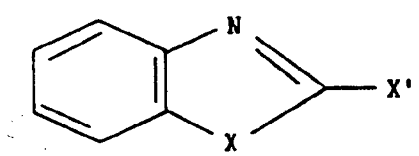

(II)

wherein :

X is as defined for formula (I) and X' is a halogen atom, is reacted

1/ **either**

with 4-aminomethylpiperidine of formula (III) :

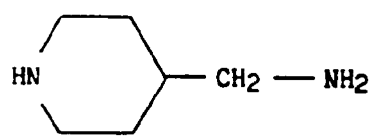

(III)

to yield a compound of formula (I/a), a particular case of compounds of formula (I) :

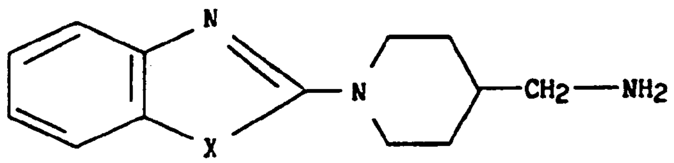

(I/a)

wherein X is as defined for formula (I), which is condensed with a compound of formula (IV) :

$R_1$ - L **(IV)**

wherein $R_1$ represents any one of the groups A, B, C, D, E and F,
L is a leaving group, such as toluenesulphonate, methanesulphonate or a halogen atom,
and the isomers of which are optionally prepared by a conventional method of preparation,
to yield a compound of formula (I/b), a particular case of compounds of formula (I)

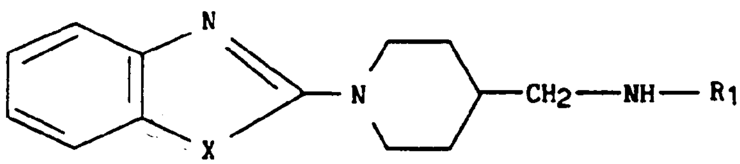

(1/b)

wherein X and $R_1$ are as defined hereinbefore, which is optionally separated into its isomers by a conventional method of separation,
and which, if desired, is subjected

- either to the action of a compound of formula (V):

  R" - L **(V)**

  wherein L is as defined hereinbefore and R" is any one of the groups A, B, C, D, E and F, to yield a compound of formula (I/c), a particular case of compounds of formula (I)

  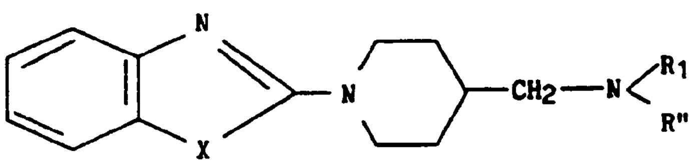

  (I/c)

  wherein X, $R_1$ and R" are as defined hereinbefore, which is optionally separated into its isomers by a conventional method of separation,
- or to the action of an aldehyde of formula (VI)

  R'" - CHO **(VI)**

  wherein R'" is a straight-chain or branched lower alkyl group, in the presence of a reducing agent, such as sodium borohydride, sodium cyanoborohydride, hydrogen, Raney nickel or formic acid, to yield a compound of formula (I/d) :

  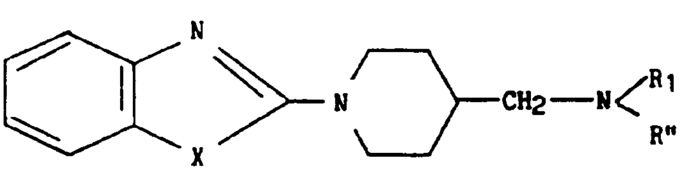

  (I/d)

  wherein X, $R_1$ and R'" are as defined hereinbefore, which is optionally separated into its isomers by a conventional method of separation,

2/ <u>or</u> :

with 4-hydroxymethylpiperidine of formula (VII) :

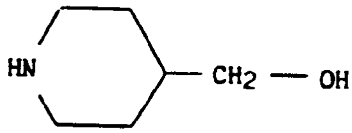

(VII)

to yield a compound of formula (VIII) :

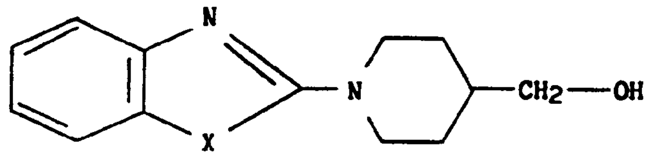

(VIII)

wherein X is as defined hereinbefore,
which is optionally separated into its isomers by a conventional method of separation,
and which is subjected to the action of tosyl chloride (Cl-Tos)
to yield a compound of formula (IX) :

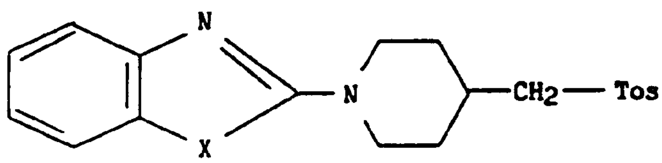

(IX)

wherein X is as defined hereinbefore,
which is reacted :

- either with a compound of formula (X) :

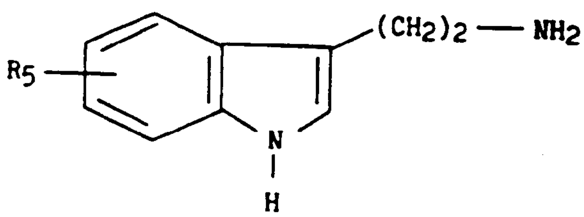

(X)

wherein $R_5$ is as defined for formula (I), to yield a compound of formula (I/e), a particular case of compounds of formula (I) :

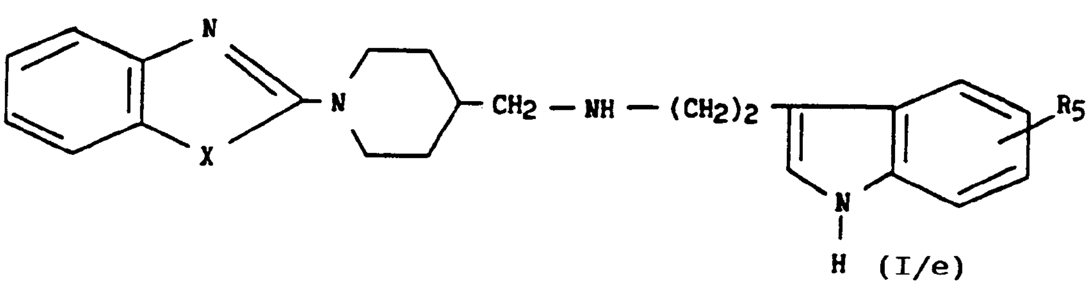

(I/e)

wherein X and $R_5$ are as defined hereinbefore, which is optionally separated into its isomers by a conventional method of separation,

- or with a piperazine of formula (XI) :

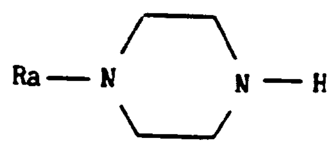

(XI)

wherein Ra represents a hydrogen atom or a phenyl group which is unsubstituted or substituted by one or more halogen atoms or $(C_1-C_6)$alkyl or $(C_1-C_6)$alkoxy groups, to yield a compound of formula (I/f), a particular case of compounds of formula (I) :

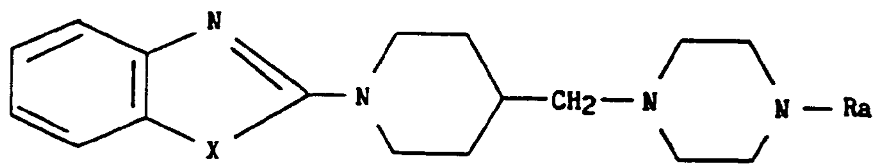

(I/f)

wherein X and Ra are as defined hereinbefore,

which is optionally separated into its isomers by a conventional method of separation,

which compounds (I/a), (I/b), (I/c), (I/d), (I/e) and (I/f) are purified by a conventional method of purification and, if desired, are converted into their addition salts with a pharmaceutically acceptable acid.

**14.** Pharmaceutical compositions comprising as active ingredient at least one compound according to any one of claims 1 to 12, alone or in combination with one or more pharmaceutically acceptable, inert, non-toxic excipients or carriers.

**15.** Pharmaceutical compositions according to claim 14 containing at least one active ingredient according to any one of claims 1 to 12, for use in the treatment of depression, stress, psychoses, anxiety, pain, schizophrenia, hypertension or cardiovascular disorders, or for the modification of eating or sexual behaviour.